(19) Europäisches Patentamt
European Patent Office
Office européen des brevets

(11) **EP 4 253 567 A1**

(12) **EUROPEAN PATENT APPLICATION**

(43) Date of publication:
**04.10.2023 Bulletin 2023/40**

(21) Application number: **22165911.3**

(22) Date of filing: **31.03.2022**

(51) International Patent Classification (IPC):
*C12Q 1/6886* (2018.01)

(52) Cooperative Patent Classification (CPC):
**C12Q 1/6886**; C12Q 2600/118; C12Q 2600/158

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **OncoAssure Limited**
**D04 V2P1 Dublin 4 (IE)**

(72) Inventors:
• **Barron, Stephen**
**Dublin 16, D16 XK83 (IE)**
• **Gallagher, William**
**Dublin 6, D06 W0Y8 (IE)**

• **Higgins, Debra**
**Dublin, A94 XR67 (IE)**
• **Loughman, Tony**
**Dublin, K67 WP11 (IE)**
• **O'Leary, Desmond**
**Dublin, A98 E6V9 (IE)**
• **Wang, Angel Chan-Ju**
**London, W3 6LY (GB)**

(74) Representative: **Purdylucey Intellectual Property**
**6-7 Harcourt Terrace**
**D02 FH73 Dublin 2 (IE)**

Remarks:
The complete document including Reference Table(s) and the Sequence Listing(s) can be downloaded from the EPO website

(54) **A METHOD OF PREDICTING RISK OF AN AGGRESSIVE OR RECURRENT CANCER**

(57)    A method of predicting risk of an aggressive or a recurrent cancer in an individual, the method comprising a step of assaying a biological sample from the individual for expression of at least two genes, or proteins encoded by said genes, selected from TTC21B, MCM3, MTUS1, FOXM1, CUL4A, GPC1, PCNA, IRF7, BRIP1, ULK1, and SPAG5, wherein when a combination of a normalised expression value of the at least two genes, or proteins encoded by said genes, is modified relative to a combination of a normalised expression reference value of the at least two genes, or proteins encoded by said, in a biological sample from a subject with non-aggressive or non-recurrent cancer, the individual is predicted to have an increased risk of having an aggressive or a recurrent cancer.

Figure 1

EP 4 253 567 A1

## Description

### Field of the Invention

The invention relates to a method of predicting the risk of the probability of an aggressive cancer or a recurrent cancer in a subject. Specifically, the invention relates to a method of predicting the risk of aggressive cancer in a subject and the recurrence of cancer in a subject.

### Background to the Invention

As a result of increased awareness of prostate cancer, widespread prostate-specific antigen (PSA) screening and digital rectal exam (DRE) testing, many prostate cancers (~ 60%) are detected at an early stage. It is predominantly detected (60%) in older men aged 65+ years. Approximately 80% of patients have slow growing or indolent prostate cancer and can leave their disease untreated while undergoing frequent monitoring known as active surveillance. The remaining 20% of patients have aggressive prostate cancers which have the potential to metastasize to additional sites in the body and require aggressive treatment. Historically, most prostate cancer patients have been treated aggressively with many undergoing a radical prostatectomy, coupled with radiation therapy, hormone therapy, or chemotherapy resulting in significant life-long adverse side-effects. This unnecessary over-treatment greatly increases the costs to both the patient and the healthcare system. Current clinical tools cannot determine the true risk of metastases, thus accurately identifying patients with aggressive prostate cancer (*i.e.*, predicting metastatic potential) represents a major clinical unmet need and is crucially important for selecting the appropriate treatment options for prostate cancer patients.
Prostate Cancer is the second most common cancer in men after non-melanoma skin cancer (representing 26% of all male cancer diagnoses in the UK), the second leading cause of cancer death in men after lung cancer, and the fourth most prevalent cancer globally. An estimated 1.2 million men are diagnosed with prostate cancer worldwide annually. The average age at diagnosis is 67 with approximately 25% of cases in men younger than 65. As a result of increased PSA screening, most patients are diagnosed with early-stage disease.
The major clinical question faced by clinicians and patients is how best to treat the prostate cancer; a decision must be made as to whether active surveillance is the appropriate clinical route in the case of indolent cancers, or if the patient requires radical treatment for an aggressive cancer. Radical prostatectomy and radiotherapy expose the patient to significant and life-long negative side effects and quality of life issues including incontinence, erectile dysfunction, emotional distress, and depression. Statistically, 80% of men will have indolent, slow growing, low-grade prostate cancer which can be safely managed by active surveillance with 20% of men requiring aggressive treatment. To date, approximately 76% of men have been treated as if they have aggressive disease, resulting in unnecessary over-treatment and exposing patients to significant side-effects. Accurately determining prostate cancer grade, stage, and aggressiveness is crucial for appropriate patient treatment stratification. Increasing the number of men under active surveillance will (i) reduce over-treatment and increase capacity for rapid treatment of high-risk disease, (ii) reduce the number of men unnecessarily having radical treatment and experiencing adverse effects, and (iii) decrease the cost of treating and managing these adverse effects.
It is an object of the present invention to overcome at least one of the above-mentioned problems.

### Summary of the Invention

Prostate cancer is the second most common cancer in men after non-melanoma skin cancer, the second leading cause of cancer death in men after lung cancer and the fourth most prevalent cancer globally. Approximately 80% of prostate cancer patients have slow growing cancer and can safely leave their disease untreated while undergoing active surveillance; while 20% have an aggressive cancer with a high potential for metastases and require aggressive treatment including radical prostatectomy (RP) combined with radiotherapy, hormone therapy or chemotherapy.
Current clinical tools cannot accurately identify whether a patient has aggressive prostate cancer, thus over-treatment is a major issue in the clinical management of prostate cancer as many patients undergo radical prostatectomy leading to severe complications including infection, incontinence, erectile dysfunction, and depression. There is an unmet clinical need for an accurate prognostic test to establish the risk of recurrence of a patient's prostate cancer in order to distinguish those who need aggressive treatment from those who can safely undergo active surveillance. Development of such a prognostic test would reduce the over-treatment of prostate cancer patients sparing many individuals from the extensive adverse side-effects of treatment.
The method of the claimed invention is based on measuring the changes in expression levels of genes which are highly associated with cancer progression and aggressiveness. The test measures the aggressiveness of, for example, prostate cancer and the potential of prostate cancer recurrence, enabling more personalised treatment strategies.
The method is a multigene prognostic signature used to assess the probability of, for example, aggressive disease in men recently diagnosed with prostate cancer. The test is performed on RNA samples extracted from formalin-fixed, paraffin-embedded (FFPE) prostate tumour tissues using RT-qPCR assays. Those deemed to be in the lower risk category (that is, having a lower risk score) by the test may safely forego immediate definitive treatment whereas those deemed to be in the higher risk category (that is, a higher risk score) may consider treatment intensification. Lower risk means those who have lower risk of aggressive or recurrent disease and higher risk means those who have higher risk of aggressive or recurrent disease.

The test employs a patient risk score generated by adding the expression levels of at least two genes together and comparing them with a reference risk score. The expression level of each gene is generally normalised. In some embodiments, a function of the expression level of each gene is added together and compared with a reference risk score. An example of this is set out in more detail in the specification below.

The method may comprise determining the combined expression of at least two genes, or proteins encoded by said genes, from a panel of genes in a sample from a subject. The expression level of each of the at least two genes is normalised against a reference gene (a set of one or more housekeeping genes, for example). The normalised expression levels, or a function of the normalised expression levels (e.g., a weighted normalised expression level, where the weighting is gene-specific) are then added together to provide a test risk score. The same exercise is carried out on a control sample (for example, a sample from a patient with non-aggressive cancer or from a recurrence-free subject), to provide a reference risk score. When both results/scores are obtained, they are compared against one another. When the combined normalised expression of the genes from the test sample is modified (either increased or decreased) relative to the combined normalised expression of the genes in the control sample, the test subject has a higher risk of aggressive or recurrent disease. The combination is an addition of the gene-specific weighted normalised expression of the genes.

There is provided, a method of predicting risk of an aggressive or a recurrent cancer in an individual, the method comprising: assaying a biological sample obtained from the individual for expression of at least two genes, or proteins encoded by said genes, selected from TTC21B, MCM3, MTUS1, FOXM1, CUL4A, GPC1, PCNA, IRF7, BRIP1, ULK1, and SPAG5, wherein when a combination of the normalised expression value of the at least two genes, or proteins encoded by said genes, is modified relative to a combination of a normalised expression value of the at least two genes, or proteins encoded by said genes, in a biological sample from a subject with non-aggressive or non-recurrent cancer, the individual is predicted to have an increased risk of having an aggressive or a recurrent cancer.

Expression value may be, for example, a copy number or a function of the copy number such as a gene-specific weighted copy number. Gene-specific weightings may be determined based on the relative contribution of a specific gene to risk of an aggressive or recurrent phenotype as determined from analysis of the levels of the genes in a cohort of cancer patients including patients having a good outcome and those having a poor outcome. An example of such a cohort of patients is described in the TCGA cohort under the Statistical Analysis heading in the Materials and Method Section.

A method of predicting risk of recurrence or progression of cancer in an individual with cancer following treatment, the method comprising: assaying a biological sample obtained from the individual for expression of at least two genes, or proteins encoded by said genes, selected from TTC21B, MCM3, MTUS1, FOXM1, CUL4A, GPC1, PCNA, IRF7, BRIP1, ULK1, and SPAG5, wherein when a combination of a normalised expression value of the at least two genes, or proteins encoded by said genes, is modified relative to a combination of a normalised expression value of the at least two genes from a biological sample obtained from a subject following treatment with no recurrence or progression of cancer, the individual is predicted to have an increased risk of recurrence of cancer or risk of progression of cancer following the treatment.

In one aspect, the treatment is selected from active surveillance, surgery and/or radiotherapy, neoadjuvant therapy, adjuvant systemic therapy, or a combination thereof.

A method of determining whether cancer in an individual with cancer is an aggressive cancer type, the method comprising: assaying a biological sample from the individual for expression of at least two genes, or proteins encoded by said genes, selected from TTC21B, MCM3, MTUS1, FOXM1, CUL4A, GPC1, PCNA, IRF7, BRIP1, ULK1, and SPAG5, wherein when a combination of a normalised expression value of the at least two genes, or proteins encoded by said genes, is modified relative to a combination of a normalised gene expression level of the at least two genes from a subject without an aggressive cancer, the individual is predicted to have an increased risk of having an aggressive cancer type.

A method of determining a 5-year survival rate or a 10-year survival rate of an individual diagnosed with cancer, the method comprising: assaying a biological sample from the individual for expression of at least two genes, or proteins encoded by those genes, selected from TTC21B, MCM3, MTUS1, FOXM1, CUL4A, GPC1, PCNA, IRF7, BRIP1, ULK1, and SPAG5, wherein when a combination of a normalised expression value of the at least two genes, or proteins encoded by said genes, is modified relative to a combination of a normalised expression value of the at least two genes from a biological sample obtained from a subject who survived the cancer after 5 or 10 years following cancer diagnosis, the individual is predicted to have a decreased 5-year survival rate or a 10-year survival rate.

A method of determining a 5-year survival rate or a 10-year survival rate of an individual diagnosed with prostate cancer, the method comprising: assaying a biological sample from the individual for expression of at least two genes, or proteins encoded by those genes, selected from TTC21B, MCM3, MTUS1, FOXM1, CUL4A, GPC1, PCNA, IRF7, BRIP1, ULK1, and SPAG5, wherein when a combination of a normalised expression value of the at least two genes, or proteins encoded by said genes, is modified relative to a combination of a normalised expression value of the at least two genes from a biological sample obtained from a subject who survived the prostate cancer after 5 or 10 years following cancer diagnosis, the individual is predicted to have a decreased 5-year survival rate or a 10-year survival rate.

A method of identifying an individual that is suitable for treatment with a therapy for preventing recurrence or progression of the cancer, the method comprising: assaying a biological sample from the individual for expression of at least two genes, or proteins encoded by those genes, selected from TTC21B, MCM3, MTUS1, FOXM1, CUL4A, GPC1, PCNA, IRF7, BRIP1, ULK1, and SPAG5, wherein when a combination of a normalised expression value of the at least two genes, or proteins encoded by said genes, is the same or increased following administration of the therapy treatment relative to the combination of a normalised expression value of the at least two genes in a biological sample from the same individual prior to administration of the therapy treatment, the individual is predicted to not be suitable for the treatment. A different treatment should be considered and provided to the individual and assaying another biological sample from the individual following administration of the different treatment in the same manner as above to determine if the different treatment is suitable or not. If the combination of the normalised expression value of the at least two genes, or proteins encoded by said genes, is the decreased following administration of a treatment relative to the combination of a normalised expression value of the at least two genes in a biological sample from the same individual prior to administration of the treatment therapy, the individual is predicted to be suitable for the treatment.

A method for monitoring the effectiveness of treatment of cancer in an individual with cancer, the method comprising: assaying a biological sample from the individual for expression of at least two genes, or proteins encoded by those genes, selected from TTC21B, MCM3, MTUS1, FOXM1, CUL4A, GPC1, PCNA, IRF7, BRIP1, ULK1, and SPAG5, wherein when a combination of a normalised expression value of the at least two genes, or proteins encoded by said genes, is the same or increased following administration of the treatment relative to the combination of a normalised expression value of the at least two genes in a biological sample from the same individual prior to the administration of the treatment, the treatment is ineffective and the individual is predicted to have a poor outcome. In this instance, the treatment regimen can be stopped and the individual with cancer receiving treatment can be administered a new treatment regimen, or the treatment is stopped altogether. If the combination of the normalised expression value of the at least two genes, or proteins encoded by said genes, is the decreased following administration of a treatment relative to the combination of a normalised expression value of the at least two genes in a biological sample from the same individual prior to administration of the treatment, the treatment is effective and the individual is predicted to have an improved outcome.

A method of predicting risk of an aggressive or a recurrent cancer in an individual, the method comprising the step of assaying a biological sample obtained from the individual for expression of at least one gene, or a protein encoded by said gene, selected from TTC21B, FOXM1, PCNA, IRF7, ULK1, and SPAG5, wherein when a normalised expression value of the at least one gene, or protein encoded by said gene, is modified when compared to a normalised expression value of the at least one gene in a biological sample from a subject whose cancer is non-aggressive or indolent, the individual is predicted to have an increased risk of an aggressive or a recurrent cancer.

A method of predicting risk of an aggressive or a recurrent cancer in an individual, the method comprising a step of assaying a biological sample obtained from the individual for expression of at least one gene, or a protein encoded by said gene, selected from MCM3, MTUS1, GPC1, BRIP1, and CUL4A, wherein when a normalised expression value of the at least one gene, or protein encoded by said gene, is modified when compared to a normalised expression value of the at least one gene in a biological sample from a subject whose cancer is non-aggressive or indolent, the individual is predicted to have an increased risk of an aggressive or a recurrent cancer.

A method of predicting risk of an aggressive or a recurrent cancer in an individual, the method comprising: assaying a biological sample obtained from the individual for expression of at least two genes, or proteins encoded by said genes, selected from TTC21B, FOXM1, PCNA, IRF7, ULK1, and SPAG5, wherein when a combination of a normalised expression value of the at least two genes, or proteins encoded by said genes, is modified relative to a combination of a normalised expression value of the at least two genes from a subject with non-aggressive or non-recurrent cancer, the individual is predicted to have an increased risk of having an aggressive or a recurrent cancer.

A method of predicting risk of an aggressive or a recurrent cancer in an individual, the method comprising: assaying a biological sample obtained from the individual for expression of at least two genes, or proteins encoded by said genes, selected from MCM3, MTUS1, BRIP1, CUL4A, and GPC1,; wherein when a combination of a normalised expression value of the at least two genes, or proteins encoded by said genes, is modified relative to a combination of a normalised expression value of the at least two genes from a subject with non-aggressive or non-recurrent cancer, the individual is predicted to have an increased risk of having an aggressive or a recurrent cancer.

A method of predicting risk of an aggressive or a recurrent cancer in an individual, the method comprising the step of assaying a biological sample obtained from the individual for expression of a TTC21B gene or protein encoded by said gene, wherein when a normalised expression value of TTC21B gene, or the protein encoded by said gene, is modified relative to a normalised expression value of the TTC21B gene in a sample from an individual whose cancer is non-aggressive or indolent, with the individual is predicted to have an increased risk of an aggressive or a recurrent cancer.

A method of predicting risk of an aggressive or a recurrent cancer in an individual, the method comprising the step of assaying a biological sample obtained from the individual for expression of a MTUS1 gene or protein encoded by said gene, when a normalised expression value of MTUS1 gene, or the protein encoded by said gene, is modified relative to a normalised expression value of the MTUS1 gene in a sample from an individual who has cancer, with the individual is predicted to have an increased risk of an aggressive or a recurrent cancer.

In one embodiment, the at least one gene is CUL4A. In one embodiment, the at least one gene is TTC21B. In one embodiment, the at least one gene is MCM3. In one embodiment, the at least one gene is GPC1. In one embodiment, the at least one gene is PCNA. In one embodiment, the at least one gene is MTUS1. In one embodiment, the at least one gene is IRF7. In one embodiment, the at least one gene is BRIP1. In one embodiment, the at least one gene is ULK1. In one embodiment, the at least one gene is SPAG5. In one embodiment, the at least one gene is FOXM1.

In one embodiment, the at least two genes selected are CUL4A and TTC21B. In one embodiment, the at least two genes selected are CUL4A and MCM3. In one embodiment, the at least two genes selected are CUL4A and GPC1. In one embodiment, the at least two genes selected are CUL4A and PCNA. In one embodiment, the at least two genes selected are CUL4A and MTUS1. In one embodiment, the at least two genes selected are CUL4A and IRF7. In one embodiment, the at least two genes selected are CUL4A and BRIP1. In one embodiment, the at least two genes selected are CUL4A and ULK1. In one embodiment, the at least two genes selected are CUL4A and SPAG5. In one embodiment, the at least two genes selected are TTC21B and MCM3. In one embodiment, the at least two genes selected are TTC21B and GPC1. In one embodiment, the at least two genes selected are TTC21B and PCNA. In one embodiment, the at least two genes selected are TTC21B and MUTS1. In one embodiment, the at least two genes selected are TTC21B and IRF7. In one embodiment, the at least two genes selected are TTC21B and BRIP1. In one embodiment, the at least two genes selected are TTC21B and ULK1. In one embodiment, the at least two genes selected are TTC21B and SPAG5. In one embodiment, the at least two genes selected are MCM3 and GPC1. In one embodiment, the at least two genes selected are MCM3 and PCNA. In one embodiment, the at least two genes selected are MCM3 and MTUS1. In one embodiment, the at least two genes selected are MCM3 and IRF7. In one embodiment, the at least two genes selected are MCM3 and BRIP1. In one embodiment, the at least two genes selected are MCM3 and UKL1. In one embodiment, the at least two genes selected are MCM3 and SPAG5. In one embodiment, the at least two genes selected are GPC1 and PCNA. In one embodiment, the at least two genes selected are GPC1 and MTUS1. In one embodiment, the at least two genes selected GPC1 and IRF7. In one embodiment, the at least two genes selected are GPC1 and BRIP1. In one embodiment, the at least two genes selected are GPC1 and ULK1. In one embodiment, the at least two genes selected are GPC1 and SPAG5. In one embodiment, the at least two genes selected are PCNA and MTUS1. In one embodiment, the at least two genes selected are PCNA and IRF7. In one embodiment, the at least two genes selected are PCNA and BRIP1. In one embodiment, the at least two genes selected are PCNA and ULK1. In one embodiment, the at least two genes selected are PCNA and SPAG5. In one embodiment, the at least two genes selected are MTUS1 and IRF7. In one embodiment, the at least two genes selected are MTUS1 and BRIP1. In one embodiment, the at least two genes selected are MTUS1 and ULK1. In one embodiment, the at least two genes selected are MTUS1 and SPAG5. In one embodiment, the at least two genes selected are IRF7 and BRIP1. In one embodiment, the at least two genes selected are IRF7 and ULK1. In one embodiment, the at least two genes selected are IRF7 and SPAG5. In one embodiment, the at least two genes selected are BRIP1 and ULK1. In one embodiment, the at least two genes selected are BRIP1 and SPAG5. In one embodiment, the at least two genes selected are ULK1 and SPAG5. In one embodiment, the at least two genes selected are CUL4A and FOXM1. In one embodiment, the at least two genes selected are FOXM1 and TTC21B. In one embodiment, the at least two genes selected are FOXM1 and MCM3. In one embodiment, the at least two genes selected are FOXM1 and GPC1. In one embodiment, the at least two genes selected are FOXM1 and PCNA. In one embodiment, the at least two genes selected are FOXM1 and MTUS1. In one embodiment, the at least two genes selected are FOXM1 and IRF7. In one embodiment, the at least two genes selected are FOXM1 and BRIP1. In one embodiment, the at least two genes selected are FOXM1 and ULK1. In one embodiment, the at least two genes selected are FOXM1 and SPAG5.

In one embodiment, at least three genes are selected, and the genes selected are CUL4A, TTC21B and MCM3. In one embodiment, the at least three genes selected are CUL4A, TTC21B and GPC1. In one embodiment, the at least three genes selected are CUL4A, TTC21B and PCNA. In one embodiment, the at least three genes selected are CUL4A, TTC21B and MTUS1. In one embodiment, the at least three genes selected are CUL4A, TTC21B and IRF7. In one embodiment, the at least three genes selected are CUL4A, TTC21B and BRIP1. In one embodiment, the at least three genes selected are CUL4A, TTC21B and ULK1. In one embodiment, the at least three genes selected are CUL4A, TTC21B and SPAG5. In one embodiment, the at least three genes selected are TTC21B, MCM3, and GPC1. In one embodiment, the at least three genes selected are TTC21B, MCM3, and PCNA. In one embodiment, the at least three genes selected are TTC21B, MCM3, and MTUS1. In one embodiment, the at least three genes selected are TTC21B, MCM3, and IRF7. In one embodiment, the at least three genes selected are TTC21B, MCM3, and BRIP1. In one embodiment, the at least three genes selected are TTC21B, MCM3, and ULK1. In one embodiment, the at least three genes selected are TTC21B, MCM3, and SPAG5. In one embodiment, the at least three genes selected are MCM3, GPC1, and PCNA In one embodiment, the at least three genes selected are MCM3, GPC1, and MTUS1. In one embodiment, the at least three genes selected are MCM3, GPC1, and IRF7. In one embodiment, the at least three genes selected are MCM3, GPC1, and BRIP1. In one embodiment, the at least three genes selected are MCM3, GPC1, and ULK1. In one embodiment, the at least three genes selected are MCM3, GPC1, and SPAG5. In one embodiment, the at least three genes selected are GPC1, PCNA, and MTUS1. In one embodiment, the at least three genes selected GPC1, PCNA, and MTUS1. In one embodiment, the at least three genes selected are GPC1, PCNA, and IRF7. In one embodiment, the at least three genes selected are GPC1, PCNA, and BRIP1. In one embodiment, the at least three genes selected are GPC1, PCNA, and ULK1. In one embodiment, the at least three genes selected are GPC1, PCNA, and SPAG5. In one embodiment, the at least three genes selected are PCNA, MTUS1, and IRF7. In one embodiment, the at least three genes selected are PCNA, MTUS1, and BRIP1. In one embodiment, the at least three genes selected are PCNA, MTUS1, and ULK1. In one embodiment, the at least three genes selected are PCNA, MTUS1, and SPAG5. In one embodiment, the at least three genes selected are MTUS1, IRF7, and BRIP1. In one embodiment, the at least three genes selected are MTUS1, IRF7, and ULK1. In one embodiment, the at least three genes selected are MTUS1, IRF7, and SPAG5. In one embodiment, the at least three genes selected are IRF7, BRIP1 and ULK1. In one embodiment, the at least three genes selected are IRF7, BRIP1 and SPAG5. In one embodiment, the at least three genes selected are BRIP1, ULK1, and SPAG5. In one embodiment, the at least three genes selected are CUL4, MCM3, and GPC1. In one embodiment, the at least three genes selected are CUL4, MCM3, and PCNA. In one embodiment, the at least three genes selected are CUL4, MCM3, and MTUS1. In one embodiment, the at least three genes selected are CUL4, MCM3, and IRF7. In one embodiment, the at least three genes selected are CUL4, MCM3, and BRIP1. In one embodiment, the at least three genes selected are CUL4, MCM3, and ULK1. In one embodiment, the at least three genes selected are CUL4, MCM3, and SPAG5. In one embodiment, the at least three genes selected are CUL4, GPC1 and PCNA. In one embodiment, the at least three genes selected are CUL4, GPC1 and MTUS1. In one embodiment, the at least three genes selected are CUL4, GPC1 and IRF7. In one embodiment, the at least three genes selected are CUL4, GPC1 and BRIP1. In one embodiment, the at least three genes selected are CUL4, GPC1 and ULK1. In one embodiment, the at least three genes selected are CUL4, GPC1 and SPAG5. In one embodiment, the at least three genes selected are CUL4, PCNA and MTUS1. In one embodiment, the at least three genes selected are CUL4, PCNA and IRF7. In one embodiment, the at least three genes selected are CUL4, PCNA and BRIP1. In one embodiment, the at least three genes selected are CUL4, PCNA and ULK1. In one embodiment, the at least three genes selected are CUL4, PCNA and SPAG5. In one embodiment, the at least three genes selected are CUL4, MTUS1 and IRF7. In one embodiment, the at least three genes selected are CUL4, MTUS1 and BRIP1. In one embodiment, the at least three genes selected are CUL4, MTUS1 and ULK1. In one embodiment, the at least three genes selected are CUL4, MTUS1 and SPAG5. In one embodiment, the at least three genes selected are CUL4, IRF7 and BRIP1. In one embodiment, the at least three genes selected are CUL4, IRF7 and ULK1. In one embodiment, the at least three genes selected are CUL4, IRF7 and SPAG5. In one embodiment, the at least three genes selected are CUL4, BRIP1, and ULK1. In one embodiment, the at least three genes selected are CUL4, BRIP1, and SPAG5. In one embodiment, the at least three genes selected are CUL4, ULK1, and SPAG5. In one embodiment, the at least three genes selected are TTC21B, GPC1, and PCNA. In one embodiment, the at least three genes selected are TTC21B, GPC1, and PCNA. In one embodiment, the at least three genes selected are TTC21B, GPC1, and MTUS1. In one embodiment, the at least three genes selected are TTC21B, GPC1, and IRF7. In one embodiment, the at least three genes selected are TTC21B, GPC1, and BRIP1. In one embodiment, the at least three genes selected are TTC21B, GPC1, and ULK1. In one embodiment, the at least three genes selected are TTC21B, GPC1, and SPAG5. In one embodiment, the at least three genes selected are TTC21B, PCNA, and MTUS1. In one embodiment, the at least three genes selected are TTC21B, PCNA, and IRF7. In one embodiment, the at least three genes selected are TTC21B, PCNA, and BRIP1. In one embodiment, the at least three genes selected are TTC21B, PCNA, and ULK1. In one embodiment, the at least three genes selected are TTC21B, PCNA, and SPAG5. In one embodiment, the at least three genes selected are TTC21B, MTUS1, and IRF7. In one embodiment, the at least three genes selected are TTC21B, MTUS1, and BRIP1. In one embodiment, the at least three genes selected are TTC21B, MTUS1, and ULK1. In one embodiment, the at least three genes selected are TTC21B, MTUS1, and SPAG5. In one embodiment, the at least three genes selected are TTC21B, IRF7, and BRIP1. In one embodiment, the at least three genes selected are TTC21B, IRF7, and ULK1. In one embodiment, the at least three genes selected are TTC21B, IRF7, and SPAG5. In one embodiment, the at least three genes selected are TTC21B, BRIP1, and ULK1. In one embodiment, the at least three genes selected are TTC21B, BRIP1, and SPAG5. In one embodiment, the at least three genes selected are TTC21B, ULK1, and SPAG5. In one embodiment, the at least three genes selected are MCM3, PCNA, and MTUS1. In one embodiment, the at least three genes selected are MCM3, PCNA, and IRF7. In one embodiment, the at least three genes selected are MCM3, PCNA, and BRIP1. In one embodiment, the at least three genes selected are MCM3, PCNA, and ULK1. In one embodiment, the at least three genes selected are MCM3, PCNA, and SPAG5. In one embodiment, the at least three genes selected are MCM3, MTUS1, and IRF7. In one embodiment, the at least three genes selected are MCM3, MTUS1, and BRIP1. In one embodiment, the at least three genes selected are MCM3, MTUS1, and ULK1. In one embodiment, the at least three genes selected are MCM3, MTUS1, and SPAG5. In one embodiment, the at least three genes selected are MCM3, IRF7, and BRIPS. In one embodiment, the at least three genes selected are MCM3, IRF7, and ULK1. In one embodiment, the at least three genes selected are MCM3, IRF7, and SPAG5. In one embodiment, the at least three genes selected are MCM3, BRIP1, and ULK1. In one embodiment, the at least three genes selected are MCM3, BRIP1, and SPAG5. In one embodiment, the at least three genes selected are MCM3, ULK1, and SPAG5. In one embodiment, the at least three genes selected are GPC1, MTUS1, and IRF7. In one embodiment, the at least three genes selected are GPC1, MTUS1, and BRIP1. In one embodiment, the at least three genes selected are GPC1, MTUS1, and ULK1. In one embodiment, the at least three genes selected are GPC1, MTUS1, and SPAG5. In one embodiment, the at least three genes selected are GPC1, IRF7, and BRIP1. In one embodiment, the at least three genes selected are GPC1, IRF7, and ULK1. In one embodiment, the at least three genes selected are GPC1, IRF7, and SPAG5. In one embodiment, the at least three genes selected GPC1, BRIP1, and ULK1. In one embodiment, the at least three genes selected are GPC1, BRIP1, and SPAG5. In one embodiment, the at least three genes selected are GPC1, ULK1 and SPAG5. In one embodiment, the at least three genes selected are PCNA, IRF7, and BRIP1. In one embodiment, the at least three genes selected are PCNA, IRF7, and ULK1. In one embodiment, the at least three genes selected are PCNA, IRF7, and SPAG5. In one embodiment, the at least three genes selected are PCNA, BRIP1, and ULK1. In one embodiment, the at least three genes selected are PCNA, BRIP1, and SPAG5. In one embodiment, the at least three genes selected are PCNA, ULK1, and SPAG5. In one embodiment, the at least three genes selected are MTUS1, BRIP1, and UL1. In one embodiment, the at least three genes selected are MTUS1, BRIP1, and SPAG5. In one embodiment, the at least three genes selected are MTUS1, ULK1, and SPAG5. In one embodiment, the at least three genes selected are IRF7, ULK1, and SPAG5. In one embodiment, at least three genes are selected, and the genes selected are FOXM1, TTC21B and CUL4A. In one embodiment, at least three genes are selected, and the genes selected are FOXM1, TTC21B and MCM3. In one embodiment, the at least three genes selected are FOXM1, TTC21B and GPC1. In one embodiment, the at least three genes selected are FOXM1, TTC21B and PCNA. In one embodiment, the at least three genes selected are FOXM1, TTC21B and MTUS1. In one embodiment, the at least three genes selected are FOXM1, TTC21B and IRF7. In one embodiment, the at least three genes selected are FOXM1, TTC21B and BRIP1. In one embodiment, the at least three genes selected are FOXM1, TTC21B and ULK1. In one embodiment, the at least three genes selected are FOXM1, TTC21B and SPAG5. In one embodiment, the at least three genes selected are CUL4, MCM3, and GPC1. In one embodiment, the at least three genes selected are FOXM1, MCM3, and CUL4. In one embodiment, the at least three genes selected are FOXM1, MCM3, and PCNA. In one embodiment, the at least three genes selected are FOXM1, MCM3, and MTUS1. In one embodiment, the at least three genes selected are FOXM1, MCM3, and IRF7. In one embodiment, the at least three genes selected are FOXM1, MCM3, and BRIP1. In one embodiment, the at least three genes selected are FOXM1, MCM3, and ULK1. In one embodiment, the at least three genes selected are FOXM1, MCM3, and SPAG5. In one embodiment, the at least three genes selected are FOXM1, GPC1 and PCNA. In one embodiment, the at least three genes selected are FOXM1, GPC1 and MTUS1. In one embodiment, the at least three genes selected are FOXM1, GPC1 and IRF7. In one embodiment, the at least three genes selected are FOXM1, GPC1 and BRIP1. In one embodiment, the at least three genes selected are FOXM1, GPC1 and ULK1. In one embodiment, the at least three genes selected are FOXM1, GPC1 and SPAG5. In one embodiment, the at least three genes selected are FOXM1, PCNA and MTUS1. In one embodiment, the at least three genes selected are FOXM1, PCNA and IRF7. In one embodiment, the at least three genes selected are FOXM1, PCNA and BRIP1. In one embodiment, the at least three genes selected are FOXM1, PCNA and ULK1. In one embodiment, the at least three genes selected are FOXM1, PCNA and SPAG5. In one embodiment, the at least three genes selected are FOXM1, MTUS1 and IRF7. In one embodiment, the at least three genes selected are FOXM1, MTUS1 and BRIP1. In one embodiment, the at least three genes selected are FOXM1, MTUS1 and ULK1. In one embodiment, the at least three genes selected are FOXM1, MTUS1 and SPAG5. In one embodiment, the at least three genes selected are FOXM1, IRF7 and BRIP1. In one embodiment, the at least three genes selected are FOXM1, IRF7 and ULK1. In one embodiment, the at least three genes selected are FOXM1, IRF7 and SPAG5. In one embodiment, the at least three genes selected are FOXM1, BRIP1, and ULK1. In one embodiment, the at least three genes selected are FOXM1, BRIP1, and SPAG5. In one embodiment, the at least three genes selected are FOXM1, ULK1, and SPAG5.

In one embodiment, at least four genes are selected and the at least four genes selected are CUL4A, TTC21B, MCM3, and GPC1. In one embodiment, the at least four genes selected are CUL4A, TTC21B, MCM3, and PCNA. In one embodiment, the at least four genes selected are CUL4A, TTC21B, MCM3, and MTUS1. In one embodiment, the at least four genes selected are CUL4A, TTC21B, MCM3, and IRF7. In one embodiment, the at least four genes selected are CUL4A, TTC21B, MCM3, and BRIP1. In one embodiment, the at least four genes selected are CUL4A, TTC21B, MCM3, and ULK1. In one embodiment, the at least four genes selected are CUL4A, TTC21B, MCM3, and SPAG5. In one embodiment, the at least four genes selected are CUL4A, TTC21B, GPC1, and PCNA. In one embodiment, the at least four genes selected are CUL4A, TTC21B, GPC1, and MTUS1. In one embodiment, the at least four genes selected are CUL4A, TTC21B, GPC1, and IRF7. In one embodiment, the at least four genes selected are CUL4A, TTC21B, GPC1, and BRIP1. In one embodiment, the at least four genes selected are CUL4A, TTC21B, GPC1, and ULK1. In one embodiment, the at least four genes selected are CUL4A, TTC21B, GPC1, and SPAG5. In one embodiment, the at least four genes selected are CUL4A, TTC21B, PCNA, and MTUS1. In one embodiment, the at least four genes selected are CUL4A, TTC21B, PCNA, and IRF7. In one embodiment, the at least four genes selected are CUL4A, TTC21B, PCNA, and BRIP1. In one embodiment, the at least four genes selected are CUL4A, TTC21B, PCNA, and ULK1. In one embodiment, the at least four genes selected are CUL4A, TTC21B, PCNA, and SPAG5. In one embodiment, the at least four genes selected are CUL4A, TTC21B, MTUS1, and IRF7. In one embodiment, the at least four genes selected are CUL4A, TTC21B, MTUS1, and BRIP1. In one embodiment, the at least four genes selected are CUL4A, TTC21B, MTUS1, and ULK1. In one embodiment, the at least four genes selected are CUL4A, TTC21B, MTUS1, and SPAG5. In one embodiment, the at least four genes selected are CUL4A, TTC21B, IRF7, and BRIP1. In one embodiment, the at least four genes selected are CUL4A, TTC21B, IRF7, and ULK1. In one embodiment, the at least four genes selected are CUL4A, TTC21B, IRF7, and SPAG5. In one embodiment, the at least four genes selected are CUL4A, TTC21B, BRIP1, and ULK1. In one embodiment, the at least four genes selected are CUL4A, TTC21B, BRIP1, and SPAG5. In one embodiment, the at least four genes selected are CUL4A, TTC21B, ULK1, and SPAG5. In one embodiment, the at least four genes selected are CUL4A, MCM3, GPC1, and PCNA. In one embodiment, the at least four genes selected are CUL4A, MCM3, GPC1, and MTUS1. In one embodiment, the at least four genes selected are CUL4A, MCM3, GPC1, and IRF7. In one embodiment, the at least four genes selected are CUL4A, MCM3, GPC1, and BRIP1. In one embodiment, the at least four genes selected are CUL4A, MCM3, GPC1, and ULK1. In one embodiment, the at least four genes selected are CUL4A, MCM3, GPC1, and SPAG5. In one embodiment, the at least four genes selected are CUL4A, MCM3, PCNA, and MTUS1. In one embodiment, the at least four genes selected are CUL4A, MCM3, PCNA, and IRF7. In one embodiment, the at least four genes selected are CUL4A, MCM3, PCNA, and BRIP1. In one embodiment, the at least four genes selected are CUL4A, MCM3, PCNA, and ULK1. In one embodiment, the at least four genes selected are CUL4A, MCM3, PCNA, and SPAG5. In one embodiment, the at least four genes selected are CUL4A, MCM3, MTUS1, and IRF7. In one embodiment, the at least four genes selected are CUL4A, MCM3, MTUS1, and BRIP1. In one embodiment, the at least four genes selected are CUL4A, MCM3, MTUS1, and ULK1. In one embodiment, the at least four genes selected are CUL4A, MCM3, MTUS1, and SPAG5. In one embodiment, the at least four genes selected are CUL4A, MCM3, IRF7, and BRIP1. In one embodiment, the at least four genes selected are CUL4A, MCM3, IRF7, and ULK1. In one embodiment, the at least four genes selected are CUL4A, MCM3, IRF7, and SPAG5. In one embodiment, the at least four genes selected are CUL4A, MCM3, BRIP1, and ULK1. In one embodiment, the at least four genes selected are CUL4A, MCM3, BRIP1, and SPAG5. In one embodiment, the at least four genes selected are CUL4A, MCM3, ULK1, and SPAG5. In one embodiment, the at least four genes selected are CUL4A, IRF7, BRIP1, and ULK1. In one embodiment, the at least four genes selected are CUL4A, IRF7, BRIP1, and SPAG5. In one embodiment, the at least four genes selected are CUL4A, BRIP1, ULK 1 and SPAG5. In one embodiment, the at least four genes selected are TTC21B, MCM3, GPC1, and PCNA. In one embodiment, the at least four genes selected are TTC21B, MCM3, GPC1, and MTUS1. In one embodiment, the at least four genes selected are TTC21B, MCM3, GPC1, and IRF7. In one embodiment, the at least four genes selected are TTC21B, MCM3, GPC1, and BRIP1. In one embodiment, the at least four genes selected are TTC21B, MCM3, GPC1, and ULK1. In one embodiment, the at least four genes selected are TTC21B, MCM3, GPC1, and SPAG5. In one embodiment, the at least four genes selected are TTC21B, MCM3, PCNA, and MTUS1. In one embodiment, the at least four genes selected are TTC21B, MCM3, PCNA, and IRF7. In one embodiment, the at least four genes selected are TTC21B, MCM3, PCNA, and BRIP1. In one embodiment, the at least four genes selected are TTC21B, MCM3, PCNA, and ULK1. In one embodiment, the at least four genes selected are TTC21B, MCM3, PCNA, and SPAG5. In one embodiment, the at least four genes selected are TTC21B, MCM3, MTUS1, and IRF7. In one embodiment, the at least four genes selected are TTC21B, MCM3, MTUS1, and BRIP1. In one embodiment, the at least four genes selected are TTC21B, MCM3, MTUS1, and ULK1. In one embodiment, the at least four genes selected are TTC21B, MCM3, MTUS1, and SPAG5. In one embodiment, the at least four genes selected are TTC21B, MCM3, IRF7, and BRIP1. In one embodiment, the at least four genes selected are TTC21B, MCM3, IRF7, and ULK. In one embodiment, the at least four genes selected are TTC21B, MCM3, IRF7, and SPAG5. In one embodiment, the at least four genes selected are TTC21B, MCM3, BRIP1, and ULK1. In one embodiment, the at least four genes selected are TTC21B, MCM3, IRF7, and SPAG5. In one embodiment, the at least four genes selected are TTC21B, MCM3, BRIP1, and ULK1. In one embodiment, the at least four genes selected are TTC21B, MCM3, IRF7, and SPAG5. In one embodiment, the at least four genes selected are TTC21B, MCM3, ULK1, and SPAG5. In one embodiment, the at least four genes selected are TTC21B, GPC1, PCNA, and MTUS1. In one embodiment, the at least four genes selected are TTC21B, GPC1, PCNA, and IRF7. In one embodiment, the at least four genes selected are TTC21B, GPC1, PCNA, and BRIP1. In one embodiment, the at least four genes selected are TTC21B, GPC1, PCNA, and ULK1. In one embodiment, the at least four genes selected are TTC21B, GPC1, PCNA, and SPAG5. In one embodiment, the at least four genes selected are TTC21B, PCNA, MTUS1, and IRF7. In one embodiment, the at least four genes selected are TTC21B, PCNA, MTUS1, and BRIP1. In one embodiment, the at least four genes selected are TTC21B, PCNA, MTUS1, and ULK1. In one embodiment, the at least four genes selected are TTC21B, PCNA, MTUS1, and SPAG5. In one embodiment, the at least four genes selected are TTC21B, MTUS1, IRF7, and BRIP1. In one embodiment, the at least four genes selected are TTC21B, MTUS1, IRF7, and ULK1. In one embodiment, the at least four genes selected are TTC21B, MTUS1, IRF7, and SPAG5. In one embodiment, the at least four genes selected are TTC21B, IRF7, BRIP1, and ULK1. In one embodiment, the at least four genes selected are TTC21B, IRF7, BRIP1, and SPAG5. In one embodiment, the at least four genes selected are MCM3, GPC1, PCNA, and MTUS1. In one embodiment, the at least four genes selected are MCM3, GPC1, PCNA, and IRF7. In one embodiment, the at least four genes selected are MCM3, GPC1, PCNA, and BRIP1. In one embodiment, the at least four genes selected are MCM3, GPC1, PCNA, and ULK1. In one embodiment, the at least four genes selected are MCM3, GPC1, PCNA, and SPAG5. In one embodiment, the at least four genes selected are MCM3, PCNA, MTUS1, and IRF7. In one embodiment, the at least four genes selected are MCM3, PCNA, MTUS1, and BRIP1. In one embodiment, the at least four genes selected are MCM3, PCNA, MTUS1, and ULK1. In one embodiment, the at least four genes selected are MCM3, PCNA, MTUS1, and SPAG5. In one embodiment, the at least four genes selected are MCM3, MTUS1, IRF7 and BRIP1. In one embodiment, the at least four genes selected are MCM3, MTUS1, IRF7 and ULK1. In one embodiment, the at least four genes selected are MCM3, MTUS1, IRF7 and SPAG5. In one embodiment, the at least four genes selected are MCM3, IRF7, BRIP1 and ULK1. In one embodiment, the at least four genes selected are MCM3, IRF7, BRIP1 and SPAG5. In one embodiment, the at least four genes selected are MCM3, BRIP1, ULK1, and SPAG5. In one embodiment, the at least four genes selected are GPC1, PCNA, MTUS1, and IRF7. In one embodiment, the at least four genes selected are GPC1, PCNA, MTUS1, and BRIP1. In one embodiment, the at least four genes selected are GPC1, PCNA, MTUS1, and ULK1. In one embodiment, the at least four genes selected are GPC1, PCNA, MTUS1, and SPAG5. In one embodiment, the at least four genes selected are GPC1, MTUS1, IRF7, and BRIP1. In one embodiment, the at least four genes selected are GPC1, MTUS1, IRF7, and ULK1. In one embodiment, the at least four genes selected are GPC1, MTUS1, IRF7, and SPAG5. In one embodiment, the at least four genes selected are GPC1, IRF7, BRIP1, and ULK1. In one embodiment, the at least four genes selected are GPC1, IRF7, BRIP1, and SPAG5. In one embodiment, the at least four genes selected are GPC1, BRIP1, ULK1, and SPAG5. In one embodiment, the at least four genes selected are PCNA, MTUS1, BRIP1, and ULK1. In one embodiment, the at least four genes selected are PCNA, MTUS1, BRIP1, and SPAG5. In one embodiment, the at least four genes selected are PCNA, BRIP1, ULK1, and SPAG5. In one embodiment, the at least four genes selected are MTUS1, BRIP1, ULK1, and SPAG5. In one embodiment, the at least four genes selected are MTUS1, BRIP1, ULK1, and SPAG5. In one embodiment, at least four genes are selected and the at least four genes selected are FOXM1, TTC21B, MCM3, and CUL4A. In one embodiment, at least four genes are selected and the at least four genes selected are FOXM1, TTC21B, MCM3, and GPC1. In one embodiment, the at least four genes selected are FOXM1, TTC21B, MCM3, and PCNA. In one embodiment, the at least four genes selected are FOXM1, TTC21B, MCM3, and MTUS1. In one embodiment, the at least four genes selected are FOXM1, TTC21B, MCM3, and IRF7. In one embodiment, the at least four genes selected are FOXM1, TTC21B, MCM3, and BRIP1. In one embodiment, the at least four genes selected are FOXM1, TTC21B, MCM3, and ULK1. In one embodiment, the at least four genes selected are FOXM1, TTC21B, MCM3, and SPAG5. In one embodiment, the at least four genes selected are FOXM1, TTC21B, GPC1, and PCNA. In one embodiment, the at least four genes selected are FOXM1, TTC21B, GPC1, and MTUS1. In one embodiment, the at least four genes selected are FOXM1, TTC21B, GPC1, and IRF7. In one embodiment, the at least four genes selected are FOXM1, TTC21B, GPC1, and BRIP1. In one embodiment, the at least four genes selected are FOXM1, TTC21B, GPC1, and ULK1. In one embodiment, the at least four genes selected are FOXM1, TTC21B, GPC1, and SPAG5. In one embodiment, the at least four genes selected are FOXM1, TTC21B, PCNA, and MTUS1. In one embodiment, the at least four genes selected are FOXM1, TTC21B, PCNA, and IRF7. In one embodiment, the at least four genes selected are FOXM1, TTC21B, PCNA, and BRIP1. In one embodiment, the at least four genes selected are FOXM1, TTC21B, PCNA, and ULK1. In one embodiment, the at least four genes selected are FOXM1, TTC21B, PCNA, and SPAG5. In one embodiment, the at least four genes selected are FOXM1, TTC21B, MTUS1, and IRF7. In one embodiment, the at least four genes selected are FOXM1, TTC21B, MTUS1, and BRIP1. In one embodiment, the at least four genes selected are FOXM1, TTC21B, MTUS1, and ULK1. In one embodiment, the at least four genes selected are FOXM1, TTC21B, MTUS1, and SPAG5. In one embodiment, the at least four genes selected are FOXM1, TTC21B, IRF7, and BRIP1. In one embodiment, the at least four genes selected are FOXM1, TTC21B, IRF7, and ULK1. In one embodiment, the at least four genes selected are FOXM1, TTC21B, IRF7, and SPAG5. In one embodiment, the at least four genes selected are FOXM1, TTC21B, BRIP1, and ULK1. In one embodiment, the at least four genes selected are FOXM1, TTC21B, BRIP1, and SPAG5. In one embodiment, the at least four genes selected are FOXM1, TTC21B, ULK1, and SPAG5. In one embodiment, the at least four genes selected are FOXM1, MCM3, GPC1, and PCNA. In one embodiment, the at least four genes selected are FOXM1, MCM3, GPC1, and MTUS1. In one embodiment, the at least four genes selected are FOXM1, MCM3, GPC1, and IRF7. In one embodiment, the at least four genes selected are FOXM1, MCM3, GPC1, and BRIP1. In one embodiment, the at least four genes selected are FOXM1, MCM3, GPC1, and ULK1. In one embodiment, the at least four genes selected are FOXM1, MCM3, GPC1, and SPAG5. In one embodiment, the at least four genes selected are FOXM1, MCM3, PCNA, and MTUS1. In one embodiment, the at least four genes selected are FOXM1, MCM3, PCNA, and IRF7. In one embodiment, the at least four genes selected are FOXM1, MCM3, PCNA, and BRIP1. In one embodiment, the at least four genes selected are FOXM1, MCM3, PCNA, and ULK1. In one embodiment, the at least four genes selected are FOXM1, MCM3, PCNA, and SPAG5. In one embodiment, the at least four genes selected are FOXM1, MCM3, MTUS1, and IRF7. In one embodiment, the at least four genes selected are FOXM1, MCM3, MTUS1, and BRIP1. In one embodiment, the at least four genes selected are FOXM1, MCM3, MTUS1, and ULK1. In one embodiment, the at least four genes selected are FOXM1, MCM3, MTUS1, and SPAG5. In one embodiment, the at least four genes selected are FOXM1, MCM3, IRF7, and BRIP1. In one embodiment, the at least four genes selected are FOXM1, MCM3, IRF7, and ULK1. In one embodiment, the at least four genes selected are FOXM1, MCM3, IRF7, and SPAG5. In one embodiment, the at least four genes selected are FOXM1, MCM3, BRIP1, and ULK1. In one embodiment, the at least four genes selected are FOXM1, MCM3, BRIP1, and SPAG5. In one embodiment, the at least four genes selected are FOXM1, MCM3, ULK1, and SPAG5. In one embodiment, the at least four genes selected are FOXM1, IRF7, BRIP1, and ULK1. In one embodiment, the at least four genes selected are FOXM1, IRF7, BRIP1, and SPAG5. In one embodiment, the at least four genes selected are FOXM1, BRIP1, ULK 1 and SPAG5.

In one embodiment, at least five genes are selected, and the genes selected are CUL4, TTC21B, MCM3, GPC1, and PCNA. In one embodiment, the at least five genes selected are CUL4, TTC21B, MCM3, GPC1, and MTUS1. In one embodiment, the at least five genes selected are CUL4, TTC21B, MCM3, GPC1, and PCNA. In one embodiment, the at least five genes selected are CUL4, TTC21B, MCM3, GPC1, and MTUS1. In one embodiment, the at least five genes selected are CUL4, TTC21B, MCM3, GPC1, and IRF7. In one embodiment, the at least five genes selected are CUL4, TTC21B, MCM3, GPC1, and BRIP1. In one embodiment, the at least five genes selected are CUL4, TTC21B, MCM3, GPC1, and ULK1. In one embodiment, the at least five genes selected are CUL4, TTC21B, MCM3, GPC1, and SPAG5. In one embodiment, the at least five genes selected are CUL4, MCM3, GPC1, PCNA, and MTUS1. In one embodiment, the at least five genes selected are CUL4, MCM3, GPC1, PCNA, and IRF7. In one embodiment, the at least five genes selected are CUL4, MCM3, GPC1, PCNA, and BRIP1. In one embodiment, the at least five genes selected are CUL4, MCM3, GPC1, PCNA, and ULK1. In one embodiment, the at least five genes selected are CUL4, MCM3, GPC1, PCNA, and SPAG5. In one embodiment, the at least five genes selected are CUL4, GPC1, PCNA, MTUS1, and IRF7. In one embodiment, the at least five genes selected are CUL4, GPC1, PCNA, MTUS1, and BRIP1. In one embodiment, the at least five genes selected are CUL4, GPC1, PCNA, MTUS1, and ULK1. In one embodiment, the at least five genes selected are CUL4, GPC1, PCNA, MTUS1, and SPAG5. In one embodiment, the at least five genes selected are CUL4, PCNA, MTUS1, IRF7, and BRIP1. In one embodiment, the at least five genes selected are CUL4, PCNA, MTUS1, IRF7, and ULK1. In one embodiment, the at least five genes selected are CUL4, PCNA, MTUS1, IRF7, and SPAG5. In one embodiment, the at least five genes selected are CUL4, MTUS1, IRF7, BRIP1, and ULK1. In one embodiment, the at least five genes selected are CUL4, MTUS1, IRF7, BRIP1, and SPAG5. In one embodiment, the at least five genes selected are CUL4, IRF7, BRIP1, ULK1, and SPAG5. In one embodiment, the at least five genes selected are TTC21B, MCM3, GPC1, PCNA, and MTUS1. In one embodiment, the at least five genes selected are TTC21B, MCM3, GPC1, PCNA, and IRF7. In one embodiment, the at least five genes selected are TTC21B, MCM3, GPC1, PCNA, and BRIP1. In one embodiment, the at least five genes selected are TTC21B, MCM3, GPC1, PCNA, and ULK1. In one embodiment, the at least five genes selected are TTC21B, MCM3, GPC1, PCNA, and SPAG5. In one embodiment, the at least five genes selected are TTC21B, GPC1, PCNA, MTUS1, and IRF7. In one embodiment, the at least five genes selected are TTC21B, GPC1, PCNA, MTUS1, and BRIP1. In one embodiment, the at least five genes selected are TTC21B, GPC1, PCNA, MTUS1, and ULK1. In one embodiment, the at least five genes selected are TTC21B, GPC1, PCNA, MTUS1, and SPAG5. In one embodiment, the at least five genes selected are TTC21B, PCNA, MTUS1, IRF7, and BRIP1. In one embodiment, the at least five genes selected are TTC21B, PCNA, MTUS1, IRF7, and ULK1. In one embodiment, the at least five genes selected are TTC21B, PCNA, MTUS1, IRF7, and SPAG5. In one embodiment, the at least five genes selected are TTC21B, MTUS1, IRF7, BRIP1, and ULK1. In one embodiment, the at least five genes selected are TTC21B, MTUS1, IRF7, BRIP1, and SPAG5. In one embodiment, the at least five genes selected are TTC21B, IRF7, BRIP1, ULK1, and SPAG5. In one embodiment, the at least five genes selected are MCM3, GPC1, PCNA, MTUS1, and IRF7. In one embodiment, the at least five genes selected are MCM3, GPC1, PCNA, MTUS1, and IRF7. In one embodiment, the at least five genes selected are MCM3, GPC1, PCNA, MTUS1, and ULK1. In one embodiment, the at least five genes selected are MCM3, GPC1, PCNA, MTUS1, and SPAG5. In one embodiment, the at least five genes selected are MCM3, PCNA, MTUS1, IRF7, and BRIP1. In one embodiment, the at least five genes selected are MCM3, PCNA, MTUS1, IRF7, and ULK1. In one embodiment, the at least five genes selected are MCM3, PCNA, MTUS1, IRF7, and SPAG5. In one embodiment, the at least five genes selected are MCM3, MTUS1, IRF7, BRIP1, and ULK1. In one embodiment, the at least five genes selected are MCM3, MTUS1, IRF7, BRIP1, and SPAG5. In one embodiment, the at least five genes selected are MCM3, IRF7, BRIP1, ULK1, and SPAG5. In one embodiment, the at least five genes selected are GPC1, PCNA, MTUS1, IRF7, and BRIP1. In one embodiment, the at least five genes selected are GPC1, PCNA, MTUS1, IRF7, and ULK1. In one embodiment, the at least five genes selected are GPC1, PCNA, MTUS1, IRF7, and SPAG5. In one embodiment, the at least five genes selected are GPC1, MTUS1, IRF7, BRIP1, and ULK1. In one embodiment, the at least five genes selected are GPC1, MTUS1, IRF7, BRIP1, and SPAG5. In one embodiment, the at least five genes selected are GPC1, IRF7, BRIP1, ULK1, and SPAG5. In one embodiment, the at least five genes selected are PCNA, MTUS1, IRF7, BRIP1, and ULK1. In one embodiment, the at least five genes selected are PCNA, MTUS1, IRF7, BRIP1, and SPAG5. In one embodiment, the at least five genes selected are PCNA, IRF7, BRIP1, ULK1, and SPAG5. In one embodiment, the at least five genes selected are MTUS1, IRF7, BRIP1, ULK1, and SPAG5. In one embodiment, at least five genes are selected, and the genes selected are FOXM1, TTC21B, MCM3, GPC1, and CUL4. In one embodiment, at least five genes are selected, and the genes selected are FOXM1, TTC21B, MCM3, GPC1, and PCNA. In one embodiment, the at least five genes selected are FOXM1, TTC21B, MCM3, GPC1, and MTUS1. In one embodiment, the at least five genes selected are FOXM1, TTC21B, MCM3, GPC1, and PCNA. In one embodiment, the at least five genes selected are FOXM1, TTC21B, MCM3, GPC1, and MTUS1. In one embodiment, the at least five genes selected are FOXM1, TTC21B, MCM3, GPC1, and IRF7. In one embodiment, the at least five genes selected are FOXM1, TTC21B, MCM3, GPC1, and BRIP1. In one embodiment, the at least five genes selected are FOXM1, TTC21B, MCM3, GPC1, and ULK1. In one embodiment, the at least five genes selected are FOXM1, TTC21B, MCM3, GPC1, and SPAG5. In one embodiment, the at least five genes selected are FOXM1, MCM3, GPC1, PCNA, and MTUS1. In one embodiment, the at least five genes selected are FOXM1, MCM3, GPC1, PCNA, and IRF7. In one embodiment, the at least five genes selected are FOXM1, MCM3, GPC1, PCNA, and BRIP1. In one embodiment, the at least five genes selected are FOXM1, MCM3, GPC1, PCNA, and ULK1. In one embodiment, the at least five genes selected are FOXM1, MCM3, GPC1, PCNA, and SPAG5. In one embodiment, the at least five genes selected are FOXM1, GPC1, PCNA, MTUS1, and IRF7. In one embodiment, the at least five genes selected are FOXM1, GPC1, PCNA, MTUS1, and BRIP1. In one embodiment, the at least five genes selected are FOXM1, GPC1, PCNA, MTUS1, and ULK1. In one embodiment, the at least five genes selected are FOXM1, GPC1, PCNA, MTUS1, and SPAG5. In one embodiment, the at least five genes selected are FOXM1, PCNA, MTUS1, IRF7, and BRIP1. In one embodiment, the at least five genes selected are FOXM1, PCNA, MTUS1, IRF7, and ULK1. In one embodiment, the at least five genes selected are FOXM1, PCNA, MTUS1, IRF7, and SPAG5. In one embodiment, the at least five genes selected are FOXM1, MTUS1, IRF7, BRIP1, and ULK1. In one embodiment, the at least five genes selected are FOXM1, MTUS1, IRF7, BRIP1, and SPAG5. In one embodiment, the at least five genes selected are FOXM1, IRF7, BRIP1, ULK1, and SPAG5.

In one embodiment, at least six genes are selected, and the genes selected are CUL4A, TTC21B, MCM3, GPC1, PCNA, and MTUS1. In one embodiment, the at least six genes selected are CUL4A, TTC21B, MCM3, GPC1, PCNA, and IRF7. In one embodiment, the at least six genes selected are CUL4A, TTC21B, MCM3, GPC1, PCNA, and BRIP1. In one embodiment, the at least six genes selected are CUL4A, TTC21B, MCM3, GPC1, PCNA, and ULK1. In one embodiment, the at least six genes selected are CUL4A, TTC21B, MCM3, GPC1, PCNA, and SPAG5. In one embodiment, the at least six genes selected are CUL4A, MCM3, GPC1, PCNA, MTUS1, and IRF7. In one embodiment, the at least six genes selected are CUL4A, MCM3, GPC1, PCNA, MTUS1, and BRIP1. In one embodiment, the at least six genes selected are CUL4A, MCM3, GPC1, PCNA, MTUS1, and ULK1. In one embodiment, the at least six genes selected are CUL4A, MCM3, GPC1, PCNA, MTUS1, and SPAG5. In one embodiment, the at least six genes selected are CUL4A, GPC1, PCNA, MTUS1, IRF7, and BRIP1. In one embodiment, the at least six genes selected are CUL4A, GPC1, PCNA, MTUS1, IRF7, and ULK1. In one embodiment, the at least six genes selected are CUL4A, GPC1, PCNA, MTUS1, IRF7, and SPAG5. In one embodiment, the at least six genes selected are CUL4A, PCNA, MTUS1, IRF7, BRIP1, and ULK1. In one embodiment, the at least six genes selected are CUL4A, PCNA, MTUS1, IRF7, BRIP1, and SPAG5. In one embodiment, the at least six genes selected are CUL4A, MTUS1, IRF7, BRIP1, ULK1, and SPAG5. In one embodiment, the at least six genes selected are TTC21B, MCM3, GPC1, PCNA, MTUS1, and IRF7. In one embodiment, the at least six genes selected are TTC21B, MCM3, GPC1, PCNA, MTUS1, and BRIP1. In one embodiment, the at least six genes selected are TTC21B, MCM3, GPC1, PCNA, MTUS1, and ULK1. In one embodiment, the at least six genes selected are TTC21B, MCM3, GPC1, PCNA, MTUS1, and SPAG5. In one embodiment, the at least six genes selected are TTC21B, GPC1, PCNA, MTUS1, IRF7, and BRIP1. In one embodiment, the at least six genes selected are TTC21B, GPC1, PCNA, MTUS1, IRF7, and ULK1. In one embodiment, the at least six genes selected are TTC21B, GPC1, PCNA, MTUS1, IRF7, and SPAG5. In one embodiment, the at least six genes selected are TTC21B, PCNA, MTUS1, IRF7, BRIP1, and ULK1. In one embodiment, the at least six genes selected are TTC21B, PCNA, MTUS1, IRF7, BRIP1, and SPAG5. In one embodiment, the at least six genes selected are TTC21B, MTUS1, IRF7, BRIP1, ULK1, and SPAG5. In one embodiment, the at least six genes selected are TTC21B, MTUS1, IRF7, BRIP1, ULK1, and SPAG5. In one embodiment, the at least six genes selected are TTC21B, MCM3, GPC1, PCNA, IRF7, and BRIP1. In one embodiment, the at least six genes selected are TTC21B, MCM3, GPC1, PCNA, IRF7, and ULK1. In one embodiment, the at least six genes selected are TTC21B, MCM3, GPC1, PCNA, IRF7, and SPAG5. In one embodiment, the at least six genes selected are TTC21B, GPC1, PCNA, IRF7, BRIP1, and ULK1. In one embodiment, the at least six genes selected are TTC21B, GPC1, PCNA, IRF7, BRIP1, and SPAG5. In one embodiment, the at least six genes selected are TTC21B, PCNA, IRF7, BRIP1, ULK1, and SPAG5. In one embodiment, the at least six genes selected are MCM3, GPC1, PCNA, MTUS1, IRF7, and BRIP1. In one embodiment, the at least six genes selected are MCM3, GPC1, PCNA, MTUS1, IRF7, and ULK1. In one embodiment, the at least six genes selected are MCM3, GPC1, PCNA, MTUS1, IRF7, and SPAG5. In one embodiment, the at least six genes selected are MCM3, PCNA, MTUS1, IRF7, BRIP1, and ULK1. In one embodiment, the at least six genes selected are MCM3, GPC1, PCNA, MTUS1, IRF7, and SPAG5. In one embodiment, the at least six genes selected are MCM3, PCNA, MTUS1, IRF7, BRIP1, and ULK1. In one embodiment, the at least six genes selected are MCM3, PCNA, MTUS1, IRF7, BRIP1, and SPAG5. In one embodiment, the at least six genes selected are GPC1, PCNA, MTUS1, IRF7, BRIP1, and ULK1. In one embodiment, the at least six genes selected are GPC1, PCNA, MTUS1, IRF7, BRIP1, and SPAG5. In one embodiment, the at least six genes selected are PCNA, MTUS1, IRF7, BRIP1, ULK1, and SPAG5. In one embodiment, at least six genes are selected, and the genes selected are FOXM1, TTC21B, MCM3, GPC1, PCNA, and CUL4A. In one embodiment, at least six genes are selected, and the genes selected are FOXM1, TTC21B, MCM3, GPC1, PCNA, and MTUS1. In one embodiment, the at least six genes selected are FOXM1, TTC21B, MCM3, GPC1, PCNA, and IRF7. In one embodiment, the at least six genes selected are FOXM1, TTC21B, MCM3, GPC1, PCNA, and BRIP1. In one embodiment, the at least six genes selected are FOXM1, TTC21B, MCM3, GPC1, PCNA, and ULK1. In one embodiment, the at least six genes selected are FOXM1, TTC21B, MCM3, GPC1, PCNA, and SPAG5. In one embodiment, the at least six genes selected are FOXM1, MCM3, GPC1, PCNA, MTUS1, and IRF7. In one embodiment, the at least six genes selected are FOXM1, MCM3, GPC1, PCNA, MTUS1, and BRIP1. In one embodiment, the at least six genes selected are FOXM1, MCM3, GPC1, PCNA, MTUS1, and ULK1. In one embodiment, the at least six genes selected are FOXM1, MCM3, GPC1, PCNA, MTUS1, and SPAG5. In one embodiment, the at least six genes selected are FOXM1, GPC1, PCNA, MTUS1, IRF7, and BRIP1. In one embodiment, the at least six genes selected are FOXM1, GPC1, PCNA, MTUS1, IRF7, and ULK1. In one embodiment, the at least six genes selected are FOXM1, GPC1, PCNA, MTUS1, IRF7, and SPAG5. In one embodiment, the at least six genes selected are FOXM1, PCNA, MTUS1, IRF7, BRIP1, and ULK1. In one embodiment, the at least six genes selected are FOXM1, PCNA, MTUS1, IRF7, BRIP1, and SPAG5. In one embodiment, the at least six genes selected are FOXM1, MTUS1, IRF7, BRIP1, ULK1, and SPAG5.

In one embodiment, at least seven genes are selected, and the genes selected are CUL4A, TTC21B, MCM3, GPC1, PCNA, MTUS1, and IRF7. In one embodiment, the at least seven genes selected are CUL4A, TTC21B, MCM3, GPC1, PCNA, MTUS1, and IRF7. In one embodiment, the at least seven genes selected are CUL4A, TTC21B, MCM3, GPC1, PCNA, MTUS1, and BRIP1. In one embodiment, the at least seven genes selected are CUL4A, TTC21B, MCM3, GPC1, PCNA, MTUS1, and ULK1. In one embodiment, the at least seven genes selected are CUL4A, TTC21B, MCM3, GPC1, PCNA, MTUS1, and SPAG5. In one embodiment, the at least seven genes selected are CUL4A, MCM3, GPC1, PCNA, MTUS1, IRF7, and BRIP1. In one embodiment, the at least seven genes selected are CUL4A, MCM3, GPC1, PCNA, MTUS1, IRF7, and ULK1. In one embodiment, the at least seven genes selected are CUL4A, MCM3, GPC1, PCNA, MTUS1, IRF7, and SPAG5. In one embodiment, the at least seven genes selected are CUL4A, GPC1, PCNA, MTUS1, IRF7, BRIP1, and ULK1. In one embodiment, the at least seven genes selected are CUL4A, GPC1, PCNA, MTUS1, IRF7, BRIP1, and SPAG5. In one embodiment, the at least seven genes selected are CUL4A, PCNA, MTUS1, IRF7, BRIP1, ULK1, and SPAG5. In one embodiment, the at least seven genes selected are TTC21B, MCM3, GPC1, PCNA, MTUS1, IRF7, and BRIP1. In one embodiment, the at least seven genes selected are TTC21B, MCM3, GPC1, PCNA, MTUS1, IRF7, and ULK1. In one embodiment, the at least seven genes selected are TTC21B, MCM3, GPC1, PCNA, MTUS1, IRF7, and SPAG5. In one embodiment, the at least seven genes selected are TTC21B, GPC1, PCNA, MTUS1, IRF7, BRIP1, and ULK1. In one embodiment, the at least seven genes selected are TTC21B, GPC1, PCNA, MTUS1, IRF7, BRIP1, and SPAG5. In one embodiment, the at least seven genes selected are TTC21B, PCNA, MTUS1, IRF7, BRIP1, ULK1, and SPAG5. In one embodiment, the at least seven genes selected are MCM3, GPC1, PCNA, MTUS1, IRF7, BRIP1, and ULK1. In one embodiment, the at least seven genes selected are MCM3, GPC1, PCNA, MTUS1, IRF7, BRIP1, and SPAG5. In one embodiment, the at least seven genes selected are MCM3, PCNA, MTUS1, IRF7, BRIP1, ULK1, and SPAG5. In one embodiment, the at least seven genes selected are GPC1, PCNA, MTUS1, IRF7, BRIP1, ULK1, and SPAG5. In one embodiment, at least seven genes are selected, and the genes selected are FOXM1, TTC21B, MCM3, GPC1, PCNA, MTUS1, and CUL4A. In one embodiment, at least seven genes are selected, and the genes selected are FOXM1, TTC21B, MCM3, GPC1, PCNA, MTUS1, and IRF7. In one embodiment, the at least seven genes selected are FOXM1, TTC21B, MCM3, GPC1, PCNA, MTUS1, and IRF7. In one embodiment, the at least seven genes selected are FOXM1, TTC21B, MCM3, GPC1, PCNA, MTUS1, and BRIP1. In one embodiment, the at least seven genes selected are FOXM1, TTC21B, MCM3, GPC1, PCNA, MTUS1, and ULK1. In one embodiment, the at least seven genes selected are FOXM1, TTC21B, MCM3, GPC1, PCNA, MTUS1, and SPAG5. In one embodiment, the at least seven genes selected are FOXM1A, MCM3, GPC1, PCNA, MTUS1, IRF7, and BRIP1. In one embodiment, the at least seven genes selected are FOXM1, MCM3, GPC1, PCNA, MTUS1, IRF7, and ULK1. In one embodiment, the at least seven genes selected are FOXM1, MCM3, GPC1, PCNA, MTUS1, IRF7, and SPAG5. In one embodiment, the at least seven genes selected are FOXM1, GPC1, PCNA, MTUS1, IRF7, BRIP1, and ULK1. In one embodiment, the at least seven genes selected are FOXM1, GPC1, PCNA, MTUS1, IRF7, BRIP1, and SPAG5. In one embodiment, the at least seven genes selected are FOXM1, PCNA, MTUS1, IRF7, BRIP1, ULK1, and SPAG5.

In one embodiment, at least eight genes are selected, and the genes selected are CUL4A, TTC21B, MCM3, GPC1, PCNA, MTUS1, IRF7, and BRIP1. In one embodiment, the at least eight genes selected are CUL4A, TTC21B, MCM3, GPC1, PCNA, MTUS1, IRF7, and ULK1. In one embodiment, the at least eight genes selected are CUL4A, TTC21B, MCM3, GPC1, PCNA, MTUS1, IRF7, and SPAG5. In one embodiment, the at least eight genes selected are CUL4A, MCM3, GPC1, PCNA, MTUS1, IRF7, BRIP1, and ULK1. In one embodiment, the at least eight genes selected are CUL4A, MCM3, GPC1, PCNA, MTUS1, IRF7, BRIP1, and SPAG5. In one embodiment, the at least eight genes selected are CUL4A, GPC1, PCNA, MTUS1, IRF7, BRIP1, ULK1, and SPAG5. In one embodiment, the at least eight genes selected are CUL4A, TTC21B, GPC1, PCNA, MTUS1, IRF7, BRIP1, and ULK1. In one embodiment, the at least eight genes selected are CUL4A, TTC21B, GPC1, PCNA, MTUS1, IRF7, BRIP1, and SPAG5. In one embodiment, the at least eight genes selected are CUL4A, TTC21B, GPC1, PCNA, MTUS1, IRF7, ULK1, and SPAG5. In one embodiment, the at least eight genes selected are CUL4A, TTC21B, MCM3, PCNA, MTUS1, IRF7, ULK1 and SPAG5. In one embodiment, the at least eight genes selected are CUL4A, TTC21B, MCM3, GPC1, MTUS1, IRF7, ULK1, and SPAG5. In one embodiment, the at least eight genes selected are CUL4A, TTC21B, MCM3, GPC1, PCNA, IRF7, ULK1, and SPAG5. In one embodiment, the at least eight genes selected are CUL4A, TTC21B, MCM3, GPC1, PCNA, MTUS1, ULK1, and SPAG5. In one embodiment, the at least eight genes selected are CUL4A, TTC21B, MCM3, GPC1, PCNA, MTUS1, IRF7, and ULK1. In one embodiment, the at least eight genes selected are CUL4A, MCM3, GPC1, PCNA, MTUS1, IRF7, ULK1, and SPAG5. In one embodiment, the at least eight genes selected are CUL4A, GPC1, PCNA, MTUS1, IRF7, BRIP1, ULK1, and SPAG5. In one embodiment, the at least eight genes selected are CUL4A, TTC21B, PCNA, MTUS1, IRF7, BRIP1, ULK1, and SPAG5. In one embodiment, the at least eight genes selected are CUL4A, TTC21B, MCM3, MTUS1, IRF7, BRIP1, ULK1, and SPAG5. In one embodiment, the at least eight genes selected are CUL4A, TTC21B, MCM3, GPC1, IRF7, BRIP1, ULK1, and SPAG5. In one embodiment, the at least eight genes selected are CUL4A, TTC21B, MCM3, GPC1, PCNA, BRIP1, ULK1, and SPAG5. In one embodiment, the at least eight genes selected are CUL4A, TTC21B, MCM3, GPC1, PCNA, MTUS1, ULK1, and SPAG5. In one embodiment, the at least eight genes selected are CUL4A, TTC21B, MCM3, GPC1, PCNA, MTUS1, IRF7, and SPAG5. In one embodiment, the at least eight genes selected are TTC21B, MCM3, GPC1, PCNA, MTUS1, IRF7, BRIP1, and ULK1. In one embodiment, the at least eight genes selected are TTC21B, MCM3, GPC1, PCNA, MTUS1, IRF7, BRIP1, and SPAG5. In one embodiment, the at least eight genes selected are TTC21B, GPC1, PCNA, MTUS1, IRF7, BRIP1, ULK1, and SPAG5. In one embodiment, the at least eight genes selected are TTC21B, MCM3, PCNA, MTUS1, IRF7, BRIP1, ULK1, and SPAG5. In one embodiment, the at least eight genes selected are TTC21B, MCM3, GPC1, MTUS1, IRF7, BRIP1, ULK1, and SPAG5. In one embodiment, the at least eight genes selected are TTC21B, MCM3, GPC1, PCNA, IRF7, BRIP1, ULK1, and SPAG5. In one embodiment, the at least eight genes selected are TTC21B, MCM3, GPC1, PCNA, MTUS1, BRIP1, ULK1, and SPAG5. In one embodiment, the at least eight genes selected are TTC21B, MCM3, GPC1, PCNA, MTUS1, IRF7, ULK1, and SPAG5. In one embodiment, the at least eight genes selected are TTC21B, MCM3, GPC1, PCNA, MTUS1, IRF7, BRIP1, and ULK1. In one embodiment, the at least eight genes selected are TTC21B, MCM3, GPC1, PCNA, MTUS1, IRF7, BRIP1, and SPAG5. In one embodiment, the at least eight genes selected are MCM3, GPC1, PCNA, MTUS1, IRF7, BRIP1, ULK1, and SPAG5. In one embodiment, at least eight genes are selected, and the genes selected are FOXM1, TTC21B, MCM3, GPC1, PCNA, MTUS1, IRF7, and CUL4A. In one embodiment, at least eight genes are selected, and the genes selected are FOXM1, TTC21B, MCM3, GPC1, PCNA, MTUS1, IRF7, and BRIP1. In one embodiment, the at least eight genes selected are FOXM1, TTC21B, MCM3, GPC1, PCNA, MTUS1, IRF7, and ULK1. In one embodiment, the at least eight genes selected are FOXM1, TTC21B, MCM3, GPC1, PCNA, MTUS1, IRF7, and SPAG5. In one embodiment, the at least eight genes selected are FOXM1, MCM3, GPC1, PCNA, MTUS1, IRF7, BRIP1, and ULK1. In one embodiment, the at least eight genes selected are FOXM1, MCM3, GPC1, PCNA, MTUS1, IRF7, BRIP1, and SPAG5. In one embodiment, the at least eight genes selected are FOXM1, GPC1, PCNA, MTUS1, IRF7, BRIP1, ULK1, and SPAG5. In one embodiment, the at least eight genes selected are FOXM1, TTC21B, GPC1, PCNA, MTUS1, IRF7, BRIP1, and ULK1. In one embodiment, the at least eight genes selected are FOXM1, TTC21B, GPC1, PCNA, MTUS1, IRF7, BRIP1, and SPAG5. In one embodiment, the at least eight genes selected are FOXM1, TTC21B, GPC1, PCNA, MTUS1, IRF7, ULK1, and SPAG5. In one embodiment, the at least eight genes selected are FOXM1, TTC21B, MCM3, PCNA, MTUS1, IRF7, ULK1 and SPAG5. In one embodiment, the at least eight genes selected are FOXM1, TTC21B, MCM3, GPC1, MTUS1, IRF7, ULK1, and SPAG5. In one embodiment, the at least eight genes selected are FOXM1, TTC21B, MCM3, GPC1, PCNA, IRF7, ULK1, and SPAG5. In one embodiment, the at least eight genes selected are FOXM1, TTC21B, MCM3, GPC1, PCNA, MTUS1, ULK1, and SPAG5. In one embodiment, the at least eight genes selected are FOXM1, TTC21B, MCM3, GPC1, PCNA, MTUS1, IRF7, and ULK1. In one embodiment, the at least eight genes selected are FOXM1, MCM3, GPC1, PCNA, MTUS1, IRF7, ULK1, and SPAG5. In one embodiment, the at least eight genes selected are FOXM1, GPC1, PCNA, MTUS1, IRF7, BRIP1, ULK1, and SPAG5. In one embodiment, the at least eight genes selected are FOXM1, TTC21B, PCNA, MTUS1, IRF7, BRIP1, ULK1, and SPAG5. In one embodiment, the at least eight genes selected are FOXM1, TTC21B, MCM3, MTUS1, IRF7, BRIP1, ULK1, and SPAG5. In one embodiment, the at least eight genes selected are FOXM1, TTC21B, MCM3, GPC1, IRF7, BRIP1, ULK1, and SPAG5. In one embodiment, the at least eight genes selected are FOXM1, TTC21B, MCM3, GPC1, PCNA, BRIP1, ULK1, and SPAG5. In one embodiment, the at least eight genes selected are FOXM1, TTC21B, MCM3, GPC1, PCNA, MTUS1, ULK1, and SPAG5. In one embodiment, the at least eight genes selected are FOXM1, TTC21B, MCM3, GPC1, PCNA, MTUS1, IRF7, and SPAG5.

In one embodiment, at least nine genes are selected, and the genes selected are CUL4A, TTC21B, MCM3, GPC1, PCNA, MTUS1, IRF7, BRIP1, and ULK1. In one embodiment, the at least nine genes selected are CUL4A, TTC21B, MCM3, GPC1, PCNA, MTUS1, IRF7, BRIP1, and SPAG5. In one embodiment, the at least nine genes selected are TTC21B, MCM3, GPC1, PCNA, MTUS1, IRF7, BRIP1, ULK1, and SPAG5. In one embodiment, the at least nine genes selected are CUL4A, TTC21B, GPC1, PCNA, MTUS1, IRF7, BRIP1, ULK1, and SPAG5. In one embodiment, the at least nine genes selected are CUL4A, TTC21B, MCM3, PCNA, MTUS1, IRF7, BRIP1, ULK1, and SPAG5. In one embodiment, the at least nine genes selected are CUL4A, TTC21B, MCM3, GPC1, MTUS1, IRF7, BRIP1, ULK1, and SPAG5. In one embodiment, the at least nine genes selected are CUL4A, TTC21B, MCM3, GPC1, PCNA, IRF7, BRIP1, ULK1, and SPAG5. In one embodiment, the at least nine genes selected are CUL4A, TTC21B, MCM3, GPC1, PCNA, MTUS1, BRIP1, ULK1, and SPAG5. In one embodiment, the at least nine genes selected are CUL4A, TTC21B, MCM3, GPC1, PCNA, MTUS1, IRF7, ULK1, and SPAG5. In one embodiment, the at least nine genes selected are CUL4A, TTC21B, MCM3, GPC1, PCNA, MTUS1, IRF7, BRIP1, and SPAG5. In one embodiment, the at least nine genes selected are CUL4A, TTC21B, MCM3, GPC1, PCNA, MTUS1, IRF7, BRIP1, and ULK1 In one embodiment, at least nine genes are selected, and the genes selected are FOXM1, TTC21B, MCM3, GPC1, PCNA, MTUS1, IRF7, BRIP1, and CUL4A. In one embodiment, the at least nine genes selected are FOXM1, TTC21B, MCM3, GPC1, PCNA, MTUS1, IRF7, BRIP1, and SPAG5. In one embodiment, the at least nine genes selected are FOXM1, TTC21B, GPC1, PCNA, MTUS1, IRF7, BRIP1, ULK1, and SPAG5. In one embodiment, the at least nine genes selected are FOXM1, TTC21B, MCM3, PCNA, MTUS1, IRF7, BRIP1, ULK1, and SPAG5. In one embodiment, the at least nine genes selected are FOXM1, TTC21B, MCM3, GPC1, MTUS1, IRF7, BRIP1, ULK1, and SPAG5. In one embodiment, the at least nine genes selected are FOXM1, TTC21B, MCM3, GPC1, PCNA, IRF7, BRIP1, ULK1, and SPAG5. In one embodiment, the at least nine genes selected are FOXM1, TTC21B, MCM3, GPC1, PCNA, MTUS1, BRIP1, ULK1, and SPAG5. In one embodiment, the at least nine genes selected are FOXM1, TTC21B, MCM3, GPC1, PCNA, MTUS1, IRF7, ULK1, and SPAG5. In one embodiment, the at least nine genes selected are FOXM1, TTC21B, MCM3, GPC1, PCNA, MTUS1, IRF7, BRIP1, and SPAG5. In one embodiment, the at least nine genes selected are FOXM1, TTC21B, MCM3, GPC1, PCNA, MTUS1, IRF7, BRIP1, and ULK1.

In one embodiment, at least ten genes are selected, and the genes selected are CUL4A, TTC21B, MCM3, GPC1, PCNA, MTUS1, IRF7, BRIP1, ULK1, and SPAG5. In one embodiment, the at least ten genes selected are FOXM1, CUL4A, TTC21B, MCM3, GPC1, PCNA, MTUS1, IRF7, BRIP1, and ULK1. In one embodiment, the at least ten genes selected are FOXM1, CUL4A, TTC21B, MCM3, GPC1, PCNA, MTUS1, IRF7, BRIP1, and SPAG5. In one embodiment, the at least ten genes selected are FOXM1, CUL4A, TTC21B, MCM3, GPC1, PCNA, MTUS1, IRF7, ULK1, and SPAG5. In one embodiment, the at least ten genes selected are FOXM1, CUL4A, TTC21B, MCM3, GPC1, PCNA, MTUS1, BRIP1, ULK1, and SPAG5. In one embodiment, the at least ten genes selected are FOXM1, CUL4A, TTC21B, MCM3, GPC1, PCNA, IRF7, BRIP1, ULK1, and SPAG5. In one embodiment, the at least ten genes selected are FOXM1, CUL4A, TTC21B, MCM3, GPC1, MTUS1, IRF7, BRIP1, ULK1, and SPAG5. In one embodiment, the at least ten genes selected are FOXM1, CUL4A, TTC21B, MCM3, GPC1, MTUS1, IRF7, BRIP1, ULK1, and SPAG5. In one embodiment, the at least ten genes selected are FOXM1, CUL4A, TTC21B, MCM3, PCNA, MTUS1, IRF7, BRIP1, ULK1, and SPAG5. In one embodiment, the at least ten genes selected are FOXM1, CUL4A, TTC21B, GPC1, PCNA, MTUS1, IRF7, BRIP1, ULK1, and SPAG5. In one embodiment, the at least ten genes selected are FOXM1, CUL4A, MCM3, GPC1, PCNA, MTUS1, IRF7, BRIP1, ULK1, and SPAG5. In one embodiment, the at least ten genes selected are FOXM1, TTC21B, MCM3, GPC1, PCNA, MTUS1, IRF7, BRIP1, ULK1, and SPAG5.

In one embodiment, all of TTC21B, MCM3, MTUS1, FOXM1, CUL4A, GPC1, PCNA, IRF7, BRIP1, ULK1, and SPAG5 are selected.

In one embodiment, the at least two genes selected are MTUS1 and TTC21B.

In one embodiment, the at least two genes comprise MTUS1 and TTC21B, and at least one further gene selected from MCM3, FOXM1, CUL4, GPC1, PCNA, IRF7, BRIP1, ULK1, and SPAG5. Ideally, the gene selected are FOXM1, MTUS1, and TTC21B.

In one embodiment, the at least two genes comprise MTUS1 and MCM3, and at least one further gene selected from TTC21B, FOXM1, CUL4, GPC1, PCNA, IRF7, BRIP1, ULK1, and SPAG5.

In one embodiment, the at least two genes comprise TTC21B and MCM3, and at least one further gene selected from TTC21B, FOXM1, GPC1, CUL4, PCNA, MTUS1, IRF7, BRIP1, ULK1, and SPAG5.

In one embodiment, the at least two genes selected from TTC21B, MCM3, MTUS1, FOXM1, CUL4, GPC1, PCNA, IRF7, BRIP1, ULK1, and SPAG5, are MTUS1, TTC21B, MCM3, and FOXM1.

In one embodiment, the genes selected consist of MCM3, MTUS1, TTC21B, and FOXM1.

The term "consist essentially of" should be understood to mean all eleven genes, or ten genes, or nine genes, or eight genes, or seven gene, or six genes, or five genes, or four genes, or three genes, or two genes selected from TTC21B, MCM3, MTUS1, FOXM1, CUL4A, GPC1, PCNA, IRF7, BRIP1, ULK1, and SPAG5.

In one embodiment, the cancer is selected from the group comprising multiple myeloma, prostate cancer, glioblastoma, lymphoma, fibrosarcoma; myxosarcoma; liposarcoma; chondrosarcoma; osteogenic sarcoma; chordoma; angiosarcoma; endotheliosarcoma; lymphangiosarcoma; lymphangioendotheliosarcoma; synovioma; mesothelioma; Ewing's tumour; leiomyosarcoma; rhabdomyosarcoma; colon carcinoma; pancreatic cancer; ovarian cancer; squamous cell carcinoma; basal cell carcinoma; adenocarcinoma; sweat gland carcinoma; sebaceous gland carcinoma; papillary carcinoma; papillary adenocarcinomas; cystadenocarcinoma; medullary carcinoma; bronchogenic carcinoma; renal cell carcinoma; hepatoma; bile duct carcinoma; choriocarcinoma; seminoma; embryonal carcinoma; Wilms' tumour; cervical cancer; uterine cancer; testicular tumour; lung carcinoma; small cell lung carcinoma; bladder carcinoma; epithelial carcinoma; glioma; astrocytoma; medulloblastoma; craniopharyngioma; ependymoma; pinealoma; hemangioblastoma; acoustic neuroma; oligodendroglioma; meningioma; melanoma; retinoblastoma; and leukemias. Suitably, the cancer is an epithelial cancer.

In one embodiment, the cancer is a pre-cancer condition selected from Barrett's oesophagus, Bowen's disease, Familial adenomatous polyposis (FAP), Lobular carcinoma in situ (LCIS), Lynch syndrome, MEN2, Vaginal intra-epithelial neoplasia (VAIN), and Vulval intraepithelial neoplasia (VIN). Pre-cancer conditions are included in the diagnosing whether a subject is at risk of having an aggressive cancer or at risk of having a recurrence of cancer.

In one embodiment, the recurrence is development of a secondary tumour.

In one embodiment, the recurrence is developing a further, independent primary cancer unrelated to the sampled cancer.

In one embodiment of the invention, there is provided a method of predicting the risk of recurrence of prostate cancer in a patient the method comprising: wherein when the expression of the at least two genes, or proteins encoded by said genes, is modified relative to a normalised gene expression level of the at least two genes, or proteins encoded by said genes, in a biological sample from whom the cancer is non-aggressive or indolent subject, the individual has an increased risk of having a recurrent prostate cancer.

In one embodiment, the therapy or treatment is a neoadjuvant therapy. In the specification, the term "neoadjuvant therapy" should be understood to mean treatment given before primary treatment to increase the chances of long-term survival. Primary treatment is generally surgery. Neoadjuvant therapies are generally selected from chemotherapy, hormonal therapy, targeted therapy, radiation therapy, immunotherapy or a combination thereof.

In one embodiment, the therapy is an adjuvant therapy. In the specification, the term "adjuvant therapy" should be understood to mean any treatment given after primary treatment to increase the chances of long-term survival. Primary treatment is generally surgery. Adjuvant therapies are generally selected from chemotherapy, hormonal therapy, targeted therapy, radiation therapy, immunotherapy, or a combination thereof. Active surveillance can also be considered an adjuvant therapy.

In one embodiment, the therapy can be a combination of neoadjuvant and adjuvant therapy.

In one embodiment, the therapy can be an active surveillance, where the treatment plan involves closely watching an individual's condition but not giving any treatment unless there are changes in test results that show the condition is worsening.

In one aspect, the therapy can be a surgical procedure (for example, a surgical procedure to remove a primary or a secondary tumour), or a combination of neoadjuvant therapy and surgery; or adjuvant therapy and surgery; or neoadjuvant therapy, surgery, and adjuvant therapy.

In one embodiment, the cancer patient may be suitable for treatment with a neoadjuvant therapy for preventing recurrence or progression of the cancer.

In one embodiment, the cancer is prostate cancer.

In one aspect, the adjuvant therapy and neoadjuvant therapy is a chemotherapeutic therapy. In one embodiment, the adjuvant therapy and neoadjuvant therapy is selected from cyclophosphamide, methotrexate, 5-fluorouracil, gemcitabine, doxorubicin (Adriamycin®; $C_{27}H_{29}NO_{11}$), paclitaxel (Taxol®; $C_{47}H_{51}NO_{14}$), capecitabine (Xeloda®; $C_{15}H_{22}FN_3O_6$), docetaxel (Taxotere®; $C_{43}H_{53}NO_{14}$), cabazitaxel (Jeytana®; $C_{45}H_{57}NO_{14}$), mitoxantrone (Novantrone®; $C_{22}H_{28}N_4O_6$), and estramustine (Estradiol 3-(bis(2-chloroethyl)carbamate) ester; $C_{23}H_{31}Cl_2NO_3$).

In one aspect, the therapy is electing not to have surgery, adjuvant therapy, and/or neoadjuvant therapy.

In one embodiment of the invention, there is provided a method for treating cancer or pre-cancerous condition comprising the steps of: identifying an individual with increased potential for recurrence of cancer by assaying a biological sample from the individual for expression of at least two genes selected from the group consisting of TTC21B, MCM3, MTUS1, FOXM1, CUL4A, GPC1, PCNA, IRF7, BRIP1, ULK1, and SPAG5, wherein when a normalised expression of the at least two genes, or proteins encoded by said genes, is modified relative to a normalised gene expression level of the at least two genes from a biological sample obtained from a subject whose cancer is non-aggressive or indolent, the individual has an increased risk of having a recurrent cancer; and

treating the individual with a therapeutically effective amount of an adjuvant therapy.

In one embodiment, the individual is treated with a therapeutically effective amount of a neoadjuvant therapy.

In one embodiment of the invention, there is provided a method for treating cancer or a pre-cancerous condition comprising the steps of: identifying an individual with increased potential for recurrence of cancer by assaying a biological sample from the individual for expression of at least two genes selected from the group consisting of TTC21B, MCM3, MTUS1, FOXM1, CUL4A, GPC1, PCNA, IRF7, BRIP1, ULK1, and SPAG5, wherein when a combination of a normalised expression value of the at least two genes, or proteins encoded by said genes, is modified relative to a combination of a normalised expression value of the at least two genes from a biological sample obtained from a subject with cancer, the individual is predicted to have an increased risk of having a recurrent cancer; and

treating the individual with a therapeutically effective amount of a neoadjuvant therapy.

In one embodiment, the individual is treated with a therapeutically effective amount of an adjuvant therapy.

In one embodiment, the individual is followed up with active surveillance.

In one embodiment, the neoadjuvant therapy and adjuvant therapy is an agent selected from, but not limited to, chemotherapeutic drugs selected from cyclophosphamide, methotrexate, 5-fluorouracil, gemcitabine, doxorubicin (Adriamycin®; $C_{27}H_{29}NO_{11}$), paclitaxel (Taxol®; $C_{47}H_{51}NO_{14}$), capecitabine (Xeloda®; $C_{15}H_{22}FN_3O_6$), docetaxel (Taxotere®; $C_{43}H_{53}NO_{14}$), cabazitaxel (Jeytana®; $C_{45}H_{57}NO_{14}$), mitoxantrone (Novantrone®; $C_{22}H_{28}N_4O_6$), and estramustine (Estradiol 3-(bis(2-chloroethyl)carbamate) ester; $C_{23}H_{31}Cl_2NO_3$); and luteinising hormone-releasing (LHRH) agonists selected from leuprorelin (Lupron®; $C_{59}H_{84}N_{16}O_{12}$); goserelin (Zoladex®; $C_{59}H_{84}N_{18}O_{14}$); triptorelin (Decapeptyl®; $C_{64}H_{82}N_{18}O_{13}$); leuprolide mesylate ($C_{60}H_{88}N_{16}O_{15}S$); degarelix (Firmagon®; $C_{82}H_{103}CIN_{18}O_{16}$); and relugolix (Orgovyx®; $C_{29}H_{27}F_2N_7O_5S$).

The invention also relates to a method of treating an individual to prevent or inhibit recurrence of the cancer comprising a step of identifying a cancer patient at risk of recurrence using a method of the invention, and then treating the cancer patient with an agent or agents to prevent or inhibit recurrence of the cancer. Typically, the agent or agents comprise adjuvant or neoadjuvant therapy, or a combination of both.

In one embodiment, there is provided a system for obtaining data from at least one test sample obtained from at least one individual, the system comprising a determination module configured to receive at least one test sample and perform at least one test analysis on the test sample to assay for expression of at least three genes or proteins encoded by those genes selected from TTC21B, MCM3, MTUS1, FOXM1, CUL4A, GPC1, PCNA, IRF7, BRIP1, ULK1, and SPAG5, optionally, a storage system for storing expression data generated by the determination module; and a display module for displaying a content based in part on the data output from said determination module, wherein the content comprises a signal indicative of a combination of a normalised expression value of at the least three genes or proteins encoded by those genes.

In one embodiment, the at least three genes, or proteins encoded by said genes, are TTC21B, MTUS1, and MCM3.

In one embodiment, there is provided a method of predicting risk of recurrence or progression of cancer in a patient, and treating the patient with a therapy for preventing recurrence of the cancer, the method comprising a step of assaying a biological sample from the patient for expression of at least four genes selected from TTC21B, MCM3, MTUS1, FOXM1, CUL4A, GPC1, PCNA, IRF7, BRIP1, ULK1, and SPAG5, ; wherein when a combination of a normalised expression value of the at least four genes, or proteins encoded by said genes, is modified relative to a combination of a normalised expression value of the at least four genes in a sample obtained from a subject who is cancer free, the individual is administered a neoadjuvant or an adjuvant therapy, or a combination of both, to the patient to prevent recurrence or progression of the cancer. In one embodiment, the at least four genes, or proteins encoded by said genes, are TTC21B, MTUS1, MCM3, and FOXM1.

As described herein, levels of one or more of TTC21B, MCM3, MTUS1, FOXM1, CUL4A, GPC1, PCNA, IRF7, BRIP1, ULK1, and SPAG5, can be modified or dysregulated in subjects with cancer, and in particular in subjects with prostate cancer. Accordingly, in one aspect of any of the embodiments, described herein is a method of treating cancer or pre-cancerous condition in a subject in need thereof, the method comprising administering a local therapy (surgical, radiation therapy, or both), a neoadjuvant therapy, an adjuvant systemic therapy, or a combination thereof, to a subject determined to have a combination of a normalised expression value of one or more of TTC21B, MCM3, MTUS1, FOXM1, CUL4A, GPC1, PCNA, IRF7, BRIP1, ULK1, and SPAG5 that is modified relative to a combination of a normalised expression value in a reference sample. In one aspect of any of the embodiments, described herein is a method of treating prostate cancer in a subject in need thereof, the method comprising: a) determining the level of one or more of TTC21B, MCM3, MTUS1, FOXM1, CUL4A, GPC1, PCNA, IRF7, BRIP1, ULK1, and SPAG5 in a biological sample obtained from a subject; and b) administering to the subject if a combination of a normalised value of one or more of TTC21B, MCM3, MTUS1, FOXM1, CUL4A, GPC1, PCNA, IRF7, BRIP1, ULK1, and SPAG5 is modified relative to a combination of a normalized expression value in a reference sample. The therapy can be selected from surgery and radiotherapy; and/or hormonal therapy, chemotherapy, and/or immune therapy, or a combination thereof. In one aspect, the therapy can be a surgical procedure (for example, a surgical procedure to remove a primary or a secondary tumour), or a combination of neoadjuvant therapy and surgery; or adjuvant therapy and surgery; or neoadjuvant therapy, surgery, and adjuvant therapy. Active surveillance can also be included.

In one aspect, the adjuvant therapy and neoadjuvant therapy is a chemotherapeutic therapy. In one embodiment, the adjuvant therapy and neoadjuvant therapy is selected from cyclophosphamide, methotrexate, 5-fluorouracil, gemcitabine, doxorubicin (Adriamycin®; $C_{27}H_{29}NO_{11}$), paclitaxel (Taxol®; $C_{47}H_{51}NO_{14}$), capecitabine (Xeloda®; $C_{15}H_{22}FN_3O_6$), docetaxel (Taxotere®; $C_{43}H_{53}NO_{14}$), cabazitaxel (Jeytana®; $C_{45}H_{57}NO_{14}$), mitoxantrone (Novantrone®; $C_{22}H_{28}N_4O_6$), and estramustine (Estradiol 3-(bis(2-chloroethyl)carbamate) ester; $C_{23}H_{31}Cl_2NO_3$).

In one embodiment, the genes selected are MTUS1 and TTC21B, MTUS1 and MCM3, MCM3 and TTC21B, or MCM3, MTUS1, TTC21B, and FOXM1.

In some embodiments of any of the aspects, the method comprises administering to a subject previously determined to have a combination of a normalised expression value of one or more of TTC21B, MCM3, MTUS1, FOXM1, CUL4A, GPC1, PCNA, IRF7, BRIP1, ULK1, and SPAG5 that is modified or dysregulated relative to a combination of a normalised expression value in a reference sample. In some embodiments of any of the aspects, described herein is a method of treating prostate cancer in a subject in need thereof, the method comprising: a) first determining a combination of a normalised expression value of one or more of TTC21B, MCM3, MTUS1, FOXM1, CUL4A, GPC1, PCNA, IRF7, BRIP1, ULK1, and SPAG5 in a biological sample obtained from a subject; and b) then administering a local therapy (surgical, radiation therapy, or both), a neoadjuvant therapy, an adjuvant systemic therapy, or a combination thereof, to the subject if the combination of the normalised expression value of one or more of TTC21B, MCM3, MTUS1, FOXM1, CUL4A, GPC1, PCNA, IRF7, BRIP1, ULK1, and SPAG5 is modified or dysregulated relative to a combination of a normalised expression value in a reference sample.

In one embodiment, the genes selected are MTUS1 and TTC21B, MTUS1 and MCM3, MCM3 and TTC21B, or MCM3, MTUS1, TTC21B, and FOXM1.

In one aspect of any of the embodiments, described herein is a method of treating cancer or a pre-cancerous condition in a subject in need thereof, the method comprising: a) determining if the subject has a modified or a dysregulated combination of a normalised expression value of one or more of TTC21B, MCM3, MTUS1, FOXM1, CUL4A, GPC1, PCNA, IRF7, BRIP1, ULK1, and SPAG5, and b) administering a local therapy (surgical, radiation therapy, or both), a neoadjuvant therapy, an adjuvant systemic therapy, or a combination thereof, to the subject if the combination of a normalised expression value of one or more of TTC21B, MCM3, MTUS1, FOXM1, CUL4A, GPC1, PCNA, IRF7, BRIP1, ULK1, and SPAG5 is modified or dysregulated relative to a combination of a normalised expression value of a reference sample. In some embodiments of any of the aspects, the step of determining if the subject has a modified or a dysregulated combination of a normalised expression value of one or more of TTC21B, MCM3, MTUS1, FOXM1, CUL4A, GPC1, PCNA, IRF7, BRIP1, ULK1, and SPAG5 can comprise (i) obtaining or having obtained a biological sample from the subject and (ii) performing or having performed an assay on the biological sample obtained from the subject to determine/measure the expression value of one or more of TTC21B, MCM3, MTUS1, FOXM1, CUL4A, GPC1, PCNA, IRF7, BRIP1, ULK1, and SPAG5 in the subject. In some embodiments of any of the aspects, the step of determining if the subject has a modified or a dysregulated expression value of one or more of TTC21B, MCM3, MTUS1, FOXM1, CUL4A, GPC1, PCNA, IRF7, BRIP1, ULK1, and SPAG5 can comprise performing or having performed an assay on a biological sample obtained from the subject to determine/measure the expression value of one or more of TTC21B, MCM3, MTUS1, FOXM1, CUL4A, GPC1, PCNA, IRF7, BRIP1, ULK1, and SPAG5 in the subject. In some embodiments of any of the aspects, the step of determining if the subject has a modified or a dysregulated expression value of one or more of TTC21B, MCM3, MTUS1, FOXM1, CUL4A, GPC1, PCNA, IRF7, BRIP1, ULK1, and SPAG5 can comprise ordering or requesting an assay on a biological sample obtained from the subject to determine/measure the expression value of one or more of TTC21B, MCM3, MTUS1, FOXM1, CUL4A, GPC1, PCNA, IRF7, BRIP1, ULK1, and SPAG5 in the subject. In some embodiments of any of the aspects, the step of determining if the subject has a modified or a dysregulated expression value of one or more of TTC21B, MCM3, MTUS1, FOXM1, CUL4A, GPC1, PCNA, IRF7, BRIP1, ULK1, and SPAG5 can comprise receiving the results of an assay on a biological sample obtained from the subject to determine/measure the expression value of one or more of TTC21B, MCM3, MTUS1, FOXM1, CUL4A, GPC1, PCNA, IRF7, BRIP1, ULK1, and SPAG5 in the subject. In some embodiments of any of the aspects, the step of determining if the subject has a modified or a dysregulated expression value of one or more of TTC21B, MCM3, MTUS1, FOXM1, CUL4A, GPC1, PCNA, IRF7, BRIP1, ULK1, and SPAG5 can comprise receiving a report, results, or other means of identifying the subject as a subject with a modified or dysregulated expression value of one or more of TTC21B, MCM3, MTUS1, FOXM1, CUL4A, GPC1, PCNA, IRF7, BRIP1, ULK1, and SPAG5 . In one embodiment, the genes selected are MTUS1 and TTC21B, MTUS1 and MCM3, MCM3 and TTC21B, or MCM3, MTUS1, TTC21B, and FOXM1.

In one aspect of any of the embodiments, described herein is a method of treating prostate cancer in a subject in need thereof, the method comprising: a) determining if the subject has a modified or a dysregulated combination of a normalised expression value of one or more of TTC21B, MCM3, MTUS1, FOXM1, CUL4A, GPC1, PCNA, IRF7, BRIP1, ULK1, and SPAG5; and b) instructing or directing that the subject be administered a local therapy (surgical, radiation therapy, or both), a neoadjuvant therapy, an adjuvant systemic therapy, or a combination thereof, if the combination of a normalised expression value of one or more of TTC21B, MCM3, MTUS1, FOXM1, CUL4A, GPC1, PCNA, IRF7, BRIP1, ULK1, and SPAG5 is modified or dysregulated relative to a combination of a normalised expression value in a reference sample. In some embodiments of any of the aspects, the step of determining if the subject has a modified or a dysregulated combination of a normalised expression value of one or more of TTC21B, MCM3, MTUS1, FOXM1, CUL4A, GPC1, PCNA, IRF7, BRIP1, ULK1, and SPAG5 can comprise (i) obtaining or having obtained a biological sample from the subject and (ii) performing or having performed an assay on the biological sample obtained from the subject to determine/measure the combination of a normalised expression value of one or more of TTC21B, MCM3, MTUS1, FOXM1, CUL4A, GPC1, PCNA, IRF7, BRIP1, ULK1, and SPAG5 in the subject. In some embodiments of any of the aspects, the step of determining if the subject has a modified or a dysregulated expression value of one or more of TTC21B, MCM3, MTUS1, FOXM1, CUL4A, GPC1, PCNA, IRF7, BRIP1, ULK1, and SPAG5 can comprise performing or having performed an assay on a biological sample obtained from the subject to determine/measure the combination of a normalised expression value of one or more of TTC21B, MCM3, MTUS1, FOXM1, CUL4A, GPC1, PCNA, IRF7, BRIP1, ULK1, and SPAG5 in the subject. In some embodiments of any of the aspects, the step of determining if the subject has a modified or a dysregulated expression level of one or more of TTC21B, MCM3, MTUS1, FOXM1, CUL4A, GPC1, PCNA, IRF7, BRIP1, ULK1, and SPAG5 can comprise ordering or requesting an assay on a biological sample obtained from the subject to determine/measure the combination of a normalised expression value of one or more of TTC21B, MCM3, MTUS1, FOXM1, CUL4A, GPC1, PCNA, IRF7, BRIP1, ULK1, and SPAG5 in the subject. In some embodiments of any of the aspects, the step of instructing or directing that the subject be administered a particular treatment can comprise providing a report of the assay results. In some embodiments of any of the aspects, the step of instructing or directing that the subject be administered a particular treatment can comprise providing a report of the assay results and/or treatment recommendations in view of the assay results. In one embodiment, the genes selected are MTUS1 and TTC21B, MTUS1 and MCM3, MCM3 and TTC21B, or MCM3, MTUS1, TTC21B, and FOXM1.

In one aspect of any of the embodiments, described herein is a method of determining if a subject has cancer or is in need of treatment for cancer, the method comprising: determining the expression of one or more of TTC21B, MCM3, MTUS1, FOXM1, CUL4A, GPC1, PCNA, IRF7, BRIP1, ULK1, and SPAG5 in a biological sample obtained from the subject, wherein a combination of a normalised expression value of one or more of TTC21B, MCM3, MTUS1, FOXM1, CUL4A, GPC1, PCNA, MTUS1, IRF7, BRIP1, ULK1, and SPAG5 which is modified or dysregulated relative to a combination of a normalised expression value in a reference sample indicates the subject is predicted to have cancer or is predicted to be in need of treatment for cancer. In one aspect, the cancer is prostate cancer. In one embodiment, the genes selected are MTUS1 and TTC21B, MTUS1 and MCM3, MCM3 and TTC21B, or MCM3, MTUS1, TTC21B, and FOXM1.

In some embodiments of any of the aspects, the measurement of the expression value of a target and/or detection of the expression value or presence of a target, e.g., of an expression product (nucleic acid or polypeptide of one of the genes described herein) or a mutation can comprise a transformation. As used herein, the term "transforming" or "transformation" refers to changing an object or a substance, e.g., biological sample, nucleic acid, or protein, into another substance. The transformation can be physical, biological, or chemical. Exemplary physical transformation includes, but is not limited to, pre-treatment of a biological sample, e.g., from whole blood to blood serum by differential centrifugation. A biological/chemical transformation can involve the action of at least one enzyme and/or a chemical reagent in a reaction. For example, a DNA sample can be digested into fragments by one or more restriction enzymes, or an exogenous molecule can be attached to a fragmented DNA sample with a ligase. In some embodiments of any of the aspects, a DNA sample can undergo enzymatic replication, e.g., by polymerase chain reaction (PCR).

Transformation, measurement, and/or detection of a target molecule, e.g., a gene, nucleotide, mRNA, or polypeptide can comprise contacting a sample obtained from a subject with a reagent (e.g., a detection reagent) which is specific for the target, e.g., a target-specific reagent. In some embodiments of any of the aspects, the target-specific reagent is detectably labeled. In some embodiments of any of the aspects, the target-specific reagent can generate a detectable signal. In some embodiments of any of the aspects, the target-specific reagent generates a detectable signal when the target molecule is present.

Methods to measure gene expression products are known to a skilled artisan. Such methods to measure gene expression products, e.g., protein level, include ELISA (enzyme linked immunosorbent assay), western blot, immunoprecipitation, and immunofluorescence using detection reagents such as an antibody or protein binding agents. Alternatively, a peptide can be detected in a subject by introducing into a subject a labeled anti-peptide antibody and other types of detection agent. For example, the antibody can be labeled with a detectable marker whose presence and location in the subject is detected by standard imaging techniques.

For example, antibodies for the various targets described herein are commercially available and can be used for the purposes of the invention to measure protein expression levels, e.g., anti-FOXM1 (MilliporeSigma/MerckKGaA Cat. No. SAB1412254), anti- PTTG1 (MilliporeSigma/MerckKGaA Cat. No. WH0009232M1), anti-ZNF367 (MilliporeSigma/MerckKGaA Cat. No. HPA015785), anti-AGER (MilliporeSigma/MerckKGaA Cat. No. AV41642), anti-CUL4A (MilliporeSigma/MerckKGaA Cat. No. SAB1411512), anti-TTC21B (MilliporeSigma/MerckKGaA Cat. No. HPA035495), anti-MCM3 (MilliporeSigma/MerckKGaA Cat. No. SAB1412669), anti-GPC1 (MilliporeSigma/MerckKGaA Cat. No. HPA030571), anti-PCNA (MilliporeSigma/MerckKGaA Cat. No. HPA030521), anti-MTUS1 (MilliporeSigma/MerckKGaA Cat. No. WH0057509M1), anti-IRF7 (MilliporeSigma/MerckKGaA Cat. No. PRS3941), anti-BRIP1 (MilliporeSigma/MerckKGaA Cat. No. HPA005474), anti-ULK1 (MilliporeSigma/MerckKGaA Cat. No. WH0008408M1), and anti-SPAG5 (MilliporeSigma/MerckKGaA Cat. No. HPA022479). Alternatively, since the amino acid sequences for the targets described herein are known and publicly available at the NCBI website, one of skill in the art can raise their own antibodies against these polypeptides of interest for the purpose of the methods described herein.

In some embodiments of any of the aspects, immunohistochemistry ("IHC") and immunocytochemistry ("ICC") techniques can be used. IHC is the application of immunochemistry to tissue sections, whereas ICC is the application of immunochemistry to cells or tissue imprints after they have undergone specific cytological preparations such as, for example, liquid-based preparations. Immunochemistry is a family of techniques based on the use of an antibody, wherein the antibodies are used to specifically target molecules inside or on the surface of cells. The antibody typically contains a marker that will undergo a biochemical reaction, and thereby experience a change of color, upon encountering the targeted molecules. In some instances, signal amplification can be integrated into the particular protocol, wherein a secondary antibody, that includes the marker stain or marker signal, follows the application of a primary specific antibody.

In some embodiments of any of the aspects, the assay can be a Western blot analysis. Alternatively, proteins can be separated by two-dimensional gel electrophoresis systems. Two-dimensional gel electrophoresis is well known in the art and typically involves iso-electric focusing along a first dimension followed by SDS-PAGE electrophoresis along a second dimension. These methods also require a considerable amount of cellular material. The analysis of 2D SDS-PAGE gels can be performed by determining the intensity of protein spots on the gel or can be performed using immune detection. In other embodiments, protein samples are analyzed by mass spectroscopy.

Immunological tests can be used with the methods and assays described herein and include, for example, competitive and non-competitive assay systems using techniques such as Western blots, radioimmunoassay (RIA), ELISA (enzyme linked immunosorbent assay), "sandwich" immunoassays, immunoprecipitation assays, immunodiffusion assays, agglutination assays, *e.g.* latex agglutination, complement-fixation assays, immunoradiometric assays, fluorescent immunoassays, *e.g.*, FIA (fluorescence-linked immunoassay), chemiluminescence immunoassays (CLIA), electrochemiluminescence immunoassay (ECLIA, counting immunoassay (CIA), lateral flow tests or immunoassay (LFIA), magnetic immunoassay (MIA), and protein A immunoassays. Methods for performing such assays are known in the art, provided an appropriate antibody reagent is available. In some embodiments of any of the aspects, the immunoassay can be a quantitative or a semi-quantitative immunoassay.

An immunoassay is a biochemical test that measures the concentration of a substance in a biological sample, typically a fluid sample such as blood or serum, using the interaction of an antibody or antibodies to its antigen. The assay takes advantage of the highly specific binding of an antibody with its antigen. For the methods and assays described herein, specific binding of the target polypeptides with respective proteins or protein fragments, or an isolated peptide, or a fusion protein described herein occurs in the immunoassay to form a target protein/peptide complex. The complex is then detected by a variety of methods known in the art. An immunoassay also often involves the use of a detection antibody.

Enzyme-linked immunosorbent assay, also called ELISA, enzyme immunoassay or EIA, is a biochemical technique used mainly in immunology to detect the presence of an antibody or an antigen in a sample. The ELISA has been used as a diagnostic tool in medicine and plant pathology, as well as a quality control check in various industries.

In one embodiment, an ELISA involving at least one antibody with specificity for the desired antigen (*e.g.*, any of the targets as described herein) can also be performed. A known amount of sample and/or antigen is immobilized on a solid support (usually a polystyrene micro titer plate). Immobilization can be either non-specific (*e.g.*, by adsorption to the surface) or specific (*e.g.*, where another antibody immobilized on the surface is used to capture antigen or a primary antibody). After the antigen is immobilized, the detection antibody is added, forming a complex with the antigen. The detection antibody can be covalently linked to an enzyme or can itself be detected by a secondary antibody which is linked to an enzyme through bio-conjugation. Between each step the plate is typically washed with a mild detergent solution to remove any proteins or antibodies that are not specifically bound. After the final wash step the plate is developed by adding an enzymatic substrate to produce a visible signal, which indicates the quantity of antigen in the sample. Older ELISAs utilize chromogenic substrates, though newer assays employ fluorogenic substrates with much higher sensitivity.

In another embodiment, a competitive ELISA is used. Purified antibodies that are directed against a target polypeptide or fragment thereof are coated on the solid phase of multi-well plate, *i.e.*, conjugated to a solid surface. A second batch of purified antibodies that are not conjugated on any solid support is also needed. These nonconjugated purified antibodies are labeled for detection purposes, for example, labeled with horseradish peroxidase to produce a detectable signal. A sample (*e.g.*, a blood sample) from a subject is mixed with a known amount of desired antigen (*e.g.*, a known volume or concentration of a sample comprising a target polypeptide) together with the horseradish peroxidase labeled antibodies and the mixture is then added to coated wells to form competitive combination. After incubation, if the polypeptide level is high in the sample, a complex of labeled antibody reagent-antigen will form. This complex is free in solution and can be washed away. Washing the wells will remove the complex. Then the wells are incubated with TMB (3,3',5,5'-tetramethylbenzidene) color development substrate for localization of horseradish peroxidase-conjugated antibodies in the wells. There will be no color change or little color change if the target polypeptide level is high in the sample. If there is little or no target polypeptide present in the sample, a different complex in formed, the complex of solid support bound antibody reagents-target polypeptide. This complex is immobilized on the plate and is not washed away in the wash step. Subsequent incubation with TMB will produce significant color change. Such a competitive ELSA test is specific, sensitive, reproducible, and easy to operate.

There are other different forms of ELISA, which are well known to those skilled in the art. The standard techniques known in the art for ELISA are described in "Methods in Immunodiagnosis", 2nd Edition, Rose and Bigazzi, eds. John Wiley & Sons, 1980; and Oellerich, M. 1984, J. Clin. Chem. Clin. Biochem. 22:895-904. These references are hereby incorporated by reference in their entirety.

In one embodiment, the levels of a polypeptide in a sample can be detected by a lateral flow immunoassay test (LFIA), also known as the immunochromatographic assay, or strip test. LFIAs are a simple device intended to detect the presence (or absence) of antigen, e.g., a polypeptide, in a fluid sample. There are currently many LFIA tests used for medical diagnostics, either for home testing, point of care testing, or laboratory use. LFIA tests are a form of immunoassay in which the test sample flows along a solid substrate via capillary action. After the sample is applied to the test strip it encounters a colored reagent (generally comprising antibody specific for the test target antigen) bound to microparticles which mixes with the sample and transits the substrate encountering lines or zones which have been pretreated with another antibody or antigen. Depending upon the level of target polypeptides present in the sample the colored reagent can be captured and become bound at the test line or zone. LFIAs are essentially immunoassays adapted to operate along a single axis to suit the test strip format or a dipstick format. Strip tests are extremely versatile and can be easily modified by one skilled in the art for detecting an enormous range of antigens from fluid samples such as urine, blood, water, and/or homogenized tissue samples etc. Strip tests are also known as dip stick tests, the name bearing from the literal action of "dipping" the test strip into a fluid sample to be tested. LFIA strip tests are easy to use, require minimum training and can easily be included as components of point-of-care test (POCT) diagnostics to be use on site in the field. LFIA tests can be operated as either competitive or sandwich assays. Sandwich LFIAs are similar to sandwich ELISA. The sample first encounters colored particles which are labeled with antibodies raised to the target antigen. The test line will also contain antibodies to the same target, although it may bind to a different epitope on the antigen. The test line will show as a colored band in positive samples. In some embodiments of any of the aspects, the lateral flow immunoassay can be a double antibody sandwich assay, a competitive assay, a quantitative assay, or variations thereof. Competitive LFIAs are similar to competitive ELISA. The sample first encounters colored particles which are labeled with the target antigen or an analogue. The test line contains antibodies to the target/its analogue. Unlabelled antigen in the sample will block the binding sites on the antibodies preventing uptake of the colored particles. The test line will show as a colored band in negative samples. There are several variations on lateral flow technology. It is also possible to apply multiple capture zones to create a multiplex test.

The use of "dip sticks" or LFIA test strips and other solid supports have been described in the art in the context of an immunoassay for a number of antigen biomarkers. U.S. Pat. Nos. 4,943,522; 6,485,982; 6,187,598; 5,770,460; 5,622,871; 6,565,808, U. S. patent applications Ser. No. 10/278,676; U.S. Ser. No. 09/579,673 and U.S. Ser. No. 10/717,082, which are incorporated herein by reference in their entirety, are non-limiting examples of such lateral flow test devices. Examples of patents that describe the use of "dip stick" technology to detect soluble antigens via immunochemical assays include but are not limited to US Patent Nos. 4,444,880; 4,305,924; and 4,135,884; which are incorporated by reference herein in their entireties. The apparatuses and methods of these three patents broadly describe a first component fixed to a solid surface on a "dip stick" which is exposed to a solution containing a soluble antigen that binds to the component fixed upon the "dip stick," prior to detection of the component-antigen complex upon the stick. It is within the skill of one in the art to modify the teachings of this "dip stick" technology for the detection of polypeptides using antibody reagents as described herein.

Other techniques can be used to detect the level of a polypeptide in a sample. One such technique is the dot blot, an adaptation of Western blotting (Towbin et al., Proc. Nat. Acad. Sci. 76:4350 (1979)). In a Western blot, the polypeptide or fragment thereof can be dissociated with detergents and heat and separated on an SDS-PAGE gel before being transferred to a solid support, such as a nitrocellulose or PVDF membrane. The membrane is incubated with an antibody reagent specific for the target polypeptide or a fragment thereof. The membrane is then washed to remove unbound proteins and proteins with non-specific binding. Detectably labeled enzyme-linked secondary or detection antibodies can then be used to detect and assess the amount of polypeptide in the sample tested. A dot blot immobilizes a protein sample on a defined region of a support, which is then probed with antibody and labelled secondary antibody as in Western blotting. The intensity of the signal from the detectable label in either format corresponds to the amount of enzyme present, and therefore the amount of polypeptide. Levels can be quantified, for example by densitometry.

In some embodiments of any of the aspects, the level of a target can be measured, by way of non-limiting example, by Western blot; immunoprecipitation; enzyme-linked immunosorbent assay (ELISA); radioimmunological assay (RIA); sandwich assay; fluorescence in situ hybridization (FISH); immunohistological staining; radioimmunometric assay; immunofluorescence; chromogenic assay; mass spectroscopy and/or immunoelectrophoresis assay.

In certain embodiments, the gene expression products as described herein can be instead determined by determining the level of messenger RNA (mRNA) expression of the genes described herein. Such molecules can be isolated, derived, or amplified from a biological sample, such as a blood sample. Techniques for the detection of mRNA expression is known by persons skilled in the art, and can include but not limited to, PCR procedures, RT-PCR, quantitative RT-PCR Northern blot analysis, differential gene expression, RNAse protection assay, microarray-based analysis, next-generation sequencing, hybridization methods, *etc.*

In general, the PCR procedure describes a method of gene amplification which is comprised of (i) sequence-specific hybridization of primers to specific genes or sequences within a nucleic acid sample or library, (ii) subsequent amplification involving multiple rounds of annealing, elongation, and denaturation using a thermostable DNA polymerase, and (iii) screening the PCR products for a band of the correct size. The primers used are oligonucleotides of sufficient length and appropriate sequence to provide initiation of polymerization, *i.e.*, each primer is specifically designed to be complementary to a strand of the genomic locus to be amplified. In an alternative embodiment, mRNA level of gene expression products described herein can be determined by reverse-transcription (RT) PCR and by quantitative RT-PCR (QRT-PCR) or real-time PCR methods. Methods of RT-PCR and QRT-PCR are well known in the art.

In some embodiments of any of the aspects, the level of an mRNA can be measured by a quantitative sequencing technology, *e.g.*, a quantitative next-generation sequence technology. Methods of sequencing a nucleic acid sequence are well known in the art. Briefly, a sample obtained from a subject can be contacted with one or more primers which specifically hybridize to a single-strand nucleic acid sequence flanking the target gene sequence and a complementary strand is synthesized. In some next-generation technologies, an adaptor (double or single-stranded) is ligated to nucleic acid molecules in the sample and synthesis proceeds from the adaptor or adaptor compatible primers. In some third-generation technologies, the sequence can be determined, *e.g.*, by determining the location and pattern of the hybridization of probes or measuring one or more characteristics of a single molecule as it passes through a sensor (*e.g.*, the modulation of an electrical field as a nucleic acid molecule passes through a nanopore). Exemplary methods of sequencing include, but are not limited to, Sanger sequencing, dideoxy chain termination, high-throughput sequencing, next generation sequencing, 454 sequencing, SOLiD sequencing, polony sequencing, Illumina sequencing, Ion Torrent sequencing, sequencing by hybridization, nanopore sequencing, Helioscope sequencing, single molecule real time sequencing, RNAP sequencing, and the like. Methods and protocols for performing these sequencing methods are known in the art, see, *e.g.*, "Next Generation Genome Sequencing" Ed. Michal Janitz, Wiley-VCH; "High-Throughput Next Generation Sequencing" Eds. Kwon and Ricke, Humanna Press, 2011; and Sambrook et al., Molecular Cloning: A Laboratory Manual (4 ed.), Cold Spring Harbor Laboratory Press, Cold Spring Harbor, N.Y., USA (2012); which are incorporated by reference herein in their entireties.

The nucleic acid sequences of the genes described herein have been assigned NCBI accession numbers for different species such as human, mouse and rat. For example, the human TTC21B, MCM3, MTUS1, FOXM1, CUL4A, GPC1, PCNA, IRF7, BRIP1, ULK1, and SPAG5, nucleic acid sequences (*e.g.*, SEQ ID NO: 1-11) and mRNA is known. Similarly, the NCBI accession number is also known for cancer reference markers ALAS1, MMADHC, PPP2CA, TFRC, and CLTC (*e.g.*, SEQ ID NO: 12-16). Accordingly, a skilled artisan can design an appropriate primer based on the known sequence for determining the nucleic acid or mRNA level of the respective gene.

Nucleic acid and ribonucleic acid (RNA) molecules can be isolated from a particular biological sample using any of a number of procedures, which are well-known in the art, the particular isolation procedure chosen being appropriate for the particular biological sample. For example, freeze-thaw and alkaline lysis procedures can be useful for obtaining nucleic acid molecules from solid materials; heat and alkaline lysis procedures can be useful for obtaining nucleic acid molecules from urine; and proteinase K extraction can be used to obtain nucleic acid from blood (Roiff, A et al. PCR: Clinical Diagnostics and Research, Springer (1994)).

In some embodiments of any of the aspects, one or more of the reagents (*e.g.*, an antibody reagent and/or nucleic acid probe) described herein can comprise a detectable label and/or comprise the ability to generate a detectable signal (*e.g.*, by catalyzing reaction converting a compound to a detectable product). Detectable labels can comprise, for example, a light-absorbing dye, a fluorescent dye, or a radioactive label. Detectable labels, methods of detecting them, and methods of incorporating them into reagents (*e.g.*, antibodies and nucleic acid probes) are well known in the art.

In some embodiments of any of the aspects, detectable labels can include labels that can be detected by spectroscopic, photochemical, biochemical, immunochemical, electromagnetic, radiochemical, or chemical means, such as fluorescence, chemifluoresence, or chemiluminescence, or any other appropriate means. The detectable labels used in the methods described herein can be primary labels (where the label comprises a moiety that is directly detectable or that produces a directly detectable moiety) or secondary labels (where the detectable label binds to another moiety to produce a detectable signal, *e.g.*, as is common in immunological labeling using secondary and tertiary antibodies). The detectable label can be linked by covalent or non-covalent means to the reagent. Alternatively, a detectable label can be linked such as by directly labeling a molecule that achieves binding to the reagent via a ligand-receptor binding pair arrangement or other such specific recognition molecules. Detectable labels can include, but are not limited to radioisotopes, bioluminescent compounds, chromophores, antibodies, chemiluminescent compounds, fluorescent compounds, metal chelates, and enzymes.

In other embodiments, the detection reagent is label with a fluorescent compound. When the fluorescently labeled reagent is exposed to light of the proper wavelength, its presence can then be detected due to fluorescence. In some embodiments of any of the aspects, a detectable label can be a fluorescent dye molecule, or fluorophore including, but not limited to fluorescein, phycoerythrin, phycocyanin, o-phthaldehyde, fluorescamine, Cy3™, Cy5™, allophycocyanine, Texas Red, peridinin chlorophyll, cyanine, tandem conjugates such as phycoerythrin-Cy5™, green fluorescent protein, rhodamine, fluorescein isothiocyanate (FITC) and Oregon Green™, rhodamine and derivatives (*e.g.,* Texas red and tetrarhodimine isothiocynate (TRITC)), biotin, phycoerythrin, AMCA, CyDyes™, 6-carboxyfhiorescein (commonly known by the abbreviations FAM and F), 6-carboxy-2',4',7',4,7-hexachlorofiuorescein (HEX), 6-carboxy-4',5'-dichloro-2',7'-dimethoxyfiuorescein (JOE or J), N,N,N',N'-tetramethyl-6carboxyrhodamine (TAMRA or T), 6-carboxy-X-rhodamine (ROX or R), 5-carboxyrhodamine-6G (R6G5 or G5), 6-carboxyrhodamine-6G (R6G6 or G6), and rhodamine 110; cyanine dyes, *e.g.,* Cy3, Cy5 and Cy7 dyes; coumarins, *e.g.,* umbelliferone; benzamide dyes, *e.g.,* Hoechst 33258; phenanthridine dyes, *e.g.,* Texas Red; ethidium dyes; acridine dyes; carbazole dyes; phenoxazine dyes; porphyrin dyes; polymethine dyes, *e.g.,* cyanine dyes such as Cy3, Cy5, *etc.*; BODIPY dyes and quinoline dyes. In some embodiments of any of the aspects, a detectable label can be a radiolabel including, but not limited to $^{3}$H, $^{125}$I, $^{35}$S, $^{14}$C, $^{32}$P, and $^{33}$P. In some embodiments of any of the aspects, a detectable label can be an enzyme including, but not limited to horseradish peroxidase and alkaline phosphatase. An enzymatic label can produce, for example, a chemiluminescent signal, a color signal, or a fluorescent signal. Enzymes contemplated for use to detectably label an antibody reagent include, but are not limited to, malate dehydrogenase, staphylococcal nuclease, delta-V-steroid isomerase, yeast alcohol dehydrogenase, alpha-glycerophosphate dehydrogenase, triose phosphate isomerase, horseradish peroxidase, alkaline phosphatase, asparaginase, glucose oxidase, beta-galactosidase, ribonuclease, urease, catalase, glucose-VI-phosphate dehydrogenase, glucoamylase and acetylcholinesterase. In some embodiments of any of the aspects, a detectable label is a chemiluminescent label, including, but not limited to lucigenin, luminol, luciferin, isoluminol, theromatic acridinium ester, imidazole, acridinium salt and oxalate ester. In some embodiments of any of the aspects, a detectable label can be a spectral colorimetric label including, but not limited to colloidal gold or colored glass or plastic (*e.g.*, polystyrene, polypropylene, and latex) beads.

In some embodiments of any of the aspects, detection reagents can also be labeled with a detectable tag, such as c-Myc, HA, VSV-G, HSV, FLAG, V5, HIS, or biotin. Other detection systems can also be used, for example, a biotin-streptavidin system. In this system, the antibodies immunoreactive (*i.e.*, specific for) with the biomarker of interest is biotinylated. Quantity of biotinylated antibody bound to the biomarker is determined using a streptavidin-peroxidase conjugate and a chromogenic substrate. Such streptavidin peroxidase detection kits are commercially available, *e.g.*, from DAKO; Carpinteria, CA. A reagent can also be detectably labeled using fluorescence emitting metals such as $^{152}$Eu, or others of the lanthanide series. These metals can be attached to the reagent using such metal chelating groups as diethylenetriaminepentaacetic acid (DTPA) or ethylenediaminetetraacetic acid (EDTA).

An expression value or risk score which is less than a reference expression value or reference risk score can be an expression value which is less by at least about 10%, at least about 20%, at least about 50%, at least about 60%, at least about 80%, at least about 90%, or less relative to the reference expression value or reference risk score. In some embodiments of any of the aspects, an expression value or risk score which is less than a reference expression value or risk score can be an expression value or risk score which is statistically significantly less than the reference expression value or risk score.

An expression value or risk score which is more than a reference expression value or a reference risk score can be an expression value or risk score which is greater by at least about 10%, at least about 20%, at least about 50%, at least about 60%, at least about 80%, at least about 90%, at least about 100%, at least about 200%, at least about 300%, at least about 500% or more or less than the reference expression value or reference risk score. In some embodiments of any of the aspects, an expression value or a risk score which is more or less than a reference expression value or a reference risk score can be an expression value or a risk score which is statistically significantly greater than the reference expression value or reference risk score.

In some embodiments of any of the aspects, the reference can be an expression value of the target molecule in a population of subjects who do not have or are not diagnosed as having, and/or do not exhibit signs or symptoms of cancer. In some embodiments of any of the aspects, the reference can also be an expression value of the target molecule in a control sample, a pooled sample of control individuals or a numeric value or range of values based on the same. In some embodiments of any of the aspects, the reference can be the expression value of a target molecule in a sample obtained from the same subject at an earlier point in time, *e.g.*, the methods described herein can be used to determine if a subject's sensitivity or response to a given therapy is changing over time.

In some aspect, the risk score can be used when using a combination of gene expression levels to determine the outcome or direction to take for the subject with cancer or having been diagnosed with cancer, or for the subject suspected of having cancer, or who is undergoing treatment for cancer. The "direction of effect" where genes are increased or decreased in a cancer patient are based on single gene models of the adverse pathology (AP) outcome. However, the effect of the direction of gene expression levels can change when there are multiple genes (2 or more) in a signature (also known as modified expression). A more general description that is accurate for any gene combination and implicitly incorporates the directions of effects would be using a risk score (related to modified expression). To calculate a risk score, a weighted linear combination of normalised expression values of multiple genes is used to calculate the risk score for any gene combination. The risk score estimates the probability of, for example, aggressive cancer; larger values of the risk score indicate a higher probability of aggressive cancer and *vice versa.* The same is true for determining the efficacy of treatment, or determining whether a treatment is suitable, or whether the outcome for the cancer patient is poor or good prognostically. For example, the term "combination of genes" or "risk score" should be understood to mean a linear combination, or weighted sum, of the normalised expression of said genes. Larger values of a risk score indicate higher risk of aggressive or recurrent cancer, a poor outcome, a lower 5-year or 10-year survival rate, treatment not working, and the like. Smaller values of a risk score indicate lower risk of aggressive or recurrent cancer, better outcome, higher rate of 5-year and/or 10-year survival rate, a treatment that is working, and the like. The risk score for a combination of N genes is calculated as follows:

$$\text{Risk score} = W_1 \times G_1 + W_2 \times G_2 + \ldots + W_N \times G_N \qquad (Equation\ 1)$$

Where,

W = A gene-specific real number constant that can be positive or negative
G = A gene-specific normalised expression value

In some embodiments of any of the aspects, the expression value of expression products of no more than 200 other genes is determined. In some embodiments of any of the aspects, the expression value of expression products of no more than 100 other genes is determined. In some embodiments of any of the aspects, the expression value of expression products of no more than 20 other genes is determined. In some embodiments of any of the aspects, the expression value of expression products of no more than 15 other genes is determined. In some embodiments of any of the aspects, the expression value of expression products of no more than 10 other genes is determined.

In some embodiments of the foregoing aspects, the expression value of a given gene can be normalized relative to the expression value of one or more reference genes or reference proteins.

In some embodiments, the reference value can be the expression value in a sample of similar cell type, sample type, sample processing, and/or obtained from a subject of similar age, sex and other demographic parameters as the sample/subject for which the expression value of one or more of TTC21B, MCM3, MTUS1, FOXM1, CUL4A, GPC1, PCNA, IRF7, BRIP1, ULK1, and SPAG5, FOXM1 is to be determined. In some embodiments, the test sample and control reference sample are of the same type, that is, obtained from the same biological source, and comprising the same composition, *e.g.*, the same number and type of cells.

The test sample can be obtained by removing a sample from a subject but can also be accomplished by using a previously isolated sample (*e.g.*, isolated at a prior timepoint and isolated by the same or another person).

In some embodiments of any of the aspects, the test sample can be an untreated test sample. As used herein, the phrase "untreated test sample" refers to a test sample that has not had any prior sample pre-treatment except for dilution and/or suspension in a solution. Exemplary methods for treating a test sample include, but are not limited to, centrifugation, filtration, sonication, homogenization, heating, freezing, and thawing, and combinations thereof. In some embodiments of any of the aspects, the test sample can be a frozen test sample, *e.g.*, a frozen tissue. The frozen sample can be thawed before employing methods, assays and systems described herein. After thawing, a frozen sample can be centrifuged before being subjected to methods, assays and systems described herein. In some embodiments of any of the aspects, the test sample is a clarified test sample, for example, by centrifugation and collection of a supernatant comprising the clarified test sample. In some embodiments of any of the aspects, a test sample can be a pre-processed test sample, for example, supernatant or filtrate resulting from a treatment selected from the group consisting of centrifugation, filtration, thawing, purification, and any combinations thereof. In some embodiments of any of the aspects, the test sample can be treated with a chemical and/or biological reagent. Chemical and/or biological reagents can be employed to protect and/or maintain the stability of the sample, including biomolecules (*e.g.*, nucleic acid and protein) therein, during processing. One exemplary reagent is a protease inhibitor, which is generally used to protect or maintain the stability of protein during processing. The skilled artisan is aware of methods and processes appropriate for pre-processing of biological samples required for determination of the level of an expression product as described herein.

In some embodiments of any of the aspects, the methods, assays, and systems described herein can further comprise a step of obtaining or having obtained a test sample from a subject. In some embodiments of any of the aspects, the subject can be a human subject. In some embodiments of any of the aspects, the subject can be a subject in need of treatment for (*e.g.*, having or diagnosed as having prostate cancer) or a subject at risk of or at increased risk of developing prostate cancer as described elsewhere herein.

In some embodiments of any of the aspects, the sample obtained from a subject can be a biopsy sample. In some embodiments of any of the aspects, the sample obtained from a subject can be a blood or serum sample.

## Brief Description of the Drawings

The invention will be more clearly understood from the following description of an embodiment thereof, given by way of example only, with reference to the accompanying drawings, in which:-

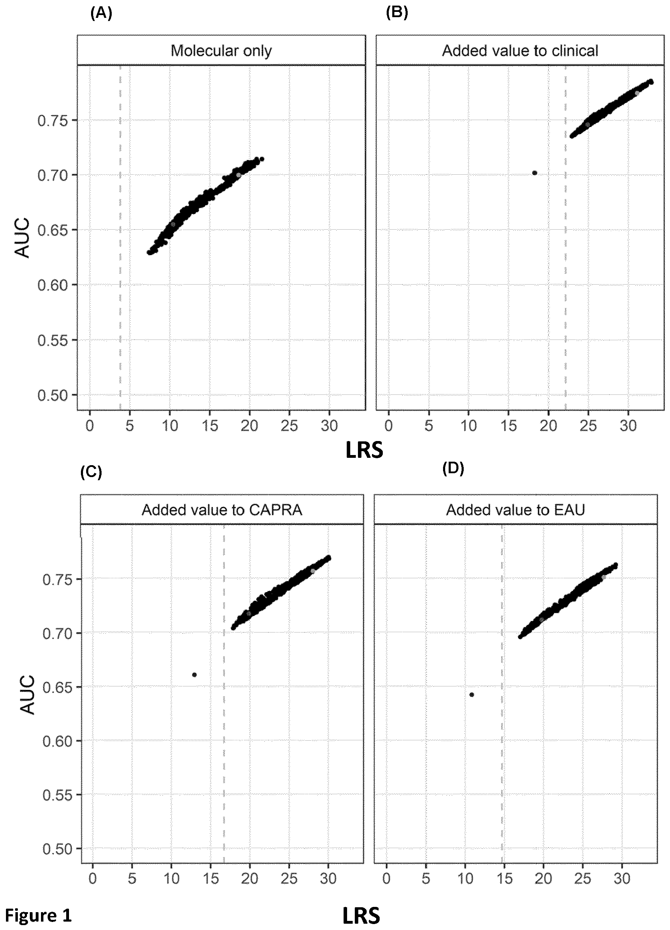

**Figure 1** illustrates four plots (A)-(D) showing the prognostic performance for adverse pathology (AP) outcome for 512 combinations of 2 to 11 genes that include MTUS1 and TTC21B, and MTUS1 and TTCC21B one or more of the other 9 genes (Figure 1(A)). The combination of the two genes MTUS1 and TTC21B are highlighted as the red dot, which shows that the combination is significantly prognostic (p-value = 0.0013) and adds significant prognostic value to benchmark clinical information (Figure 1(B), optimally weighted biopsy information consisting of Gleason score, clinical stage and PSA, p-value = 0.0101), CAPRA risk score (Figure 1(C), clinical information consisting of Cancer of the Prostate Risk Assessment (CAPRA) 0 to 10 score, p-value = 0.0082) and EAU risk categories (Figure 1(D), clinical information consisting of the European Association of Urology risk categories of low, intermediate, or high, p-value = 0.0028). The AP is determined by the cross-validated likelihood ratio statistic (LRS) by area under the ROC curve (AUC). Larger values of LRS and AUC indicate better prognostic performance, so dots closer to the top right of each plot represent gene combinations with better prognostic performance. The benchmark clinical information is shown by the blue dot, single genes are indicated by black dots, the two gene combination MTUS1 and TTC21B is indicated by the red dot, and the four gene combination of MTUS1, TTC21B, MCM3 and FOXM1 is indicated by the green dot. Values to the right of the dashed line are statistically significant at LRS p < 0.05.

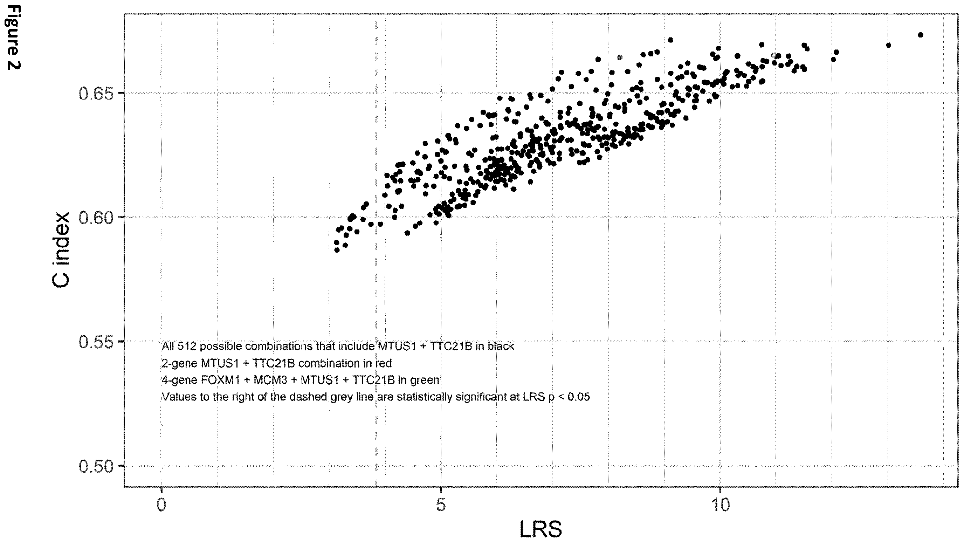

**Figure 2** illustrates the prognostic performance for biochemical recurrence of the 512 gene combinations. Each dot represents 1 of 512 unique gene combinations. The red dot is the 2-gene combination MTUS1 and TTC21B. The green dot is the 4-gene combination FOXM1, MCM3, MTUS1, and TTC21B. Prognostic performance is measured by the likelihood ratio statistic (LRS) and the C index from cross-validation analysis. Larger values of LRS and C index indicate better prognostic performance, so dots closer to the top right of the plot represent gene combinations with better prognostic performance. Values to the right of the dashed vertical line are statistically significant at LRS p-value < 0.05.

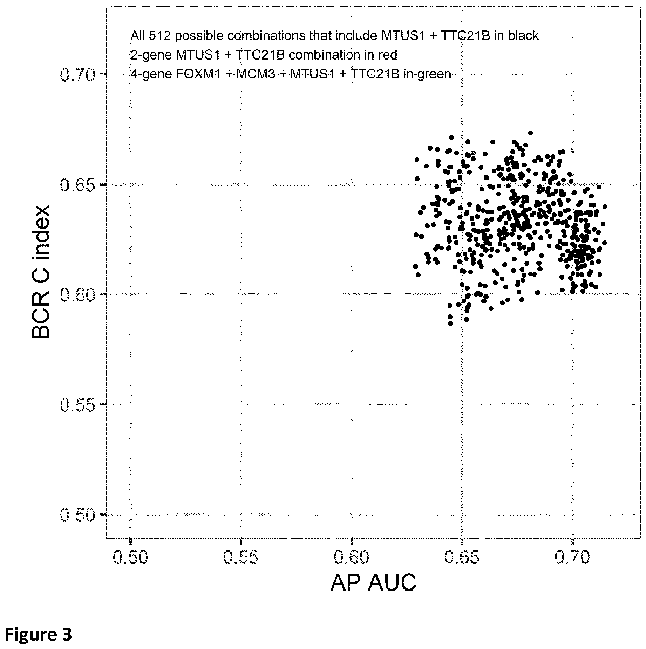

**Figure 3** illustrates a plot that shows the prognostic performance for adverse pathology and biochemical recurrence of the 512 gene combinations. The two-gene combination MTUS1 and TTC21B is indicated by the red dot and the four-gene combination FOXM1, MCM3, MTUS1, and TTC21B is indicated by the green dot. Prognostic performance is measured by the area under the ROC curve (AUC) and the C index from cross-validation analysis. Larger values of AUC and C index indicate better prognostic performance, so dots closer to the top right of the plot represent gene combinations with better prognostic performance.

## Detailed Description of the Drawings

### *Definitions*

In this specification, the term "cancer sample" should be understood to mean tumour cells, tumour tissue, or other biological material derived from a tumour, for example conditioned media.

The term "sample", "biological sample", or "test sample" as used herein denotes a sample taken or isolated from a biological organism, *e.g.*, a blood or plasma sample from a subject. In some embodiments of any of the aspects, the present invention encompasses several examples of a biological sample. In some embodiments of any of the aspects, the biological sample is cells, or tissue, or peripheral blood, or bodily fluid. Exemplary biological samples include, but are not limited to, a biopsy, a biological sample (for example, a prostate cancer sample, a colorectal cancer sample, a lung cancer sample, a head and neck cancer sample, a sample for examining a pre-cancerous condition (for example, a Barrett's esophagus endoscopic sample) and the like), biofluid sample; blood; blood derivatives; serum; plasma; urine; sperm; mucus; tissue biopsy; organ biopsy; synovial fluid; bile fluid; cerebrospinal fluid; mucosal secretion; effusion; sweat; saliva; and/or tissue sample *etc.* The term also includes a mixture of the above-mentioned samples. The term "test sample" also includes untreated or pretreated (or pre-processed) biological samples. In some embodiments of any of the aspects, a test sample can comprise cells from a subject. In some embodiments of any of the aspects, the test sample can be a urine sample or a tissue sample from a subject where the urine sample or the tissue sample may contain tumour cells. In addition to the definition summarised above, the sample can also include non-tumour tissue, a pre-cancerous tissue, conditioned media, or formalin-fixed paraffin-embedded (FFPE) tumour or non-tumour tissue.

In the specification, the term "positive expression" as applied to a gene or a protein encoded by that gene should be understood to mean a level of expression of the gene or protein encoded by that gene (an expression value) that is increased above an average level of expression of the same gene or protein encoded by that same gene found in a cohort of matched control individuals with cancer (the "control group"). The cohort of matched individuals may consist of individuals who did not experience a recurrence of a cancer following surgery to remove the cancer. In relation to controls, the usual practise for one skilled in the art would be to use a 'standard' control, for example, for Immunohistochemistry (IHC), a cell line or cell lines where the expression level of the biomarker is known, or for qPCR (quantitative Polymerase Chain Reaction), a similar standard control or a pool of a number of samples is known.

In the specification, the term "dysregulated expression" or "modified expression" should be understood to mean a normalised expression value that is increased over or decreased below a normalised expression value of the same gene or combination of genes found in a cohort of matched individuals with cancer that did not recur following surgery to remove the cancer or following non-surgical treatment, or found in a cohort of matched individuals who are cancer free or who have never been diagnosed with cancer. Put another way, the terms should be understood to mean that a risk score calculated from the expression of a gene or combination of genes is larger or smaller than the risk score calculated from the expression of the same gene or combination of genes in individuals with cancer that did not recur following surgery to remove the cancer or following non-surgical treatment, or found in a cohort of matched individuals who are cancer free or who have never been diagnosed with cancer.

In the specification, the term "normalised gene expression" should be understood to mean the expression value of a gene adjusted by the expression value of a control or a reference gene(s). In this instance, the reference genes used are PPP2CA, CLTC, ALAS1, MMADHC and/or TFRC (SEQ ID NOs 12-16). Normalised gene expression for a gene G (or a combination of genes G) is calculated as follows:

$$\text{Normalised gene expression} = (\text{Mean RT-qPCR quantification cycle value}$$
$$\text{of a (set of) reference gene(s))} - (\text{RT-qPCR quantification cycle value of}$$
$$\text{gene G (or a combination of genes G))} \qquad (Equation\ 2)$$

The terms "normal expression" as applied to a gene or protein should be understood to mean a level of expression of the gene (or protein encoded by that gene) that is equivalent to a level of expression of the same gene or protein encoded by that same gene found in a cohort of matched control individuals with cancer. The cohort of matched individuals may consist of individuals who did not experience a recurrence of a cancer following surgery to remove the cancer.

The method used to set thresholds is different for the microarray analysis, qRT-PCR analysis, and protein expression. For microarrays, the threshold is relative (samples were split into three equal groups, so the threshold is dataset dependent), and for the qPCR and protein expression it is set at specific points. For RNA (microarrays), expression levels of 'low', 'moderate' and 'high' refer to expression values that fall within the lower, middle or upper third of the expression range; or alternatively, 'low' and 'high' expression can refer to expression values that fall within the lower or upper half of the expression range. For qRT-PCR and protein expression levels, specific thresholds have been set, but in general, the term "dysregulated" refers to tumours with expression values falling above or below set values in the range of expression. For the terms "moderate" and "normal", the terms refer to tumours with expression values falling within set values in the range of expression. For example, the gene expression values (or proteins) of the at least 2 of the 11 genes are first normalised against the gene expression values (or proteins) of the reference genes PPP2CA, CLTC, ALAS1, MMADHC and/or TFRC in the same biological sample, and the Ct value of each of the 16 genes is obtained. The mean expression level of all reference genes is calculated, and the mean RefCt value is obtained. The normalised expression level ($\Delta$CTgene1) of each of the 11 genes is calculated by subtracting the expression level of the gene of interest (Ctgene1) from the mean RefCt value (mean RefCt - Ctgene1 = $\Delta$CTgene1). The $\Delta$CT for at least 2 of the 11 genes is then obtained and the combined normalised expression value calculated.

Typically, the normalised qRT-PCR thresholds for 'modified' expression are 0.7 and 1.99. Typically, the normalised protein thresholds (using IHC) are 1% and 50% of positive cells. That is, a modified score here refers to a tumour with >1% and <50% tumour cells positive for the normalised gene expression values of the at least 2 of the 11 genes (or proteins). These values may be adjusted based on any new data, but the same theory applies for the terms "normal", "moderate" and "dysregulated" with respect to expression levels of the at least 2 of the 11 genes (or proteins).

In the specification, the term "adjuvant therapy" should be understood to mean any treatment given after primary treatment to increase the chances of long-term survival. In the specification, the term "neoadjuvant therapy" should be understood to mean treatment given before primary treatment to increase the chances of long-term survival. Primary treatment is generally surgery. Adjuvant therapy and neoadjuvant therapy are generally selected from chemotherapy, hormonal therapy, targeted therapy, radiation therapy, immunotherapy or a combination thereof.

In the specification, the term "active surveillance" should be understood to mean monitoring the cancer closely.

Usually this includes a doctor visit with a prostate-specific antigen (PSA) blood test about every 6 months and a digital rectal exam (DRE) at least once a year. Prostate biopsies and imaging tests may be done every 1 to 3 years as well. Active surveillance may be best suited if you have a low Gleason score (usually 6 or lower), which indicates a less aggressive, slower growing form of cancer.

Detection of expression generally involves measuring the RNA expression of a selected panel of prognostic genes in formalin-fixed paraffin-embedded (FFPE) tissue specimens from prostate cancer biopsies using reverse transcriptase quantitative polymerase chain reaction (RT-qPCR) assays. Other means or measuring include immunohistological staining of a tumour biopsy tissue or a control biopsy tissue using suitable means such as immunohistochemical staining. Many other means of detecting the biomarkers of the invention will be apparent to those skilled in the art. For example, RNA sequencing, quantitative polymerase chain reaction (qPCR), reverse transcriptase PCR (RT-PCR), quantitative real time RT-PCR (qRT-PCR), ELISA, Western Blot, protein determination on polyacrylamide gels, and the like.

In this specification, the term "cancer" should be understood to mean a cancer that is treated by chemotherapeutic regimens. An example of such a cancer include multiple myeloma, prostate cancer, glioblastoma, lymphoma, fibrosarcoma; myxosarcoma; liposarcoma; chondrosarcom; osteogenic sarcoma; chordoma; angiosarcoma; endotheliosarcoma; lymphangiosarcoma; lymphangioendotheliosarcoma; synovioma; mesothelioma; Ewing's tumour; leiomyosarcoma; rhabdomyosarcoma; colon carcinoma; pancreatic cancer; ovarian cancer; squamous cell carcinoma; basal cell carcinoma; adenocarcinoma; sweat gland carcinoma; sebaceous gland carcinoma; papillary carcinoma; papillary adenocarcinomas; cystadenocarcinoma; medullary carcinoma; bronchogenic carcinoma; renal cell carcinoma; hepatoma; bile duct carcinoma; choriocarcinoma; seminoma; embryonal carcinoma; Wilms' tumour; cervical cancer; uterine cancer; testicular tumour; lung carcinoma; small cell lung carcinoma; bladder carcinoma; epithelial carcinoma; glioma; astrocytoma; medulloblastoma; craniopharyngioma; ependymoma; pinealoma; hemangioblastoma; acoustic neuroma; oligodendroglioma; meningioma; melanoma; retinoblastoma; head and neck cancer types (in the sinuses, inside and behind the nose, mouth (tongue, gums, roof of the mouth, back of the mouth), throat (nasopharynx, oropharynx, hypopharynx), larynx, lips, salivary glands)); bladder cancer types; colorectal cancers; and leukemias.

In the specification, the term "pre-cancer" should be understood to mean abnormal cells which have undergone some changes that are associated with an increased risk of becoming cancerous but are not yet cancer. Examples of such pre-cancer conditions include Barrett's oesophagus, Bowen's disease, Familial adenomatous polyposis (FAP), Lobular carcinoma in situ (LCIS), Lynch syndrome, MEN2, Vaginal intra-epithelial neoplasia (VAIN), and Vulval intraepithelial neoplasia (VIN). Pre-cancer conditions are included in the diagnosing whether a subject is at risk of having an aggressive cancer or at risk of having a recurrence of cancer.

In the specification, the term "recurrence" should be understood to mean the recurrence of the cancer, which is being sampled in the patient, in which the cancer has returned to the sampled area after treatment, for example, if sampling colon or prostate cancer, recurrence of the colon or prostate cancer in the source tissue. The term should also be understood to mean recurrence of a primary cancer whose site is different to that of the cancer initially sampled, that is, the cancer has returned to a non-sampled area after treatment, such as non-locoregional recurrences.

In this specification, the term "poor outcome" should be understood to mean that the chances of disease-free survival are low.

In the specification, the term "indolent" should be understood to mean a cancer that is typically slow-growing (lazy) and which would not become symptomatic in an individual's lifetime and would not contribute to death.

In the specification, the term "survival rate" should be understood to mean the period of time during which a patient diagnosed with cancer such as colon or prostate cancer, will likely survive. The survival rate is expressed as a 5-year survival rate, a 10-year survival rate, a 15-year survival rate, a 20-year survival rate, a 25-year survival rate, a 30-year survival rate, a 35-year survival rate, a 40-year survival rate, a 45-year survival rate, or a 50-year survival rate. Ideally, the survival rate is expressed as a 5-year survival rate or a 10-year survival rate.

In this specification, the term "treatment" should be understood to mean its generally accepted meaning which encompasses prohibiting, preventing, restraining, and slowing, stopping or reversing progression or severity of a metastatic, recurrent or existing cancer phenotype.

In this specification, the term "at least one" should be understood to mean and encompass that at least one, at least two, at least three, at least four, at least five, at least six, at least seven, at least eight, at least nine, at least ten, or all genes can be selected from the group consisting of TTC21B, MCM3, MTUS1, FOXM1, CUL4A, GPC1, PCNA, IRF7, BRIP1, ULK1, and SPAG5. Preferably, the at least one gene is one, two or all of TTC21B, MTUS1, MCM3 and FOXM1.

In this specification, the term "at least two" should be understood to mean and encompass that at least two, at least three, at least four, at least five, at least six, at least seven, at least eight, at least nine, at least ten, or all genes can be selected from the group consisting of TTC21B, MCM3, MTUS1, FOXM1, CUL4A, GPC1, PCNA, IRF7, BRIP1, ULK1, and SPAG5 . Preferably, the at least two genes are TTC21B and MTUS1, MTUS1 and PCNA, MCM3 and TTC21B, MCM3 and MTUS1, or a combination of those pairs. Preferably the at least three genes are MTUS1, MCM3, and TTC21B. Preferably, the at least four genes selected are MTUS1, MCM3, TTC21B, and FOXM1.

*Materials and Methods*

**RNA sequencing**

Total RNA was extracted from proliferating and senescent HMECs using the RNeasy kit (Qiagen). Polyadenylated RNA species were enriched from 5µg total RNA, and sequencing libraries were prepared from PolyA+ RNA using the TruSeq Sample Prep kit (Illumina). Libraries were used directly for cluster generation and sequencing analysis using the Genome Analyser II (Illumina) following the protocol of the manufacturer. Base calling and mapping to the human genome (build hg19) were performed using the BWA sequence alignment tool. The mRNA fold changes were calculated based on the total number of sequence reads mapped per gene in the two experiments.

**Real-Time Quantitative PCR**

Total RNA was extracted from cells using the RNeasy kit (Qiagen) according to manufacturer's protocol. 1ug RNA was used to generate cDNA by reverse transcriptase PCR using the TaqMan Reverse Transcription kit (Applied Biosytems). Relative mRNA expression levels were determined using the SYBR Green I detection chemistry (Applied Biosystems) on the ABI Prism 7500 Fast Real-Time PCR System. The ribosomal constituent RPLPO was used as a control gene for normalization (SEQ ID NO: XX (Forward - CCC) and SEQ ID NO: YY (Reverse - CCC). Primer sequence pairs used are as follows (For = Forward Primer; Rev = Reverse Primer):

*Sequences*

SEQ ID NO: 1 (BRIP1; NM_032043):

aggcgggaattcgtctcgggttgtgtggttgaggggtctggtgggtcgaggaaaggtaacggcggccccagtcctgcacaca

aggccggggaagtagcagcacccccaggaagagggaggaggaagggctcgtgccctttcttctcttccagggctccgcttt

atttgctctcagaagtcggtttcctttccttttcttcagtgaatcggagctcagagcgttgcttcggtttccctccagacagttaggaat

ctgaaataaacaggaaagcactatgtcttcaatgtggtctgaatatacaattggtgggggtgaagatttactttccttataaagctta

cccgtcacagcttgctatgatgaattctattctcagaggattaaacagcaagcaacattgtttgttggagagtcccacaggaagt

ggaaaaagcttagccttactttgttctgctttagcatggcaacaatctcttagtgggaaaccagcagatgagggcgtaagtgaa

aaagctgaagtacaattgtcatgttgttgtgcatgccattcaaaggattttacaaacaatgacatgaaccaaggaacttcacgt

catttcaactatccaagcacaccaccttctgaaagaaatggcacttcatcaacttgtcaagactcccctgaaaaaaccactctg

gctgcaaagttatctgctaagaaacaggcatccatatacagagatgaaaatgatgattttcaagtagagaagaaaagaattc

gacccttagaaactacacagcagattagaaaacgtcattgctttggaacagaagtacacaatttggatgcaaaagttgattca

ggaaagactgtaaaactcaactctccactggaaaagataaactccttttcgccacagaaacccccctggccactgttctaggtg

ctgttgttctactaaacaaggaaacagtcaagagtcatcgaataccattaagaaggatcatacagggaaatccaagataccc

aaaatatattttgggacacgcacacacaagcagattgctcagattactagagagctccggaggacggcatattcaggggttc

caatgactattctttccagcagggatcatacttgtgtccatcctgaggtagtcggtaacttcaacagaaatgagaagtgcatgga

attgctagatgggaaaaacggaaaatcctgctattttatcatggagttcataaaattagtgatcagcacacattacagactttcc

aagggatgtgcaaagcctgggatatagaagaacttgtcagcctggggaagaaactaaaggcctgtccatattacacagccc

gagaactaatacaagatgctgacatcatattttgtccctacaactatcttctagatgcacaaataagggaaagtatggatttaaa

tctgaaagaacaggttgtcattttagatgaagctcataacatcgaggactgtgctcgggaatcagcaagttacagtgtaacag

aagttcagcttcggtttgctcgggatgaactagatagtatggtcaacaataatataaggaagaaagatcatgaacccctacga

gctgtgtgctgtagcctcattaattggttagaagcaaacgctgaatatcttgtagaaagagattatgaatcagcttgtaaaatatg

gagtggaaatgaaatgctcttaactttacacaaaatgggtatcaccactgctacttttcccattttgcagggacattttctgctgttc

ttcaaaaagaggaaaaaatctcaccaatttatggtaaagaggaggcaagagaagtacctgttattagtgcatcaactcaaat

aatgcttaaaggactttttatggtacttgactatcttttaggcaaaatagcagatttgcagatgattataaaattgcgattcaacag

acttactcctggacaaatcagattgatatttcagacaaaaatgggttgttggttctaccaaaaaataagaaacgttcacgacag

aaaactgcagttcatgtgctaaacttttggtgcttaaatccagctgtggcctttcagatattaatggcaaagttcagaccattgtttt

gacatctggtacattatcaccaatgaaatcctttcgtcagaacttggtgttacatttactatccagctggaggctaatcatatcatt

aaaaattcacaggtttgggttggtaccattgggtcaggcccccaagggtcggaatctctgtgctaccttccagaatactgaaaca

tttgagttccaagatgaagtgggagcacttttgttatctgtgtgccagactgtgagccaaggaattttgtgtttcttgccatcttacaa

gttattagaaaaattaaaagaacgttggctctctactggtttatggcataatctggagttggtgaagacagtcattgtagaaccac

agggaggagaaaaaacaaattttgatgaattactgcaggtgtactatgacgcaatcaaatacaaaggagagaaagatgga

gctctcctggtagcagtttgtcgtggtaaagtgagtgagggtctggatttctcagatgacaatgcccgtgctgtcataacaatag

gaattcctttccaaatgtgaaagatctacaggttgaactaaaacgacaatacaatgaccaccattcaaaattgagaggtcttct

acctggccgtcagtggtatgaaattcaagcatacagggccttaaaccaggcccttggtagatgtattagacacagaaatgatt

ggggagctcttattctagtggatgatcgctttaggaataacccaagtcgctatatatctggactttctaaatgggtacggcagca

gattcagcaccattcaacctttgaaagtgcactggaatccttggctgaattttccaaaaagcatcaaaaagttcttaatgtatcca

taaaggacagaaccaatatacaggacaatgagtctacacttgaagtgacctctttaaagtacagtacctcaccttatttactgg

aagcagcaagtcatctatcaccagaaaattttgtggaagatgaagcaaagatatgtgtccaggaactacagtgtcctaaaatt

attaccaaaaattcacctctaccaagtagcattatctccagaaaggagaaaaatgatccagtattcctggaagaagcaggga

aagcagaaaaaattgtgatttccagatccacaagcccaactttcaacaaacaaacaaagagagttagctggtcaagctttaa

ttctttgggacagtatttttactggtaaaataccgaaggcaacacctgagctcgggtcatcagagaatagtgcctctagtcctccc

cgtttcaaaacagagaagatggaaagtaaaactgttttgcccttcactgataaatgtgaatcctcaaatctgacagtaaacac

atcgtttggatcatgccctcaatcagaaaccattatttcatcattaaagattgatgccacccttactagaaaaaatcattctgaac

atccgctctgttctgaagaagccctggatccagacattgaattgtctctagtaagtgaagaagataaacagtccacttcaaata

gagattttgaaacagaagcagaagatgaatctatctattttacacctgaactttatgatcctgaagatacagatgaagaaaaa

aatgacctagctgaaactgatagaggaaatagattggctaacaattcagattgcattttagctaaagacctttttgaaattagaa

ctataaaagaagtagattcagccagagaagtgaaagctgaggattgcatagatacaaagttgaatggaattctgcatattga

agaaagtaaaattgatgacattgatggtaatgtaaaaacaacttggataaatgaactggaactgggaaaaactcatgaaata

gaaataaagaactttaaaccatctccttccaaaaataaaggcatgtttcctggttttaagtaataatacttaactctcaagctaag

taaaaatatgtcatcatgcttatgttaaactctgttgtaagtaataatttgtaaattgaataagtggcatactttttaaaaaactatttta

tgttcagaaatgtaaatgttattattcttgagttttgggttttttttttttgagacagagtcttggtctgttgcccaggctggaatgcagtg

gtgtgctctgggctcactgcaaccttcacctccaggttcaagtgattctcctgcctcagccttctgagtagctgggactacaggtg

tgcaccaccatgcccagctagtttttgtattttttagtagagatgggggtttcaccatgttggccaggctggtctcgaactcctggcctc

gtgatctgcccttctctgcctccctaagttgctgggattacaggtgtgagccacagtgcctggcccattcttgagttttgataaagta

attcatacaaagtactgtcctcaaataagtcttccttagctaaatgcaatttaaaattattcaaagatcctagggcacttctagtttc

acgtaaatattcatattaggtggttctcttcatccatttgttttcacactgatacataaaaattaacagcagtctaatctagtgacacc

tcagtcatatttcgctatagattttacctcaaatcagtccaagactttttcagagatcaccatttgtcttgaaaggtttatttcgttattaa

actgcctacttataagtaattaagagaaattaagaaagtagtatgcatttttaattgaaattgttttacattctttgtataataaaccta

aaaccaaacatgtcataaacaaattgacgtaaagatataaaatgccaaatgaagtattccaaattttctattctaattatttagctt

caccatcattgtggaaaaaaatactagatcctgcttagtattatatattttcctagtggatcagtgagtaataagtaccaaacact

agactagaaggtaatttctacattgtttagaaagggtgaaacaatttatcccctctggtattgttctagcataagctttagttataca

atgattaagatagaaaacttcatatataaatttgataagcaaacccacatttatagctgcagctaaaatatgtttccttagggcac

agtaatcctttctgtgaattttgaccttgtttgtgttttttgtgaatgaagctatatgtctaatcaaaaatgattataaaagaggctcatct

ctgacatcattccaaaaatacattcattgatctcttttaagaaacatctgttattcactgggcattgggactttttgtgagtaatttga

attgaaatttatgagctatccaagaattctgtatggtctattattttcaagtcaaaatttccagtaaggatttactttacatttcatttgg

ataaatgaatcattatataggtatgtctttgcttccattttgagacatttagattttttacagcctgtttctatagcatttgatgttacaactc

taagcgtagttcaaagacatttaaattgacaagttaccagttaaagaatttagaatatattagatcccatctagtattatatatttttt

ctagttgatcattgagcagtaaataccaaatactcgattagaaggtaattttttacattgtttttgaaagggtgaaacaatttatctcct

ctggtattattcttaaaccacagatagggatagtagggtagtgaaacgaataaatacctggtagaagacaagagacttgggc

tctacacctggctctgccactgatttgctaagtcatattggcaatcaccacacccttcagggaattagtttcatctgtaaaatgcag

cggttagtactataaaatcatacaaatttctttgtgctttgagaatctataaaggaatgtctgttgatattctgagtcgattttcatttgct

tttgttccagaacggttaaaataaagcatattatttcatttaaaaagtaaagtggctcttactcatttacatttagggaacgatggtg

acgtggtttggctatgtccccagccagatctcatcttgaattgtagttcccataatcccgacgtgttgtgggaaggacccagtgg

gaggtaattgaatcgtgggggcggttaccctcatgcggttctcgtgatactgagttcttatgagacctgatgatttataaggagct

tttccctgcttcactctcattcttctctcctgctgccctgtgaaaagttgccttccgccatgattgtaagtttcttgaggcctcctcagtc

atgcagaactgtgagtcaattaaacctctttcctttataaattacccagtcttgggtatgactttattagcagcatgagaacagact

gacacagatggtatatgtgcataataacttggaaagctagatatttattttcgcaatgctacaattaaaacattttgaggacttttta

aaattacctttagggccaggcgcggtggctcacgcctgtaatcccagcactttgggaggccgaggcgggcggatcacgagg

tcaggagatcgagaccatcctggttaacacggtgaaaccccgtctctactaaaaatacaaaaaattagccaggcgtggtgg

cgggcgcctgtagtcccagctactcaggaggctgaggcaggagaatggcgtgaacccgggaggtggagcttgcagtgag

ccgagattgcgccactgcactccagcctgggcgacagagcgggactccgtctcaaaaaaaaaaaaaaaaaagaaattacc

tttagaattggtaaactacatgagaaaattaagcatattattcatatcttagtgttattacccaacttcatctccaatactttcccctttc

cctagctagatatgttttttgtttgtttgttttagacagtcttgctctgtcgctcaggctggagtgcaaaggcacgatcttggctcactgc

aacctctgcctcgcaggttaaagcgattctcatgtctcagcctcctgcgtagctgggattacaggtgtacaccactgcacccag

ctaatttttttttttttttttttttttaagtatcgacagggtttcaccatgttggccaggctggtctcaaactcctgacctcaagtgatctgccc

acctcggcctcccaaagtgttgggattacaggcgtgagccaccatgcctggcctagatattttttattctttccaaaatttaattctc

cctgaagttaaaaattttcattactgagaatgtacatagagatgtgtcacaaccctttagtaattccaaaaggtgtttcaaaaatttt

atacaatgataatcactgttgaaacaggtgtatattctcctcagatcatgactgaccaagatgatattccaagaagtaaactact

ggcctttatagagtaggatgtaggccattttcattactgataacatacttttaaaagaaatgtttacagatttaaataagttaaaac
atctaaatgcttttaaaagagcacctggcacattgcaagttattcattattaattagtagatgaatcatatatctggagtgcaccctt
gctctatatgaaagcttccctaactatagatatataggtgatagactgacaataaagatttgaggaaagaaaaattatttggctg
gcttattttttaagcttttgagatgtataaggtagagattgtttatcaaataatttatgtgacacattcaatgcatatgaaataattatct
aatactgaatttattcaaggactgaaagtatatagagcacacaggctgcaaaccaggaaaactaccagttagagacaggg
ctttattcttggaataaaggtacaatgtaaagagtagatgtttcatgacttgattaaattatttaaaactgtctagaattgtgttataaa
actattactatgtttctatgcacttagttattacatgggtttaaatttggcactgtttctaagtttctataaggctttgttgttaagatctttct
attcaaaacattaattttaacaaaaagcttttccccgttatttttctgcaactggacttttttgtattactttcctatattaaaacgcattta
aaata

SEQ ID NO: 2 (CUL4; NM_001008895):

ggttccggcccagccatggcggacgaggccccgcggaagggcagcttctcggcgctcgtgggccgcaccaacggcctca
ccaagcccgcggccctggccgccgcgcccgccaagccggggggcgcgggcggctccaagaagctggtcatcaagaac
ttccgagacagacctcggctgcccgacaactacacgcaggacacgtggcggaagctgcacgaggcggtgcgggccgtg
cagagcagcacctccatcaggtacaacctcgaggagctctaccaggctgtggaaaatctctgttctcacaaagtctccccaat
gctctacaagcaactgcgtcaggcctgtgaagaccacgtccaggcacagatccttccgtttagagaagactcactagatagt
gttttattttaaagaagattaacacgtgctggcaggaccactgcagacaaatgatcatgatcagaagcatcttcctgttcttgga
ccgcacctatgtgctgcagaactccacgctgccctccatctgggatatgggattagaactgtttagaacccatattattagtgata
aaatggttcagagtaaaaccattgatggaatcctactgctgatcgagcgcgagaggagcggcgaggccgtggaccggagc
ctgttgcggagcctcctgggcatgctgtctgacctgcaggtgtataaagattcatttgaactgaaatttttggaagagactaattg
cttatatgctgccgaaggccaaaggttaatgcaggaaagagaggttccagaatatcttaaccatgtaagtaaacgcttagag
gaagagggagacagagtaatcacttacttggaccacagcacacagaaaccactgattgcttgtgtggagaaacagctatta
ggagaacatttaacagcaattctgcagaaagggctcgaccacttactggatgagaacagagtgccggacctcgcacagat
gtaccagctgttcagccgggtgaggggcgggcagcaggcgctgctgcagcactggagcgagtacatcaagacttttggaa
cagcgatcgtaatcaatcctgagaaagacaaagacatggtccaagacctgttggacttcaaggacaaggtggaccacgtg
atcgaggtctgcttccagaagaatgagcggttcgtcaacctgatgaaggagtcctttgagacgttcatcaacaagagaccca
acaagcctgcagaactgatcgcaaagcatgtggattcaaagttaagagcaggcaacaaagaagccacagacgaggagc
tggagcggacgttggacaagatcatgatcctgttcaggtttatccacggtaaagatgtctttgaagcattttataaaaaagatttg
gcaaaaagactccttgttgggaaaagtgcctcagtcgatgctgaaaagtctatgttgtcaaagctcaagcatgagtgcggtgc
agccttcaccagcaagctggaaggcatgttcaaggacatggagctttcgaaggacatcatggttcatttcaagcagcatatgc
agaatcagagtgactcaggccctatagacctcacagtgaacatactcacaatgggctactggccaacatacacgcccatgg
aagtgcacttaacccagaaatgattaaacttcaggaagtatttaaggcattttatcttggaaagcacagtggtcgaaaacttc
agtggcaaactactttgggacatgctgtttttaaaagcggagtttaaagaagggaagaaggaattccaggtgtccctcttccag
acactggtgctcctcatgttcaacgagggagatggcttcagctttgaggagataaaaatggccacggggatagaggatagtg
aattgcgcagaacgctgcagtccctggcctgtggcaaagcacgtgtgctgattaaaagtcccaaaggaaaggaagtggaa
gatggagacaagttcatttttaatggagagttcaagcacaagttgtttagaataaagatcaatcaaattcagatgaaggaaact

gttgaggaacaggttagcaccactgagagagtgtttcaggatagacaatatcagattgatgctgctatcgtcagaataatgaa

gatgagaaagactcttggtcataatcttctagtttctgaattatataatcagctgaaatttccagtaaagcctggagatttgaaaaa

gagaattgaatctctgatagacagagactatatggagagagacaaagacaatccgaatcagtaccactacgtggcctgac

gcatctgcagacggttccccttcatgaaacactagaatgtaccctcagagcaggaagcacacctgtgccatttctgggactct

gattgatccagctgtggacattggaaggcgaaggaagggaggtggctcctgggtcatctttcacaaggctcaagacttcaac

ctgcagatgtatctttttccctccagttttcctctagttcttttaggcatttaaattgtttctgttactctgtgcaaaataactttgagattgg

acaagaagatgttactaaagagaagttcctttaaaaggtcttgttcttgtgtcaaaaagctgcaagtttggtttgttctcgtgtgtga

tcatgagtgcacaatgaagaagaccctagatgctgcattttttagctctgaagattccttaggtatccctgaagacagctcgctc

agatgatcagcatttagagtgaaaacaagggcccttcatgggtgaacattagaaagagccagggttcaaagctggcgaatg

gatgacgcaccctagccactggcccctctctgtttcatgtatttccaaaagttgtaaactttgatggctgattttttcgtaagtcaggtt

tctaagtgagctccctgaggtgccaaggccatggtgtccgccctgctgcgtctgttcgtcagctgagttccttgtgaatctctgtttt

agggtttggggctagtgtgtttgtgtttccattctaagattgagtctggcagtccctgtttttttgcattgggggtaactgctctttgattttttt

taattgcagtatttgtgtgattgcaataataaagtttggtttggttttttacagtcatgcgcagggacgatccttgttctctgctgtaaact

gtaaaaagtttatggagacttaaagtcttgatgttgtgaagcagaggttattttgtggaaagattaaaaggattttgttggtacctg

gttttgtgttgtgtatatacatgaggttgaacagtgaaaggaaagttcagtagtgatgttagaagggtaactatgacaaagat

acttttgagataacatttaaaagtactttatatttacataatagcatgtttcattttgattaaaagctaccaaaggaattttgatcatg

gcataagtgtttaaagcaatattttctggaatataccaagtttatataatttgattttgtgctaaattattaagagtctcttttttgaaacat

gcgggtttgaaatatgacaccttgtgggtttccatattaaaatcctcactctttaattgtcatttctatctttgaaaattttcatttatgagt

tccatgatatgtggtctaagaaagaccaaacagatttctattttttttttttcttataagttcgttgtgtctagagattgttaatattgtaattt

aatgtagacttactttgaataaaattagtttaattggccttaaaattacattaataaaactttgtgatatgcaaatgacacattcttca

taactctttagcagctttgactactagtgcactagacagaagtcagagcagggagcacctgtcccctctcaagtgattattctttc

ctggccacctctaccacaaaataccagtggggccaggtagtgacaagtgggtcttttttacttggacgattcgttgtgctgtgcct

ttgtttcatttgagcacagtggctcacgcctgtagtcccagctatgggagggcaaggtgggaggatcgtttgagcctgagttcca

gaccatcctgagcaccatagggagatcctgtttccattttagtttatttatttttaattgaggtggagtcttgctctgttgcccaagctg

gagtgcagtggtgcaatctcagctcactgcaccctccgcctcctgggttcaagcagttctcccacctcagtctcccaagtagct

gggattacaggcacccgccatcatgcccagctaatatttgtatttttgtagagacggggtttcactatgttggccagactggtcttg

aactcctaacctcaggtgatctgcccgccttggcctcccaaagtgctgggattacaggcgtgagccaccatgcccggcctgc

ctgtttctgttttataaaagaaaggaaatctatttatatctattctcttatttagcaatatttaatgttttattgttaaaggtcagtgtggta

aaacaaatagtatagaagaaatcattgaaaaagaaacagcacttagacagaaatgctgtaaaagtggaaaggagaaggt

gaaattacttgcaggttagatggttgcatacccaggtcatctaaggagcaaatgaaaatctgtgactgaagaaagaattcacc

aagttaaccagacacaaaattaacaaaaacatcagtagctttcctataaatataggctaatggagggtcattttttatttttattt

agagacagggtcttaactgtcacctggactggagtgcagtggcatgatcatgtttcacggcagcctctaccttctgggctcaag

cgatcctctcttctcagccacctgagtagctgggcctacaggtgtacatcaccacgcccagataatttttattgtattttttatagag

gcagtgtcttgctatgttgccaaggctgttcttgtactcctggcctcaagcgaacctcctacctcagcctcccaaatgctgggatt

acaggcatgagacactgcacctgaccagaagtcatttttcaatagcaaccccaaaaaagataaaatacctagtaacaaa

tagggaaacacctatgtgaaaggtattataacattactaaggaacacaggccctgaataagtgaaagttctgttgattctcaca

ttttgctatacagtatcttaagatgtaaaaccttcctttaaaacacaagcggattctaaagttaaaacagaaaagcaaatatgcc agactagtcaaattgttttaaattctgctgtttttagaacattgtaattgaaacagcatgatgctggccctttaaaaagattcctgga acaagaaagtccacaaataagccaaatacacaggaactcggcgtggcacaaaggggccgtgtgtgatcaggaactgga actttgcaacaactacccatgctcagaagtttgggccaccttgaaaagagaaaagttggattagactccttttttatactaaggga agttccaagtggtcaagtatttacgtgtaaaaaaatacatatatatgaaaccataatcataagatactttttttgtaaattgactatt attgtataaatttatagagtataaagtgatcttataaattatgcatacattgtggactagtcaagctagttacatccatcacctcaaa tacttaacattttttgtagtgagaacatctgaaatttactcttagcaattttgaaatgtacaatactcaatttttgactgtattcaccatgc tgtacaatagaactcagaaaaagaaaaatatagtcctcctttctaaga

SEQ ID NO: 3 (GPC1; NM_002081):

gccgagccgggactgcgctagcccgccgcgctctgggctgcccgagcgagcgttcggacctcgcaccccgcgcgccccg cgccgccgccgccgccggcttttgttgtctccgcctcctcggccgccgccgcctctggaccgcgagccgcgcgcgccgggaa ccttggctctgcccttcgcgggcgggaactgcgcaggacccggccaggatccgagagaggcgcgggcgggtggccggg ggcgccgccggccccgccatggagctccgggcccgaggctggtggctgctatgtgcggccgcagcgctggtcgcctgcgc ccgcggggacccggccagcaagagccggagctgcggcgaggtccgccagatctacggagccaagggcttcagcctga gcgacgtgccccaggcggagatctcgggtgagcacctgcggatctgtccccagggctacacctgctgcaccagcgagatg gaggagaacctggccaaccgcagccatgccgagctggagaccgcgctccgggacagcagccgcgtcctgcaggccatg cttgccacccagctgcgcagcttcgatgaccacttccagcacctgctgaacgactcggagcggacgctgcaggccaccttcc ccggcgccttcggagagctgtacacgcagaacgcgagggccttccgggacctgtactcagagctgcgcctgtactaccgcg gtgccaacctgcacctggaggagacgctggccgagttctgggcccgcctgctcgagcgcctcttcaagcagctgcacccccc agctgctgctgcctgatgactacctggactgcctgggcaagcaggccgaggcgctgcggcccttcgggggaggccccgaga gagctgcgcctgcgggccacccgtgccttcgtggctgctcgctcctttgtgcagggcctgggcgtggccagcgacgtggtccg gaaagtggctcaggtcccccctgggcccggagtgctcgagagctgtcatgaagctggtctactgtgctcactgcctgggagtcc ccggcgccaggccctgccctgactattgccgaaatgtgctcaagggctgccttgccaaccaggccgacctggacgccgagt ggaggaacctcctggactccatggtgctcatcaccgacaagttctggggtacatcgggtgtggagagtgtcatcggcagcgt gcacacgtggctggcggaggccatcaacgccctccaggacaacagggacacgctcacggccaaggtcatccagggctg cgggaaccccaaggtcaaccccagggccccgggcctgaggagaagcggcgccggggcaagctggccccgcgggag aggccaccttcaggcacgctggagaagctggtctccgaagccaaggcccagctccgcgacgtccaggacttctggatcag cctcccagggacactgtgcagtgagaagatggccctgagcactgccagtgatgaccgctgctggaacgggatggccagag gccggtacctccccgaggtcatgggtgacggcctggccaaccagatcaacaaccccgaggtggaggtggacatcaccaa gccggacatgaccatccggcagcagatcatgcagctgaagatcatgaccaaccggctgcgcagcgcctacaacggcaac gacgtggacttccaggacgccagtgacgacggcagcggctcgggcagcggtgatggctgtctggatgacctctgcagccg gaaggtcagcaggaagagctccagctcccggacgcccttgacccatgccctcccaggcctgtcagagcaggaaggacag aagacctcggctgccagctgcccccagcccccgaccttcctcctgcccctcctcctcttcctggcccttacagtagccaggccc cggtggcggtaactgccccaaggccccagggacagaggccaaggactgactttgccaaaaatacaacacagacgatattt aattcacctcagcctggagaggcctggggtgggacagggagggccggcggctctgagcaggggcaggcgcagaggtcc

cagccccaggcctggcctcgcctgcctttctgccttttaattttgtatgaggtcctcaggtcagctgggagccagtgtgcccaaaa

gccatgtatttcagggacctcaggggcacctccggctgcctagccctccccccagctccctgcaccgccgcagaagcagcc

cctcgaggcctacagaggaggcctcaaagcaacccgctggagcccacagcgagcctgtgccttcctccccgcctcctccc

actgggactcccagcagagcccaccagccagccctggcccaccccccagcctccagagaagccccgcacgggctgtctg

ggtgtccgccatccagggtctggcagagcctctgagatgatgcatgatgccctcccctcagcgcaggctgcagagcccggc

cccacctccctgcgcccttgaggggccccagcgtctgcaggtgacgcctgagacagcaccactgctgaggagtctgagg

actgtcctcccacagacctgcagtgaggggccctccatgcgcagatgaggggccactgacccacctgcgcttctgctggag

gaggggaagctgggcccaaaggcccagggaggcagcgtgggctctgccaatgtgggctgcccctcgcacacagggctc

acagggcaggccttgctggggtccagggctgttggaggaccccgagggctgaggagcagccaggacccgcctgctccca

tcctcacccagatcaggaaccagggcctccctgttcacggtgacacaggtcagggctcagagtgaccctcagctgtcacctg

ctcacagggatgctggtggctggtgagaccccgcactgcagacgggaatgcctaggtcccttcccgacccagccagctgca

gggcacgggggacctggatagttaagggcttttccaaacatgcatccatttactgacacttcctgtccttgttcatggagagctgtt

cgctcctcccagatggcttcggagggccgcagggcccaccttggaccctggtgacctcctgtcactcactgaggccatcagg

gccctgccccaggcctggacgggccctccttccctcctgtgccccagctgccaggcggccctggggaggggtggtgtggtgt

tgggaaggggtcctgcaggggaggaggacttggagggtctgggggcagctgtcctgaaccgactgaccctgaggaggc

cgcttagtgctgctttgctttcatcaccgtcccgcacagtggacggaggtccccggttgctggtcaggtccccatggcttgttctct

ggaacctgactttagatgtttgggatcaggagcccccaacacaggcaagtccaccccataataaccctgccagtgccagg

gtgggctggggactctggcacagtgatgccgggcgccaggacagcagcactcccgctgcacacagacggcctaggggtg

gcgctcagaccccaccctacgctcatctctggaaggggcagccctgagtggtcactggtcagggcagtggccaagcctgct

gtgtccttcctccacaaggtccccccaccgctcagtgtcagcgggtgacgtgtgttctttgagtccttgtatgaataaaaggctg

gaaaccta

SEQ ID NO: 4 (IRF7; NM_001572):

gagacgaaacttcccgtcccggcggctctggcacccagggtccggcctgcgccttcccgccaggcctggacactggttcaa

cacctgtgacttcatgtgtgcgcgccggccacacctgcagtcacacctgtagcccccctctgccaagagatccataccgaggc

agcgtcggtggctacaagccctcagtccacacctgtggacacctgtgacacctggccacacgacctgtggccgcggcctgg

cgtctgctgcgacaggagcccttacctcccctgttataacacctgaccgccacctaactgcccctgcagaaggagcaatggc

cttggctcctgagagggcagccccacgcgtgctgttcggagagtggctccttggagagatcagcagcggctgctatgaggg

gctgcagtggctggacgaggcccgcacctgtttccgcgtgccctggaagcacttcgcgcgcaaggacctgagcgaggccg

acgcgcgcatcttcaaggcctgggctgtggcccgcggcaggtggccgcctagcagcagggggaggtggcccgccccccga

ggctgagactgcggagcgcgccggctggaaaaccaacttccgctgcgcactgcgcagcacgcgtcgcttcgtgatgctgcg

ggataactcggggggacccggccgacccgcacaaggtgtacgcgctcagccgggagctgtgctggcgagaaggcccagg

cacggaccagactgaggcagaggcccccgcagctgtcccaccaccacagggtgggcccccagggccattcctggcaca

cacacatgctggactccaagccccaggccccctccctgccccagctggtgacaaggggggacctcctgctccaggcagtgc

aacagagctgcctggcagaccatctgctgacagcgtcatggggggcagatccagtcccaaccaaggctcctggagaggg

acaagaagggcttcccctgactggggcctgtgctggaggcccagggctccctgctggggagctgtacgggtgggcagtag

agacgacccccagccccgggccccagcccgcggcactaacgacaggcgaggccgcggccccagagtccccgcacca ggcagagccgtacctgtcaccctccccaagcgcctgcaccgcggtgcaagagcccagcccaggggcgctggacgtgacc atcatgtacaagggccgcacggtgctgcagaaggtggtgggacacccgagctgcacgttcctatacggccccccagaccc agctgtccgggccacagaccccagcaggtagcattccccagccctgccgagctcccggaccagaagcagctgcgctac acggaggaactgctgcggcacgtggcccctgggttgcacctggagcttcggggggccacagctgtgggcccggcgcatggg caagtgcaaggtgtactgggaggtgggcggaccccagcctccgccagcccctccaccccagcctgcctgctgcctcgga actgtgacacccccatcttcgacttcagagtcttcttccaagagctggtggaattccgggcacggcagcgccgtggctcccca cgctataccatctacctgggcttcgggcaggacctgtcagctggaggcccaaggagaagagcctggtcctggtgaagctg gaaccctggctgtgccgagtgcacctagagggcacgcagcgtgagggtgtgtcttccctggatagcagcagcctcagcctct gcctgtccagcgccaacagcctctatgacgacatcgagtgcttccttatggagctggagcagcccgcctagaacccagtcta atgagaactccagaaagctggagcagcccacctagagctggccgcggccgcccagtctaataaaaagaactccagaac a

SEQ ID NO: 5 (MCM3; NM_002388):

Gcgccggggtggagtcatcctgggaacctccacgcgactttggtggaggtagttctttggcagcgggcatggcgggtaccgt

ggtgctggacgatgtggagctgcgggaggctcagagagattacctggacttcctggacgacgaggaagaccagggaattt

atcagagcaaagttcgggagctgatcagtgacaaccaataccggctgattgtcaatgtgaatgacctgcgcaggaaaaacg

agaagagggctaaccggcttctgaacaatgcctttgaggagctggttgccttccagcgggccttaaaggattttgtggcctcca

ttgatgctacctatgccaagcagtatgaggagttctacgtaggactggaaggcagctttggctccaagcacgtctccccgcgg

actcttacctcctgcttcctcagctgtgtggtctgtgtggagggcattgtcactaaatgttctctagttcgtcccaaagtcgtccgca

gtgtccactactgtcctgctactaagaagaccatagagcgacgttattctgatctcaccaccctggtggcctttccctccagctct

gtctatcctaccaaggatgaggagaacaatccccttgagacagaatatggcctttctgtctacaaggatcaccagaccatcac

catccaggagatgccggagaaggccccagccggccagctcccccgctctgtggacgtcattctggatgatgacttggtggat

aaagcgaagcctggtgaccgggttcaggtggtgggaacctaccgttgccttcctggaaagaagggaggctacacctctggg

accttcaggactgtcctgattgcctgtaatgttaagcagatgagcaaggatgctcagccctctttctctgctgaggatatagcca

agatcaagaagttcagtaaaacccgatccaaggatatctttgaccagctggccaagtcattggccccaagtatccatgggca

tgactatgtcaagaaagcaatcctctgcttgctcttgggaggggtggaacgagacctagaaaatggcagccacatccgtggg

gacatcaatattcttctaataggagacccatccgttgccaagtctcagcttctgcggtatgtgctttgcactgcaccccgagctat

ccccaccactggccgggggctcctctggagtgggtctgacggctgctgtcaccacagaccaggaaacaggagagcgccgtc

tggaagcaggggccatggtcctggctgaccgaggcgtggtttgcattgatgaatttgacaaaatgtctgacatggatcgcaca

gccatccatgaagtgatggagcagggtcgagtgaccattgccaaggctggcatccatgctcggctgaatgcccgctgcagtg

ttttggcagctgccaaccctgtctacggcaggtatgaccagtataagactccaatggagaacattgggctacaggactcactg

ctgtcacgatttgacttgctcttcatcatgctggatcagatggatcctgagcaggatcgggagatctcagaccatgtccttcggat

gcaccgttacagagcacctggggagcaggatggcgatgctatgcccttgggtagtgctgtggatatcctggccacagatgat

cccaactttagccaggaagatcagcaggacacccagatttatgagaagcatgacaaccttctacatgggaccaagaagaa

aaaggagaagatggtgagtgcagcattcatgaagaagtacatccatgtggccaaaatcatcaagcctgtcctgacacagg

agtcggccacctacattgcagaagagtattcacgcctgcgcagccaggatagcatgagctcagacaccgccaggacatct
ccagttacagcccgaacactggaaactctgattcgactggccacagcccatgcgaaggcccgcatgagcaagactgtgga
cctgcaggatgcagaggaagctgtggagttggtccagtatgcttactttaagaaggttctggagaaggagaagaaacgtaa
gaagcgaagtgaggatgaatcagagacagaagatgaagaggagaaaagccaagaggaccaggagcagaagagga
agagaaggaagactcgccagccagatgccaaagatggggattcatacgaccccctatgacttcagtgacacagaggagga
aatgcctcaagtacacactccaaagacggcagactcacaggagaccaaggaatcccagaaagtggagttgagtgaatcc
aggttgaaggcattcaaggtggccctcttggatgtgttccgggaagctcatgcgcagtcaatcggcatgaatcgcctcacaga
atccatcaaccgggacagcgaagagcccttctcttcagttgagatccaggctgctctgagcaagatgcaggatgacaatcag
gtcatggtgtctgagggcatcatcttcctcatctgaggaggcctcgtctctgaacttgggttgtgccgagagagtttgttctgtgtttc
ccaccctctccctgacccaagtctttgcctctactcccttaacagtgttgaattcaactgaaggcgaggaatgttggtgatgaag
ctgagttcaggactcggtggacccctttgggaatgggtcatgaaagctgccatgggggtgaggaaagaggagacagtgggag
aggacaatgactattgcatcttcattgcaaaagcactggctcatccgccctacttcccatcccacacaaacccaattgtaaata
acatatgacttctgagtacttttgggggcacaactgttttctgtttgctgtttttttgttttgttttttttctccagagcactttggtctagacta
ggctttgggtggttccaattggtggagagaagctctgaggcacgtcatgcaggtcaagaaagctttctttgcagtagcaccagt
taaggtgaatatgtattgtatcacaaaacaaacccaatatccagatgaatatccgagatgttgaataaacttagccatttcgtac
acatgg

SEQ ID NO: 6 (MTUS1; NM_001001924):

aaaggggggcggcagcgccggcggagcggaggcgggtctcacgtgggccagcgcagagcctgcggaagggacggatg
cggatctcgtcgctgtcaccttgaaagtgaccgaggggcttgactgtggactccttacgccgcccacccgggcccggcggtc
ccagccttctcgcagggcccccttctcagcagaagcaagcggggccgagaaagcgggtggaatagggttgctgcaggtccc
aaagacccctcgtggcgcctcgctactttctgcagcttgtttgcacttttttcacgctctagaaaaatctcatcttaattaagggaac
aacaaatcatttaatcttcagagcatcttagactgaaaacctttcaactgtgctgaaaaacctagaagacagaccattttgccc
accctctcatttaaaaggaattgaagaagaaataaaatggcagaggtttaaggttactattcaggatgactgatgataattcag
atgataaaatagaagatgaattgcaaaccttctttaccagtgataaagatggaaatacacatgcatacaacccgaaatcacc
acctacacaaaactcttcagccagcagtgtgaactggaattctgccaacccagatgacatggtggttgattatgaaactgacc
ctgctgtagttactggtgaaaatatttctttaagccttcaggtgttgaagtatttggtcatgaaaagtcttctagtgatttcattagta
agcaggtgttagatatgcataaagattctatttgtcagtgtcctgcacttgtaggtactgagaagcccaaatatctgcaacacag
ttgtcattccctagaagcagttgagggccagagtgttgagccatctttgccttttgtgtggaagcctaatgacaatttgaactgtgc
aggctactgtgatgccttggagctaaaccaaacatttgacatgacagtggataaagttaactgcacctttatatcacatcatgcc
atcggaaagagtcagtccttccatactgctggaagcctgccaccaactggtaggagaagtggaagtacatcttctttatcctatt
ccacttggacatcttcccattctgataagacgcatgcaagagaaactactatgatagagaaagctttgaaaaccctcaagtc
acaccatcagaagcccaagacatgacttacacagcattttctgatgtggtgatgcaaagtgaggttttttgtttcagatattggaa
atcagtgtgcatgttcttcaggaaaggtcaccagtgagtacacagatggatcacaacaaagactagttggagaaaaggaga
cacaagcactaacaccagtttctgatggcatggaagtccccaatgattctgcattacaagagttcttttgtttatcccatgatgaat
ccaatagcgaaccacattcacagagctcatacaggcacaaggaaatgggccaaaatctgagagagacagtgtcctattgt

cttattgatgatgaatgccctttaatggtgccagcttttgataagagcgaagctcaagtgctgaacccagagcataaagtcactg

agactgaagacacacaaatggtctccaaaggaaaggatttgggaacccaaaatcatacctcagaattgattctaagtagcc

cgccaggacaaaaggtgggctcgtcatttggactgacttgggatgcaaatgatatggtcattagcacagacaaaacgatgtg

catgtcaacaccagtcctagaacccacaaaaagtaaccttttctgtttcaccgattgaagcgacggagaaatgtaagaaagtg

gagaagggtaatcgagggcttaaaaacataccagactcgaaggaggcacctgtgaacctgtgtaaacccagtttaggaaa

atcaacaatcaaaacgaataccccaataggctgcaaagttagaaaaactgaaattataagttacccaagaccaaacttcaa

gaatgtcaaagcaaaagttatgtctagagcagtgttgcagcccaaagatgctgctttatcaaaggtcacgcccagacctcag

cagaccagtgcctcatcaccctcatcagtgaattcaagacaacaaacagtcttgagcagaacaccgagatctgacttgaatg

cagacaaaaaagcagaaattctaattaacaagacacataagcagcagtttaataaaactcattactagccaggctgtgcatgt

tacaactcattctaaaaatgcttcacacagggttccaagaacaacatctgccgtgaaatcgaatcaggaagatgttgacaaa

gccagttcttctaactcagcatgcgagaccgggtccgtttctgcgttgtttcagaagatcaaaggcatactccctgttaaaatgg

aaagtgcagaatgtttggaaatgacctatgttcccaacattgataggattagccctgaaaagaagggtgaaaagaaaatg

ggacatctatggaaaaacaagagctgaaacaagagattatgaatgagactttgaatatggttctctgttttgggctctgcttca

aaaacaacgaccacctcaggtaggaatatatccaagcctgactcctgcggtttgaggcaaatagctgctccaaaagccaaa

gtggggccccctgtttcctgtttgaggcggaacagtgacaatagaaatcccagtgctgatcgagccgtatctcctcagaggatc

aggcgtgtgtccagttctggaaagcctacatccttgaaaactgcacagtcgtcatgggtgaatttgcctagaccacttcctaaat

ccaaagcatctttgaaaagtcctgcgctgcggaggacaggaagcacccctcaatagccagcacccacagtgagctgag

cacttacagcaacaattctggtaatgccgctgtcatcaaatatgaggagaaacctccaaaaccagcatttcagaatggttcctc

aggatcctttatttgaagcctttggtatccagggctcatgttcacttgatgaaaactcctccaaaaggtccttcgagaaaaattt

atttacagctcttaatgcagttgaaaagagcaggcaaaagaatcctcgaagcttatgtatccagccacagacagctcccgat

gcgctgcccctgagaaaacacttgaattgacgcaatataaaacaaaatgtgaaaaccaaagtggatttatcctgcagctca

agcagcttcttgcctgtggtaataccaagtttgaggcattgacagttgtgattcagcacctgctgtctgagcgggaggaagcact

gaaacaacacaaaaccctatctcaagaacttgttaacctccggggagagctagtcactgcttcaaccacctgtgagaaatta

gaaaagccaggaatgagttacaaacagtgtatgaagcattcgtccagcagcaccaggctgaaaaaacagaacgagag

aatcggcttaaagagttttacaccagggagtatgaaaagcttcgggacacttacattgaagaagcagagaagtacaaaatg

caattgcaagagcagtttgacaacttaaatgctgcgcatgaaacctctaagttggaaattgaagctagccactcagagaaact

tgaattgctaaagaaggcctatgaagcctcctttcagaaattaagaaaggccatgaaatagaaaagaaatcgcttgaagat

ttactttctgagaagcaggaatcgctagagaagcaaatcaatgatctgaagagtgaaaatgatgctttaaatgaaaaattgaa

atcagaagaacaaaaagaagagcaagagaaaaagcaaatttgaaaaatcctcagatcatgtatctagaacaggagtta

gaaagcctgaaagctgtgttagagatcaagaatgagaaactgcatcaacaggacatcaagttaatgaaaatggagaaact

ggtggacaacaacacagcattggttgacaaattgaagcgtttccagcaggagaatgaagaattgaaagctcggatggaca

agcacatggcaatctcaaggcagctttccacggagcaggctgttctgcaagagtcgctggagaaggagtcgaaagtcaac

aagcgactctctatggaaaacgaggagcttctgtggaaactgcacaatggggacctgtgtagccccaagagatcccccaca

tcctccgccatcccttttgcagtcaccaaggaattcgggctccttccctagccccagcatttcacccagatgacacctccccaaa

gtccacagactctctgaaagcattttgatgcaggtctgcaggactgaccccaaggaggaacgtgggcacaagaggtatatc

agcacacgtgtgatcaccgtagggtaactggagcgtcaccaccggcggaatcgcagcttctgagactggaactctggagg

aagacttttgcctccgtccaaaagattcctccaaaaaaagatttaaaaaaagatttcggcatcgacacggacgttgttgcaca

aagcacttaaagaacgagagcatcttgttcattgcctttttcacctaagcatagggggaaaaactctcagggccctattaagatt

tataacctttgtaatgttcttcaccacagacaccttcttgtgagttttcagtctgactgtgggggtgggggtgtgaatgaaatggat

gtcacagagtgtcatgtgtctgatgcagcctcctgctgtgtattaaatgtcaaaatctgaatatatctggatatgtactaatcaa

ataataatcaatcaatcagcatatacatttcagccaaagccatagaagaaaaagcaatagttgcttgaattatgatcatctacc

accaactctgctcagccctgtaacagggtagggagagggtataacaggaagagctttgacttgtccctgtctatacattctctgt

atcttttgggggtaacttcttggcagttttcagtgttcagccatgtcagttgaaactagattttctgtagattttttacttacccatgtga

gcctaacactatcctgtaattcattttctcaggctatgtgtaaatgtagaaccctaattttctataaaaaaacaaactaactaacta

actgtgtaaagaaagaaaaagggaagtaccaatgggttttccaccttatttttacctttgatctacccttgcagatttaacctgtctt

cttccctcccattattctcattttcctttacctttctccaccatccagagccacaaaagcaaaccttctacctcctacctacttttctct

gggacaaggataaaggaatatgattttccagagccccagagccagctcatcttccaggtgctgaaaccactttccaaataaa

ctaaagcctggatttgatattacaaattttgggaaatcttagaataaagaacgagaacaaggaagtcattggctagtataatta

agaaaggtaggattcagtgcttaccgatgatgcagtacttgatagaagaaaacagtctgggaggatagcgctcatttttcagtt

accctttaaggagtccctttgtctttgggaaagtagcagaatggtccgcttctttcccatgagtggaaaatgtggcttgtccaactc

tcctccaggttgcatttcagtttctttccaaaacttattacctcccctaatcctgagactttggaaaaggtggaaggaagaactgtt

gctttatctcccctccctgcatgtgtcaacattgtgatgtcagtatttactaatctacattcagtggctgtacaaataacagctgtag

taagaagagattcaggatgctagaggtgaatatttgggtcatttacatgtacactacatagcaagttgatactcatgttgcatgttc

ttttaaattagtgattttgtgtcttaagtctttaacttccaatacttcatcatgtatgtaaccttccatgtttgcttctgataaatggaaatgt

aggttcactgccacttcatgagatatctctgctcacgcttccaagttgttctcaatgacattagccaaagttgggtttgccattcatc

ccctaggcatggtaaatcttgtgttgttccctgctgtcctccgtattacgtgaccggcaaataaatctcatagcagttaatataaaa

catctttggaggatgggagagaacaggagggaagatgggaaacaaaatagagaattcttaagattttgtttaaaccaaatgtt

tcatgtagaatgcaaaatgttggcacgtcaaaaatatgaatgtgtagacaactgtagttgtgctcagtttgtagtgatgggaagt

gtattttactctgatcaaataaataatgctggaatactcaa

SEQ ID NO: 7 (PCNA; NM_002592):

ggatggccggagctggcgccctggttctggaggtaaccggttactgagggcgagaagcgccacccggaggctctagcctg

acaaatgcttgctgacctgggccagagctcttcccttacgcaagtctcagccggtcgtcgcgacgttcgcccgctcgctctgag

gctcctgaagccgaaaccagctagactttcctccttcccgcctgcctgtagcggcgttgttgccactccgccaccatgttcgagg

cgcgcctggtccagggctccatcctcaagaaggtgttggaggcactcaaggacctcatcaacgaggcctgctgggatattag

ctccagcggtgtaaacctgcagagcatggactcgtcccacgtctctttggtgcagctcacctgcggtctgagggcttcgacac

ctaccgctgcgaccgcaacctggccatgggcgtgaacctcaccagtatgtccaaaatactaaaatgcgccggcaatgaag

atatcattacactaagggccgaagataacgcggataccttggcgctagtatttgaagcaccaaaccaggagaaagtttcaga

ctatgaaatgaagttgatggatttagatgttgaacaacttggaattccagaacaggagtacagctgtgtagtaaagatgccttct

ggtgaatttgcacgtatatgccgagatctcagccatattggagatgctgttgtaatttcctgtgcaaaagacggagtgaaattttct

gcaagtggagaacttggaaatggaaacattaaattgtcacagacaagtaatgtcgataaagaggaggaagctgttaccata

gagatgaatgaaccagttcaactaactttttgcactgaggtacctgaacttctttacaaaagccactccactctcttcaacggtga

cactcagtatgtctgcagatgtaccccttgttgtagagtataaaattgcggatatgggacacttaaaatactacttggctcccaag

atcgaggatgaagaaggatcttaggcattcttaaaattcaagaaaataaaactaagctctttgagaactgcttctaagatgcca

gcatatactgaagtcttttctgtcaccaaatttgtacctctaagtacatatgtagatattgttttctgtaaataacctattttttttctctattct

ctgcaatttgtttaaagaataaagtccaaagtcagatctggtctagttaacctagaagtattttttgtctcttagaaatacttgtgatttttt

ataatacaaaagggtcttgactctaaatgcagttttaagaattgttttttgaatttaaataaagttacttgaatttcaaacatca

SEQ ID NO: 8 (SPAG5; NM_006461):

aggttcaaacacagacggcgggtgaacatggcgtcctcgacttggtctgagacgtgataggcctgccttctggttgaagatgt

ggcgagtgaaaaaactgagcctcagcctgtcgccttcgccccagacgggaaaaccatctatgagaactcctctccgtgaac

ttaccctgcagcccggtgccctcaccaactctggaaaaagatcccccgcttgctcctcgctgaccccatcactgtgcaagctg

gggctgcaggaaggcagcaacaactcatctccagtggattttgtaaataacaagaggacagacttatcttcagaacatttcag

tcattcctcaaagtggctagaaacttgtcagcatgaatcagatgagcagcctctagatccaattccccaaattagctctactcct

aaaacgtctgaggaagcagtagacccactgggcaattatatggttaaaaccatcgtccttgtaccatctccactggggcagc

aacaagacatgatatttgaggcccgtttagataccatggcagagacaaacagcatatctttaaatggacctttgagaacaga

cgatctggtgagagaggaggtggcaccctgcatgggagacaggttttcagaagttgctgctgtatctgagaaacctatctttca

ggaatctccgtcccatctcttagaggagtctccaccaaatccctgttctgaacaactacattgctccaaggaaagcctgagca

gtagaactgaggctgtgcgtgaggacttagtaccttctgaaagtaacgccttcttgccttcctctgttctctggctttccccttcaact

gccttggcagcagatttccgtgtcaatcatgtggacccagaggaggaaattgtagagcatggagctatggaggaaagagaa

atgaggtttcccacacatcctaaggagtctgaaacagaagatcaagcacttgtctcaagtgtggaagatattctgtccacatgc

ctgacaccaaatcagtagaaatggaatcccaagaagctccaggcccagcagtagaagatgttggtaggattcttggctctg

atacagagtcttggatgtccccactggcctggctggaaaaaggtgtaaatacctccgtcatgctggaaaatctccgccaaagc

ttatcccttccctcgatgcttcgggatgctgcaattggcactacccctttctctacttgctcggtggggacttggtttactccttcagca

ccacaggaaaagagtacaaacacatcccagacaggcctggttggcaccaagcacagtacttctgagacagagcagctcc

tgtgtggccggcctccagatctgactgccttgtctcgacatgacttggaagataacctgctgagctctcttgtcattctggaggttct

ctcccgccagcttcgggactggaagagccagctggctgtccctcacccagaaacccaggacagtagcacacagactgac

acatctcacagtgggataactaataaacttcagcatcttaaggagagccatgagatgggacaggccctacagcaggccag

aaatgtcatgcaatcatgggtgcttatctctaaagagctgatatccttgcttcacctatccctgttgcatttagaagaagataagac

tactgtgagtcaggagtctcggcgtgcagaaacattggtctgttgctgttttgatttgctgaagaaattgagggcaaagctccag

agcctcaaagcagaaagggaggaggcaaggcacagagaggaaatggctctcagaggcaaggatgcggcagagatag

tgttggaggctttctgtgcacacgccagccagcgcatcagccagctggaacaggacctagcatccatgcgggaattcagag

gccttctgaaggatgcccagacccaactggtagggcttcatgccaagcaagaagagctggttcagcagacagtgagtctta

cttctaccttgcaacaagactggaggtccatgcaactggattatacaacatggacagctttgctgagtcggtcccgacaactca

cagagaaactcacagtcaagagccagcaagccctgcaggaacgtgatgtggcaattgaggaaaagcaggaggtttctag

ggtgctggaacaagtctctgcccagttagaggagtgcaaaggccaaacagaacaactggagttggaaaacagtcgtctag

caacagatctccgggctcagttgcagattctggccaacatggacagccagctaaaagagctacagagtcagcatacccatt

gtgcccaggacctggctatgaaggatgagttactctgccagcttacccagagcaatgaggagcaggctgctcaatggcaaa

aggaagagatggcactaaaacacatgcaggcagaactgcagcagcaacaagctgtcctggccaaagaggtgcgggac

ctgaaagagaccttggagtttgcagaccaggagaatcaggttgctcacctggagctgggtcaggttgagtgtcaattgaaaa

ccacactggaagtgctccgggagcgcagcttgcagtgtgagaacctcaaggacactgtagagaacctaacggctaaactg

gccagcaccatagcagataaccaggagcaagatctggagaaaacacggcagtactctcaaaagctagggctgctgactg

agcaactacagagcctgactctctttctacagacaaaactaaaggagaagactgaacaagagaccttctgctgagtacag

cctgtcctcccacccaggaacaccctctgcctaatgacaggaccttcctgggaagcatcttgacagcagtggcagatgaaga

gccagaatcaactcctgtgcccttgcttggaagtgacaagagtgctttcacccgagtagcatcaatggtttcccttcagcccgc

agagaccccaggcatggaggagagcctggcagaaatgagtattatgactactgagcttcagagtctttgttccctgctacaag

agtctaaagaagaagccatcaggactctgcagcgaaaaatttgtgagctgcaagctaggctgcaggcccaggaagaaca

gcatcaggaagtccagaaggcaaaagaagcagacatagagaagctgaaccaggccttgtgcttgcgctacaagaatga

aaaggagctccaggaagtgatacagcagcagaatgagaagatcctagaacagatagacaagagtggcgagctcataag

ccttagagaggaggtgacccaccttacccgctcacttcggcgtgcggagacagagaccaaagtgctccaggaggccctgg

caggccagctggactccaactgccagcctatggccaccaattggatccaggagaaagtgtggctctctcaggaggtggaca

aactgagagtgatgttcctggagatgaaaaatgagaaggaaaaactcatgatcaagttccagagccatagaaatatcctag

aggagaaccttcggcgctctgacaaggagttagaaaaactagatgacattgttcagcatatttataagaccctgctctctattcc

agaggtggtgaggggatgcaaagaactacagggattgctggaatttctgagctaagaaactgaaagccagaatctgcttca

cctctttttacctgcaatacccccttaccccaataccaagaccaactggcatagagccaactgagataaatgctatttaaataa

agtgtatttaatgaa

SEQ ID NO: 9 (TTC21B; NM_024753):

atcatgtattactggttgccagtgaaggaattaagaggtatggaagtgatccagtcttcaggttttatcatgcctatggcacattaa

tggaaggtaaaactcaagaagctcttcgagaatttgaggctattaaaaataaacaagatgtatcactttgttctctacttgcactg

atatatgcccataaaatgagtcctaatccagatagagaagctattctggaatcagatgccagagtgaaggaacaacgtaaa

ggagctggagagaaagccttataccatgcaggcttattttttatggcacattggtcgccatgataaagcaagggaatatattgac

agaatgatcaaaatatcagatggtagtaaacagggacacgttttgaaagcatggcttgatattacaagaggaaaagagcctt

acactaaaaaagcactgaagtattttgaagagggactccaagatgggaatgatacttttgctctgctgggtaaggcacaatgc

cttgagatgcgccagaattattcaggtgccctggagactgtgaaccagataatcgtgaattttccgagcttccttcctgcttttgtta

agaaaatgaaattacaactagccttgcaggattgggaccagacagttgagacagcacaaaggttgctgctccaagatagcc

aaaatgtggaagcactgagaatgcaggcactctactatgtgtgtagagagggggatatagagaaggcttccaccaagctgg

aaaacttgggaaatacattggatgccatggaaccacagaatgctcaacttttctataacattacactcgccttcagcagaactt

gtggacgtagtcaacttattcttcaaaaaattcaaacgttacttgagagagctttttagtttaaaccctcagcaatcagaatttgcta

cagaacttggataccaaatgattttacaaggaagagttaaagaggcactgaagtggtataagaccgccatgacacttgatga

gactagtgtgtctgccctagttggatttatccaatgtcagttgatagaagggcaattacaggatgcagatcagcagctagaatttt

taaatgaaatccagcaatccattggaaaatctgcggaattaatctatttacatgcagttcttgccatgaagaaaaataaacgac

aagaagaagttattaatttgttaaatgatgtcctggacactcactttttcacaattagaaggtttgcctcttggcatacagtattttgaa

aagctaaatcctgatttcttgttagaaattgttatggagtatctgagcttctgtccaatgcagcctgcaagtcctgggcaacctcttt

gtccacttctcaggcgttgcatctcagtcctggagactgtagtaagaactgttccaggtcttctgcaaacagtcttcctaatagca

aaagtgaaatatttgtcaggtgatattgaagcagctttcaataaccttcagcactgcttagaacacaatccctcttatgctgatgct

catctgctgctagctcaggtttacttgtctcaagaaaaagtcaaattgtgttctcagtctcttgaactttgtctgagctatgattttaag

gtgagagactatcctttataccatttgataaaagctcagtcacaaaagaaaatgggagaaatagcagacgcaattaaaaca

ctgcatatggcaatgagtttaccaggaatgaaaagaattggagcttccacaaaatcaaaagacagaaaaactgaagttgat

acaagccatcgtttatcgatctttcttgaattgatagacgttcaccgcttaaatggagagcagcatgaggcaaccaaagttttac

aagatgccatccatgaattttctggaacatctgaagaagtgcgggttaccattgctaatgcagaccttgctctagcccaaggag

atattgaacgggctttaagcatccttcagaatgttacagccgaacagccttattttatagaggccagagaaaaaatggcagat

atttatctgaagcacagaaaagataaaatgttatatatcacttgttttagagaaattgctgaaagaatggctaaccctcggtctttt

cttctccttggtgatgcatacatgaatattctagagcctgaagaagccatagtagcatatgagcaagcattaaatcagaacccg

aaagatggaacattggcaagcaaaatgggcaaagcacttatcaaaactcataactactcaatggcaatcacttactatgaa

gctgctctgaaaactggacaaaagaattatctttgctatgacctggctgagctcttattaaaattgaaatggtatgacaaagcag

aaaaagttcttcagcatgctctggctcatgaacctgtaaatgaactgtcagctctcatggaggatggacgttgtcaagttcttcta

gcaaaagtttatagtaaaatggaaaaacttggtgatgcgatcactgcattacaacaggctcgagaattacaagctcgggtact

aaaacgtgttcagatggaacagccagatgcagttcctgcacagaaacatttagcagctgaaatttgtgcagagattgcaaaa

cattctgttgctcagcgagactatgaaaaagcaattaagttttatagagaggctctggttcactgcgaaacagataataagatta

tgttggaactggcacgattatacctggcacaagatgaccctgattcctgcctgcggcagtgtgctctactgcttcagagtgacca

ggataacgaagctgctaccatgatgatggctgatctcatgttcagaaaacaagactatgaacaagcagtgtttcatttacagc

agctttcagaacgtaagccagataattatatgacattatctcgtttgattgatctcctaagaagatgtggaaaactcgaggatgtc

ccaagattttctcaatggctgagaaacgtaactccagagcaaaattggaaccaggatttcagtattgtaaaggactgtatcttt

ggtacactggagaaccaaatgatgcccttcgacattttaataaagctcggaaagatcgtgactggggccaaaatgccctttat

aatatgatagagatctgtttgaatccagataatgaaactgttggaggtgaagtatttgaaaacctggatggagacctgggtaatt

caactgagaagcaagaatctgtgcaactggcagtaagaacagcagaaaaacttcttaaggaactaaaaacctcagactgttc

agggtcacgtacagcttcgcataatggaaaactattgcttaatggctaccaaacagaaatctaatgttgaacaagcattaaat

accttcactgaaatagcagcatctgagaaggagcatatcccagcgctcttgggaatggcaacggcttatatgatcttgaaaca

gactccacgagccagaaaccagctgaagcgtattgcgaaaatgaattggaatgctattgatgctgaagagtttgagaagagt

tggctgctacttgctgatatttacattcaatcagcaaaatatgacatggcagaagacctgttaaaacggtgcctgcgtcataata

gatcttgctgcaaagcttatgaatatatgggatacattatggaaaaagagcaagcatatacagatgctgccttgaactatgaga

tggcatggaaatatagcaatcggacaaatccggcagtaggatacaaactggcatttaattacttaaaagcaaaaagatatgt

ggattcaattgacatatgtcaccaggttcttgaagcacatccaacttatccaaaaatcagaaaggatatacttgataaggcccg

tgcgtctttaagaccttgaaaataattttaacttaggtgttggtttaacaggaaatgaaagaaatctaactttcagttcttcctgttca

aaacaggtttgagctcagtgctttgttattagaagtataccctttttttctccagcagaggttgctgctgtacatcaagagaagtact

atgtgaaattggtgtttcctaatggagttgaatgagagctggtcatttgactctgttttgattgggtacagatttggtgactctgtggt

aaagactataattatttctataaagaatattttgttaaaatctaggtaattaaataccctgtatcttttctaaggaatattatttcagga

aatatatttaaaatgcattgttctcttttaaagtgttttttgttatattcttttatttatttattttttaaagacagagccttgatctgccgccc

aggctggagtgcagtggcacgatcttggctcactgcaacctccgcctcctgggtttacgcgattctcctgcttcagcctcccgag

tagctgggactaaaggcatgtgccatcacacccggctaattttttgtatttttagtagagatgggatttcaccgtgttagccagga

ggggcttgatctcttgaccttgtgatccacctgcctcggcctcccaaagtgctgggattacaggcatgagccaccgcacccag

cctgttttttgttgtattctgtgttgtttcaaaaattggaataaatctccctgtaaataactcatgaaaaagacaatatatatattttaaat

atatattttaataaaggttttataaatagatgatttatattcctcttggaactcaaaagatgtggtaagttttgcaaatcagggtaagc

tccccctgcattctgtaatgttaatgggtggtggtatagtacagaaaatgtaactttcattgaaaaatcttttttagaaaagggtag

agtgtgtgtttcccttttcctttcttgagctcattcttcctgctttcaaggccattgtggtcttaagaaataatcaggtcctgaaacctcaa

tttaaataggtcccttattagggatatttaaccttagcctagaagacttggtctgcaggtataatttcagacactgtaacatttgatg

gtaaagggtgaccaaatacttttggtcagcagggagtcacaaagcacaccatgtgaaggcaacggtttacccagctctgttta

acagcaccctggcatagaagctttcactctcttacactaaatttaacctaatctaaacttaatccttagcattattttttaatttctcatct

acatcttttcaattttccttaaaattgtagtaaacacagtcctcaatttttatttgcatttacaataaacagtatttataagtca

SEQ ID NO: 10 (ULK1; NM_003565):

agtgcggctgcgcgggcgtctcaggctctgaggcccgggcgccgcggctctttttgtttctccgttgggggccgagcccgggccc

tagttggagccggagtcggagtcggagtcggatccggattcggattagcagcccgggaagagtgccgtggcacaggcgcc

ggagggagcgcgaccctcggaccccgcctggcccgcggggctgggacccggccccggcctgcccgatggggcgcgcg

gccccggagatgcgccctcgcccggccccgcgcccccggccccgcgcccccggcccgcccgccccggcccgcgcctcc

gcctgagtcccccgcgccttggcccgccaccccccgccccgcgcccccggcccgcctgcgccatggagcccggccgcgg

cggcacagagaccgtgggcaagttcgagttctcccgcaaggacctgatcggccacggcgccttcgcggtggtcttcaaggg

ccgccaccgcgagaagcacgatttggaggtcgccgtcaagtgcattaacaagaagaacctcgccaagtctcagacgctgc

tggggaaggaaatcaaaatcctgaaggaactgaaacatgaaaacatcgtggccctgtacgacttccaggaaatggctaatt

ctgtctacctggttatggagtactgcaacggtggggacctggccgactacctgcacgccatgcgcacgctgagcgaggaca

ccatcaggctcttcctgcagcagatcgcgggcgccatgcggcttctgcacagcaaaggcatcatccaccgcgacctgaaac

cgcagaacatcctgctgtccaaccccgccggccgccgcgccaaccccaacagcatccgcgtcaagatcgctgacttcggc

ttcgcgcggtacctccagagcaacatgatggcggccacactctgcggctcccccatgtacatggcccccgaggtcatcatgt

cccagcactacgacgggaaggcggacctgtggagcatcggcaccatcgtctaccagtgcctgacggggaaggcgcccttc

caggccagcagcccccaggacctgcgcctgttctacgagaagaacaagacgttggtccccaccatcccccgggagacctc

ggccccgctgcggcagctgctcctggcccctactgcaacgcaaccacaaggaccgcatggacttcgatgagttttttcatcacc

ctttcctcgatgccagcccctcggtcaggaaatccccacccgtgcctgtgccctcgtacccaagctcggggtccggcagcag

ctccagcagcagctccacctcccacctggcctcccccgccgtccctgggcgagatgcagcagctgcagaagaccctggcctc

cccggctgacaccgctggcttcctgcacagctcccgggactctggtggcagcaaggactcttcctgtgacacagacgacttc

gtcatggtccccgcgcagtttccaggtgacctggtggctgaggcgcccagtgccaaaccccccgccagacagcctgatgtgc

agtgggagctcactggtggcctctgcgggcttggagagccacggccggaccccatctccatccccaccctgcagcagctcc

cccagtccctcaggccgggctggcccgttctccagcagcaggtgcggcgcctctgtccccatcccagtccccacgcaggtgc

agaactaccagcgcattgagcgaaacctgcagtcacccacccagttccaaacacctcggtcctctgccatccgcaggtcag

gcagcaccagccccctgggctttgcaagggccagcccctcgcccctgcccacgctgagcatggaggcgtcctggccagg

aagatgtctctgggtggaggccggccctacacgccatctcctcaagttggaaccatccctgagcggccaggctggagcggg

acgccctccccacagggagctgagatgcggggtggcaggtcccctcgtccaggctcctctgcacccgagcactctccccgc

acttccgggctgggctgccgcctgcacagcgcccccaacctgtctgacttgcacgtcgtccgccccaagctgcccaaacccc

ccacggacccctgggagctgtgttcagcccaccacaggcagccctccccagccgtcccacggcctgcagtcctgccgg

aacctgcggggctcacccaagctgcccgacttcctgcagcgaaacccctgcccccatcctgggctcccccaccaaggct

gtgccctcctttgacttcccgaagacccccagctcccagaacctgctggccctcctagcccggcagggcgtggtgatgacgc

cccctcgaaaccggacgctgcccgacctctcggaggtggggacccttccatggtcagccgttgggccctggcctgcggccag

gcgaggaccccaagggcccctttggccggtctttcagcaccagccgcctcactgacctgctccttaaggcggcgtttgggac

acaagccccggacccgggcagcacggagagcctgcaggagaagcccatggagatcgcaccctcagctggctttggagg

gagcctgcacccaggagcccgtgctgggggcaccagcagcccttccccggtggtcttcaccgtgggctctcccccgagcgg

gagcacgcccccccagggcccccgcaccaggatgttctcagcgggccccactggctctgccagctcttctgcccgccacct

ggtgcctgggccctgcagcgaggccccagcccctgagctccctgctccaggacacggctgcagctttgccgaccccattact

gcgaacctggaggggggctgtgaccttcgaggccccgacctccctgaggagaccctcatggagcaagagcacacggag

atcctgcgtggcctgcgcttcacgctgctgttcgtgcagcacgtcctggagatcgcagccctgaagggcagcgccagtgagg

cggcgggggggccctgagtaccagctgcaggagagtgtggtggccgaccagatcagcctgctgagccgagaatggggctt

cgcggaacagctggtgctgtacctgaaggtggccgagctactgtcctccggcctgcaaagtgccatcgaccagatccgggc

cggcaagctctgcctgtcgtccactgtgaagcaggtggtgcgcaggctgaatgagctgtacaaggccagcgtggtgtcctgc

cagggcctgagcctgcggctgcagcgcttcttcctggacaagcagcggctcctggaccgcattcacagcatcactgccgag

aggctcatcttcagccacgctgtgcagatggtgcagtcggctgccctggacgagatgttccagcaccgtgagggctgcgtcc

cacgctaccacaaggccctgctgctcctggagggggctgcagcacatgctctcggaccaggccgacatcgagaacgtcacc

aagtgcaagctgtgcattgagcggagactctcggcgctgctgactggcatctgtgcctgacctttctggcctggctgggcccccc

cgtcctgccgagccctgcagagtgggctctgtgtgctggctggactcctcgggacaagcccatggcgctgatcgctggtgctg

agccctgccctgggccccacggacagtcagcctgccggcctccctgcagctcacggggcagaaccagcacatctggagc

cacacagcttggggggtgtctcccatcttttacaggtgggggatcacagaatttctgcccctccagctgcctggctcagcaggcg

tgggtgccaccaccctctagccccagggcagccccggaggacaggcaagggcctgagaccactgccgactcaaagcca

aagcgagctcctgcttagggaaggtcagcaggcactgtgcccaggaagagcctgcggcctcggcgtcccccagtctccag

gagcctctccctccgagatacccacccagctttgtcaatcacccaagcactttatgcatatagagacagaacctggacctcac

cagggactgctgggcagcgattcctggcagtggcctggtgtttgtacatacacatatgcagacacatgccagggccccccaa

gcccgagcaccggaccacgttgctgcccaggtctggacctcagcgggagaactggctccggggggagtggggccctgcg

ctagaggcagaggcagttctttgttcaagcgttcctctggggaccggcagcagaggcaccgtgttctctcagccctggatacgt

cttgtaatctttcacactttattcctaaaacgtgtcttattttatgcagctcattttttctttaaaggagaaaacttgtaggtgtttaagaa

ttggttttgggagggcgaggactgggccaggttagaggcagatggcacaggggcgtgtggcgggcgggtgaggctgctttg

cacacctgtgttggtggctgtcccctgccgcccctccctgtggcagcagcaggacaggtgtgtgcccagcaccctccctacct

gggcctggaagcagatgaggggaatacttcatgcaaagaaaaaagtaacatgtgcaaaagctccccgtccagctttgaca

gtcagttttgatgtcagctcctcggcagggtaggcctgatgacagccctgtccctccctgcctctgccttgcccaaggccacgg

agggcatctgcagagaggcctgccttccggattccaggcgggcatgccctgcaaaccccgcctgggcctcccttggtctgcc

cagccctcgattagccctgcctgaatcagtagatacttgaacgagtccccagtctgcgggaggcagtggtggggccatggac

44

ccatgcggggggttccagggtcacacgccacataacagacaaaaatacacacacgtgtgtttttctttgcaatacttgaaatat

tgccactgtgcttggacttagaagaagaaaatccccgtgacttcttcctcatcaccttgatggctttattctcaccttgtggggcatg

tttgtatttattgcttcatggccgactggaatcctgagtcctgggaagctggcactgcggggatcttgcccggtgtcctggtcctctt

gcttccgtcgcggccgcatgtgcgtgtgtccaagcaggtcctgggcgcctcaactgctgcccctggttgaatgttctcttgatagt

gctggacccctttgtctattttaaagcgaattttgtgtgatttcctgccctttgcgttatattgtataataccaacgtaaggaaataaacc

tttggaattgtt

SEQ ID NO: 11 (FOXM1; NM_202002):

tttcaaacagcggaacaaactgaaagctccggtgccagacccaccccccggccccggcccgggaccccctcccctcccg

ggatccccgggggttcccaccccgcccgcaccgccggggacccggccggtccggcgcgagccccgtccggggccctg

gctcggccccaggttggaggagcccggagcccgccttcggagctacggcctaacggcggcggcgactgcagtctggag

ggtccacacttgtgattctcaatggagagtgaaaacgcagattcataatgaaaactagcccccgtcggccactgattctcaaa

agacggaggctgccccttcctgttcaaaatgccccaagtgaaacatcagaggaggaacctaagagatccctgcccaaca

ggagtctaatcaagcagaggcctccaaggaagtggcagagtccaactcttgcaagtttccagctgggatcaagattattaac

cacccaccatgcccaacacgcaagtagtggccatccccaacaatgctaatattcacagcatcatcacagcactgactgcc

aagggaaaagagagtggcagtagtgggcccaacaaattcatcctcatcagctgtgggggagccccaactcagcctccag

gactccggcctcaaacccaaaccagctatgatgccaaaaggacagaagtgaccctggagaccttgggaccaaaacctgc

agctagggatgtgaatcttcctagaccacctggagccctttgcgagcagaaacgggagacctgtgcagatggtgaggcagc

aggctgcactatcaacaatagcctatccaacatccagtggcttcgaaagatgagttctgatggactgggctcccgcagcatca

agcaagagatggaggaaaaggagaattgtcacctggagcagcgacaggttaaggttgaggagccttcgagaccatcagc

gtcctggcagaactctgtgtctgagcggccaccctactcttacatggccatgatacaattcgccatcaacagcactgagagga

agcgcatgactttgaaagacatctatacgtggattgaggaccactttccctactttaagcacattgccaagccaggctggaag

aactccatccgccacaacctttccctgcacgacatgtttgtccgggagacgtctgccaatggcaaggtctccttctggaccattc

accccagtgccaaccgctacttgacattggaccaggtgtttaagccactggacccagggtctccacaattgcccgagcacttg

gaatcacagcagaaacgaccgaatccagagctccgccggaacatgaccatcaaaaccgaactcccccctgggcgcacgg

cggaagatgaagccactgctaccacgggtcagctcatacctggtacctatccagttcccggtgaaccagtcactggtgttgca

gccctcggtgaaggtgccattgcccctggcggcttccctcatgagctcagagcttgcccgccatagcaagcgagtccgcattg

cccccaaggttttggggaacaggtggtgtttggttacatgagtaagttctttagtggcgatctgcgagattttggtacacccatca

ccagcttgtttaattttatctttctttgtttatcagtgctgctagctgaggagggggatagctcctctttcttctgcaggaccagggaaag

aggagaaactcctgtttggagaagggttttctcctttgcttccagttcagactatcaaggaggaagaaatccagcctggggag

gaaatgccacacttagcgagacccatcaaagtggagagccctcccttggaagagtggccctcccccggccccatctttcaaa

gaggaatcatctcactcctgggaggattcgtcccaatctcccaccccaagacccaagaagtcctacagtgggcttaggtccc

caacccggtgtgtctcggaaatgcttgtgattcaacacagggagaggagggagaggagccggtctcggaggaaacagca

tctactgcctccctgtgtggatgagccggagctgctcttctcagagggggcccagtacttcccgctgggccgcagagctcccgtt

cccagcagactcctctgaccctgcctcccagctcagctactcccaggaagtggggggaccttttaagacacccattaaggaa

acgctgcccatctcctccaccccgagcaaatctgtcctccccagaacccctgaatcctggaggctcacgcccccagccaaa

gtaggggggactggatttcagcccagtacaaacctcccagggtgcctctgaccccttgcctgacccctgggggctgatggatct

cagcaccactcccttgcaaagtgctcccccccttgaatcaccgcaaaggctcctcagttcagaacccttagacctcatctccgt

ccccctttggcaactcttctccctcagatatagacgtccccaagccaggctccccggagccacaggtttctggccttgcagccaa

tcgttctctgacagaaggcctggtcctggacacaatgaatgacagcctcagcaagatcctgctggacatcagctttcctggcct

ggacgaggacccactgggccctgacaacatcaactggtcccagtttattcctgagctacagtagagccctgcccttgcccctg

tgctcaagctgtccaccatcccgggcactccaaggctcagtgcaccccaagcctctgagtgaggacagcaggcagggact

gttctgctcctcatagctccctgctgcctgattatgcaaaagtagcagtcacaccctagccactgctgggaccttgtgttccccaa

gagtatctgattcctctgctgtccctgccaggagctgaagggtgggaacaacaaaggcaatggtgaaaagagattaggaac

ccccccagcctgtttccattctctgcccagcagtctcttaccttccctgatctttgcagggtggtccgtgtaaatagtataaattctcca

aattatcctctaattataaatgtaagcttatttccttagatcattatccagagactgccagaaggtgggtaggatgacctgggggttt

caattgacttctgttccttgcttttagttttgatagaagggaagacctgcagtgcacggtttcttccaggctgaggtacctggatcttg

ggttcttcactgcagggacccagacaagtggatctgcttgccagagtccttttgcccctccctgccacctccccgtgtttccaag

tcagctttcctgcaagaagaaatcctggttaaaaaagtcttttgtattgggtcaggagttgaatttggggtgggaggatggatgc

aactgaagcagagtgtgggtgcccagatgtgcgctattagatgtttctctgataatgtccccaatcataccagggagactggca

ttgacgagaactcaggtggaggcttgagaaggccgaaagggcccctgacctgcctggcttccttagcttgcccctcagctttg

caaagagccaccctaggccccagctgaccgcatgggtgtgagccagcttgagaacactaactactcaataaaagcgaag

gtggacatgaaaaaaaaaaaaaaaaaa

Cancer Reference Gene Sequences SEQ ID NO: 12 (ALAS1; NM_000688)

aggctgctcccggacaagggcaacgagcgtttcgtttggacttctcgacttgagtgcccgcctccttcgccgccgcctctgcag

tcctcagcgcagttatgcccagttcttcccgctgtggggacacgaccacggaggaatccttgcttcagggactcgggaccctg

ctggacccccttcctcgggtttaggggatgtggggaccaggagaaagtcaggatccctaagagtcttccctgcctggatggatg

agtggcttcttctccacctagattctttccacaggagccagcatacttcctgaacatggagagtgttgttcgccgctgcccattctt

atcccgagtcccccaggcctttctgcagaaagcaggcaaatctctgttgttctatgcccaaaactgccccaagatgatggaag

ttggggccaagccagcccctcgggcattgtccactgcagcagtacactaccaacagatcaaagaaacccctccggccagt

gagaaagacaaaactgctaaggccaaggtccaacagactcctgatggatcccagcagagtccagatggcacacagcttc

cgtctggacacccttgcctgccacaagccagggcactgcaagcaaatgcctttcctggcagcacagatgaatcagagag

gcagcagtgtcttctgcaaagccagtcttgagcttcaggaggatgtgcaggaaatgaatgccgtgaggaaagaggttgctga

aacctcagcaggccccagtgtggttagtgtgaaaaccgatggagggggatcccagtggactgctgaagaacttccaggacat

catgcaaaagcaaagaccagaaagagtgtctcatcttcttcaagataacttgccaaaatctgtttccacttttcagtatgatcgttt

ctttgagaaaaaaattgatgagaaaaagaatgaccacacctatcgagttttaaaactgtgaaccggcgagcacacatcttcc

ccatggcagatgactattcagactccctcatcaccaaaaagcaagtgtcagtctggtgcagtaatgactacctaggaatgagt

cgccacccacgggtgtgtggggcagttatggacactttgaaacaacatggtgctggggcaggtggtactagaaatatttctgg

aactagtaaattccatgtggacttagagcgggagctggcagacctccatgggaaagatgccgcactcttgttttcctcgtgcttt

gtggccaatgactcaaccctcttcaccctggctaagatgatgccaggctgtgagatttactctgattctgggaaccatgcctcca

tgatccaagggattcgaaacagccgagtgccaaagtacatcttccgccacaatgatgtcagccacctcagagaactgctgc
aaagatctgacccctcagtccccaagattgtggcatttgaaactgtccattcaatggatggggcggtgtgcccactggaagag
ctgtgtgatgtggcccatgagtttggagcaatcaccttcgtggatgaggtccacgcagtggggctttatggggctcgaggcgga
gggattggggatcgggatggagtcatgccaaaaatggacatcatttctggaacacttggcaaagcctttggttgtgttggaggg
tacatcgccagcacgagttctctgattgacaccgtacggtcctatgctgctggcttcatcttcaccacctctctgccacccatgctg
ctggctggagccctggagtctgtgcggatcctgaagagcgctgagggacgggtgcttcgccgccagcaccagcgcaacgt
caaactcatgagacagatgctaatggatgccggcctccctgttgtccactgccccagccacatcatccctgtgcgggttgcag
atgctgctaaaaacacagaagtctgtgatgaactaatgagcagacataacatctacgtgcaagcaatcaattaccctacggt
gccccggggagaagagctcctacggattgcccccacccctcaccacacaccccagatgatgaactacttccttgagaatct
gctagtcacatggaagcaagtggggctggaactgaagcctcattcctcagctgagtgcaacttctgcaggaggccactgcat
tttgaagtgatgagtgaaagagagaagtcctatttctcaggcttgagcaagttggtatctgctcaggcctgagcatgacctcaat
tatttcacttaaccccaggccattatcatatccagatggtcttcagagttgtctttatatgtgaattaagttatattaaattttaatctata
gtaaaaacatagtcctggaaataaattcttgcttaaatggtg

SEQ ID NO: 13 (MMADHC; NM_015702)

cctttgcctgctcaccgccagcgtaggtgctaccaccgctgccgtcgccgccgccattttgatggcaggaagagtccggttctg
ggacagctggagacagtggtggtgactgaaataactttaccaaaggaaagctattttgcgaactatcttctccagcggagatg
gccaatgtgctttgtaacagagccagactggtttcctatctcccaggattttgctctttagttaaaagggttgtcaatcccaaagcc
ttttcgactgcaggatcatcaggttcggatgagtctcatgtggctgctgcacctccagatatatgctctcgaacagtgtggcctga
tgaaactatgggaccctttggacctcaagatcagaggttccagcttcctgggaacataggttttgattgtcacctcaatgggact
gcttcacagaagaaaagcctggttcataaaactttgcctgatgttctagcagaacctttatcaagtgaaagacatgagtttgtga
tggcacaatatgtgaatgaatttcagggtaatgatgcacctgttgaacaagaaattaacagtgcagaaacttactttgaaagtg
ccagagtagagtgtgcaatacagacatgtccagaattgctgcgaaaagattttgaatcactgtttccagaagtagctaatggc
aaactaatgattctgactgtaacacaaaaaactaagaatgatatgactgtttggagtgaagaagtagaaattgaaagagaag
tgctcttagaaaagttcatcaatggtgctaaggaaatttgctatgctcttcgagctgagggttattgggctgactttattgacccatc
atctggtttggcatttttggaccatatacaaacaacactcttttgaaactgatgaacgctaccgacatttaggattctctgttgatg
accttggatgctgtaaagtgattcgtcatagtctctggggtacccatgtagttgtagggagtatcttcactaatgcaacaccagac
agccatattatgaagaaattaagtggaaattagcagaaatatccattcatttgctgtactatttgtatgtaatatttgggttgatctat
aaacactgtcagactaaagttttaaaatatatttatttctaagtatttatttcagcatttatgaatttacaacattggcaagtgatttgg
gattttaaaattgcaaatgttcatttattcatatcattgaatacacgttgaacacatccacattgtataggatgtggtaattagcttgta
accagggtatgatctgctattgttatttctcctctttattggaaaaaggcctcagttttaattattttcttcccaaaataaatcacacattt
ggttacaa

SEQ ID NO: 14 (PPP2CA; NM_002715)

acagagagccgagctctggagcctcagcgagcggaggaggaggcgcagcggccgacggccgagtactgcggtgaga
gccagcgggccagcgccagcctcaacagccgccagaagtacacgaggaaccggcggcggcgtgtgcgtgtaggcccgt

gtgcgggcggcggcgcgggagcagcgcggagcggcagccggctggggcgggtggcatcatggacgagaaggtgttca

ccaaggagctggaccagtggatcgagcagctgaacgagtgcaagcagctgtccgagtcccaggtcaagagcctctgcga

gaaggctaaagaaatcctgacaaaagaatccaacgtgcaagaggttcgatgtccagttactgtctgtggagatgtgcatggg

caatttcatgatctcatggaactgtttagaattggtggcaaatcaccagatacaaattacttgtttatgggagattatgttgacaga

ggatattattcagttgaaacagttacactgcttgtagctcttaaggttcgttaccgtgaacgcatcaccattcttcgagggaatcat

gagagcagacagatcacacaagtttatggtttctatgatgaatgtttaagaaaatatggaaatgcaaatgtttggaaatatttttac

agatcttttgactatcttcctctcactgccttggtggatgggcagatcttctgtctacatggtggtctctcgccatctatagatacact

ggatcatatcagagcacttgatcgcctacaagaagttccccatgagggtccaatgtgtgacttgctgtggtcagatccagatga

ccgtggtggttggggtatatctcctcgaggagctggttacacctttgggcaagatatttctgagacatttaatcatgccaatggcct

cacgttggtgtctagagctcaccagctagtgatggagggatataactggtgccatgaccggaatgtagtaacgattttcagtgc

tccaaactattgttatcgttgtggtaaccaagctgcaatcatggaacttgacgatactctaaaatactctttcttgcagtttgaccca

gcacctcgtagaggcgagccacatgttactcgtcgtaccccagactacttcctgtaatgaaatttttaaacttgtacagtattgcca

tgaaccatatatcgacctaatggaaatgggaagagcaacagtaactccaaagtgtcagaaaatagttaacattcaaaaaac

ttgtttttcacatggaccaaaagatgtgccatataaaaatacaaagcctcttgtcatcaacagccgtgaccactttagaatgaac

cagttcattgcatgctgaagcgacattgttggtcaagaaaccagtttctggcatagcgctatttgtagttacttttgctttctctgaga

gactgcagataataagatgtaaacattaacacctcgtaatacaatttaacttccatttagctatagctttactcagcatgactgta

gataaggatagcagcaaacaatcattggagcttaatgaacatttttaaaaataattaccaaggcctcccttctacttgtgagtttt

gaaattgttctttttattttcagggataccgtttaatttaattatatgatttgtctgcactcagtttattccctactcaaatctcagccccat

gttgttctttgttattgtcagaacctggtgagttgttttgaacagaactgttttttccccttcctgtaagacgatgtgactgcacaagag

cactgcagtgtttttcataataaacttgtgaactaagaactgagaaggtcaaatttttaattgtatcaatgggcaagactggtgctgt

ttattaaaaaagttaaatcaattgagtaaattttagaatttgtagacttgtaggtaaaataaaaatcaagggcactacataacctc

tctggtaactccttgacattcttcagattaacttcaggatttatttgtatttcacatattacaatttgtcacattgttggtgtgcactttgtg

ggttcttcctgcatattaacttgtttgtaagaaaggaaatctgtgctgcttcagtaagacttaattgtaaaaccatataacttgagatt

taagtctttgggttttgttttaataaaacagcatgttttcaggtagagcttaaactaaatgatgtgtttacttagtgcagtttctggttatg

aatattatattgctatgtgtatattatatggactctttaaaatgattgacagattggcaaattcttaaatctttgtacattgttgagtcata

tgttcttagagttaaatttgtctcagataagaaagtgttaaagcattagcctgtgtcaagttctttgagtgatactagtgaaaccaa

atagaaaactattgttggatcatgatttagtcttatgtacattcacccgaagacaaaaatggtacttaaagtggcagtgttcaaca

tttaatgagtttttcccctttatccttcgaataggattagatgtttaaaaaaaagttcttctgtgggaactaatatttgatattttaaccta

ccagagtaaacaggaacacttaatcatacttgtgagtgtagtaaataaaagttttcttgctttgtgctgtgttgaatctggaaccaa

cagggaagttatagcatatcccctttctaaaatgcttgaggaacacatacataccgaatgtcttttctgatctaattgatagtattttt

agtggcttgtggagttaattttccaaagcaaaaggccattagggtttctacatttcatttcatttcattctttttctttcacaagaaatac

attctctgtgtgtcttttgttgctctgtcactctatgcccttttctctccgactgaacaaatagcttatccatgtgcagtggttttaataccc

aaacaatctagacaccaagcagctattttttccggtcctgtgatatcagaattgaccaaggaatacgtatattgtaattgacacgt

ggtggtatcttccaggtacaaattctctaaattttgtggttagcagaatgggacttgtgataagaatagcttggttttagcataacta

gggtttaaaataattgtttaattatagagactgaccgggctttggccatctaaactggaaagtgttagtaccctaccttcttttgaaa

atggctatggtaaggaaatgtgttagtaaattatgtatttcttgaaaaatacataattatggttggatgggaatcactaagttgg

gtgttaactgatgtctcaattagtaacattaggattttcattaataaacctaaaaagctttccctaagaacaggcctggcacagtg gctcatgcctgtaatcccagcactttgggagaccagggtgggtggatcccttgaggtcaggagttgaagagcagcctggcca acatggcaaaaccccatctctacaaaaaatatgaaaatcagccgggcttggtggcataccatagtcccagctacttgggag gctgaggtgagaggatcgcctgagcccaggagatggaggttgcagtgggctgagattgtgccactgtactccagtctgggtg acagagccagaccctgtctcaaaaataaagaggattctgagtttgtatagtgagggcttgcagaaattttgaaacttattttgtaa gtttacaatgaatttgtacatgatgtgctcatgtcttgggttgagtatcctagacatgatttttttcatttgctgcatattaaacatttgttgg ttgtagtcggtatttcttaaatagaagtttgtcaatattagattagtttcaagaaggacttagctcaggaaaaggatagttatttctgt ggttctcagctttgatgcctacagagattctgatttaaattgctggaggagggcccaggcatacattttttaagtttcccaagtgatt ctaatttacatctagggttaaaaacagccaggcgcggtggctcatgcctgtaatcccagcacttggggaggccggggtgggt ggatcacctgatgtcaggagatcgagaccagcctggccagcgtggtgaaaccccatctctattaaaaatacaaaactgacc ggtgtggtggcaggagcctgtaatcccagctacttgggaggctgaggcaggagaatcacttgaactcaggaggaggaggtt acagtgagcccagatcgtgccattgcactccagcctgggcaaaaagagcaaaactccatctcaaaaaacaaaaacacct cactgctgtttcctaagtacatacttaagaaaattgggatacatggtggtggttcatggatgttgataaggaattaaaatgtaccg tgcgactctctgtttcagtggtgacttttacctgtttagtataaatattcctttgcttccaaccataaatgtgttcttagaaatgggcctat agtttagtaacctatagttttggtataggcttgtttgttttcagatggattttggttctgtgagctaaagctattttgcattaaagccttcg tcctcaca

SEQ ID NO: 15 (TFRC; NM_003234)

agagcgtcgggatatcgggtggcggctcgggacggaggacgcgctagtgtgagtgcgggcttctagaactacaccgaccc tcgtgtcctccttcatcctgcggggctggctggagcggccgctccggtgctgtccagcagccataggagccgcacggggga gcgggaaagcggtcgcggccccaggcggggcggccgggatggagcgggggccgcgagcctgtggggaaggggctgtg gcggcgcctcgagcggctgcaggttcttctgtgtggcagttcagaatgatggatcaagctagatcagcattctctaacttgtttgg tggagaaccattgtcatatacccggttcagcctggctcggcaagtagatggcgataacagtcatgtggagatgaaacttgctgt agatgaagaagaaaatgctgacaataacacaaaggccaatgtcacaaaaccaaaaaggtgtagtggaagtatctgctatg ggactattgctgtgatcgtctttttcttgattggatttatgattggctacttgggctattgtaaaggggtagaaccaaaaactgagtgt gagagactggcaggaaccgagtctccagtgaggggaggagccaggagaggacttccctgcagcacgtcgcttatattggga tgacctgaagagaaagttgtcggagaaactggacagcacagacttcaccggcaccatcaagctgctgaatgaaaattcata tgtccctcgtgaggctggatctcaaaaagatgaaaatcttgcgttgtatgttgaaaatcaatttcgtgaatttaaactcagcaaag tctggcgtgatcaacattttgttaagattcaggtcaaagacagcgctcaaaactcggtgatcatagttgataagaacggtagac ttgtttacctggtggagaatcctggggggttatgtggcgtatagtaaggctgcaacagttactggtaaactggtccatgctaattttg gtactaaaaagattttgaggatttatacactcctgtgaatggatctatagtgattgtcagagcagggaaaatcacctttgcaga aaaggttgcaaatgctgaaagcttaaatgcaattggtgtgttgatatacatggaccagactaaatttcccattgttaacgcagaa ctttcattctttggacatgctcatctggggacaggtgacccttacacacctggattcccttccttcaatcacactcagtttccaccat ctcggtcatcaggattgcctaatatacctgtccagacaatctccagagctgctgcagaaaagctgtttgggaatatggaagga gactgtccctctgactggaaaacagactctacatgtaggatggtaacctcagaaagcaagaatgtgaagctcactgtgagca atgtgctgaaagagataaaaattcttaacatctttggagttattaaaggctttgtagaaccagatcactatgttgtagttgggggccc

agagagatgcatgggggccctggagctgcaaaatccggtgtaggcacagctctcctattgaaacttgcccagatgttctcagat

atggtcttaaaagatgggtttcagcccagcagaagcattatctttgccagttggagtgctggagactttggatcggttggtgcca

ctgaatggctagagggatacctttcgtccctgcatttaaaggctttcacttatattaatctggataaagcggttcttggtaccagca

acttcaaggtttctgccagcccactgttgtatacgcttattgagaaaacaatgcaaaatgtgaagcatccggttactgggcaattt

ctatatcaggacagcaactgggccagcaaagttgagaaactcactttagacaatgctgctttccctttccttgcatattctggaat

cccagcagtttctttctgtttttgcgaggacacagattatccttatttgggtaccaccatggacacctataaggaactgattgagag

gattcctgagttgaacaaagtggcacgagcagctgcagaggtcgctggtcagttcgtgattaaactaacccatgatgttgaatt

gaacctggactatgagaggtacaacagccaactgctttcatttgtgagggatctgaaccaatacagagcagacataaagga

aatgggcctgagtttacagtggctgtattctgctcgtggagacttcttccgtgctacttccagactaacaacagatttcgggaatg

ctgagaaaacagacagatttgtcatgaagaaactcaatgatcgtgtcatgagagtggagtatcacttcctctctccctacgtatc

tccaaaagagtctcctttccgacatgtcttctggggctccggctctcacacgctgccagctttactggagaacttgaaactgcgt

aaacaaaataacggtgcttttaatgaaacgctgttcagaaaccagttggctctagctacttggactattcagggagctgcaaat

gccctctctggtgacgtttgggacattgacaatgagttttaaatgtgataccatagcttccatgagaacagcagggtagtctggt

ttctagacttgtgctgatcgtgctaaattttcagtagggctacaaaacctgatgttaaaattccatcccatcatcttggtactactag

atgtctttaggcagcagcttttaatacagggtagataacctgtacttcaagttaaagtgaataaccacttaaaaaatgtccatgat

ggaatattcccctatctctagaattttaagtgctttgtaatgggaactgcctctttcctgttgttgttaatgaaaatgtcagaaaccag

ttatgtgaatgatctctctgaatcctaagggctggtctctgctgaaggttgtaagtggtcgcttactttgagtgatcctccaacttcatt

tgatgctaaataggagataccaggttgaaagaccttctccaaatgagatctaagcctttccataaggaatgtagctggtttcctc

attcctgaaagaaacagttaactttcagaagagatgggcttgttttcttgccaatgaggtctgaaatggaggtccttctgctggat

aaaatgaggttcaactgttgattgcaggaataaggccttaatatgttaacctcagtgtcatttatgaaaagaggggaccagaa

gccaaagacttagtatattttcttttcctctgtcccttcccccataagcctccatttagttctttgttattttttgtttcttccaaagcacattg

aaagagaaccagtttcaggtgtttagttgcagactcagtttgtcagactttaaagaataatatgctgccaaattttggccaaagtg

ttaatcttaggggagagctttctgtccttttggcactgagatatttattgtttatttatcagtgacagagttcactataaatggtgtttttt

aatagaatataattatcggaagcagtgccttccataattatgacagttatactgtcggttttttttaaataaaagcagcatctgctaa

taaaacccaacagatactggaagttttgcatttatggtcaacacttaagggtttttagaaaacagccgtcagccaaatgtaattg

aataaagttgaagctaagatttagagatgaattaaatttaattaggggttgctaagaagcgagcactgaccagataagaatgc

tggttttcctaaatgcagtgaattgtgaccaagttataaatcaatgtcacttaaaggctgtggtagtactcctgcaaaattttatagc

tcagtttatccaaggtgtaactctaattcccattttgcaaaatttccagtacctttgtcacaatcctaacacattatcgggagcagtg

tcttccataatgtataaagaacaaggtagttttacctaccacagtgtctgtatcggagacagtgatctccatatgttacactaagg

gtgtaagtaattatcgggaacagtgtttcccataatttttcttcatgcaatgacatcttcaaagcttgaagatcgttagtatctaacat

gtatcccaactcctataattccctatcttttagtttttagttgcagaaacattttgtggtcattaagcattgggtgggtaaattcaaccac

tgtaaaatgaaattactacaaaatttgaaatttagcttgggttttttgttacctttatggtttctccaggtcctctacttaatgagatagta

gcatacatttataatgtttgctattgacaagtcattttaactttatcacattatttgcatgttacctcctataaacttagtgcggacaagt

tttaatccagaattgacctttgacttaaagcagagggactttgtatagaaggtttgggggctgtggggaaggagagtcccctga

aggtctgacacgtctgcctacccattcgtggtgatcaattaaatgtaggtatgaataagttcgaagctccgtgagtgaaccatca

ttataaacgtgatgatcagctgtttgtcatagggcagttggaaacggcctcctagggaaaagttcatagggtctcttcaggttctt

agtgtcacttacctagatttacagcctcacttgaatgtgtcactactcacagtctctttaatcttcagttttatctttaatctcctcttttatc ttggactgacatttagcgtagctaagtgaaaaggtcatagctgagattcctggttcgggtgttacgcacacgtacttaaatgaaa gcatgtggcatgttcatcgtataacacaatatgaatacagggcatgcattttgcagcagtgagtctcttcagaaaacccttttcta cagttagggttgagttacttcctatcaagccagtacgtgctaacaggctcaatattcctgaatgaaatatcagactagtgacaag ctcctggtcttgagatgtcttctcgttaaggagatgggccttttggaggtaaaggataaaatgaatgagttctgtcatgattcactat tctagaacttgcatgacctttactgtgttagctctttgaatgttcttgaaattttagactttctttgtaaacaaatgatatgtccttatcatt gtataaaagctgttatgtgcaacagtgtggagattccttgtctgatttaataaaatacttaaacactgaaaaaaaaaaa

SEQ ID NO: 16 (CLTC; NM_004859)

agccgtttccggagtctgcgctgcgcccggttccgccattgcggctctcctggcccctggagcctccgcccccgacccgagct
ctttcgtctgcctgccagtttcctgcgtccccggagaggatcctgctgagcccagcctcccccctcccttctcctcctctcccttg
gagagcccgggcagccactgccccgcagccccagtgacaggaggagaccataaccccgacagcgccatggcccag
attctgccaattcgttttcaggagcatctccagctccagaacctgggtatcaacccagcaaacattggcttcagtaccctgacta
tggagtctgacaaattcatctgcattagagaaaaagtaggagagcaggcccaggtggtaatcattgatatgaatgacccaag
taatccaattcgaagaccaatttcagcagacagcgccatcatgaatccagctagcaaagtaattgcactgaaagctgggaa
aactcttcagatttttaacattgaaatgaaaagtaaaatgaaggctcataccatgactgatgatgtcacctttggaaatggatct
ctttgaatacggttgctcttgttacggataatgcagtttatcactggagtatggaaggagagtctcagccagtgaaaatgtttgatc
gccattctagccttgcagggtgccagattatcaattaccgtacagatgcaaaacaaaagtggttacttctgactggtatatctgc
acagcaaaatcgtgtggtgggagctatgcagctatattctgtagataggaaagtgtctcagcccattgaaggacatgcagcta
gctttgcacagtttaagatggaaggaaatgcagaagaatcaacgttattttgttttgcagttcggggccaagctggagggaagt
tacatattattgaagttggcacaccacctacagggaaccagcccctttccaaagaaggcagtggatgtcttctttcctccagaag
cacaaaatgattttcctgttgcaatgcagatcagtgaaaagcatgatgtggtgttcttgataaccaagtatggttatatccacctct
atgatcttgagactggtacctgcatctacatgaatagaatcagtggagaaacaattttttgttactgcacctcatgaagccacagc
tggaataattggagtaaacagaaagggacaagttctgtcagtgtgtgtggaagaagaaaacataattccttacatcaccaat
gttctacaaaatcctgatttggctctgagaatggctgtacgtaataacttagccggtgctgaagaactctttgcccggaaatttaat
gctctttttgcccagggaaattactcggaggcagcaaaggtggctgctaatgcaccaaagggaattcttcgtactccagacac
tatccgtcggttccagagtgtcccagcccagccaggtcaaacttctcctctacttcagtactttggtatcctttggaccagggaca
gctcaacaaatacgaatccttagagctttgtaggcctgtacttcagcaagggcgaaaacagcttttggagaaatggttaaaag
aagataagctggaatgttctgaagaactgggtgatcttgtgaaatctgtggaccctacattggcacttagtgtgtacctaagggc
taacgtcccaaataaagtcattcagtgctttgcagaaacaggtcaagtccaaaagattgttttatatgctaaaaaagttggatac
actccagattggatatttctgctgagaaatgtaatgcgaatcagtccagatcagggacagcagtttgcccaaatgttagttcaa
gatgaagagcctcttgctgacatcacacagattgtagatgtctttatggaatacaatctaattcagcagtgtactgcattcttgcttg
atgctctgaagaataatcgcccatctgaaggtcctttacagacgcggttacttgagatgaaccttatgcatgcgcctcaagttgc
agatgctattctaggcaatcagatgttcacacattatgaccgggctcatattgctcaactgtgtgaaaaggctggcctactgcag
cgtgcattagaacatttcactgatttatatgatataaaacgtgcagtggttcacacccatcttcttaaccctgagtggttagtcaact
actttggttccttatcagtagaagactccctagaatgtctcagagccatgctgtctgccaacatccgtcagaatctgcagatttgtg

ttcaggtggcttctaaatatcatgaacaactgtcaactcagtctctgattgaactttttgaatctttcaagagttttgaaggtctctttta

ttttctgggatccattgttaactttagccaggacccagatgtgcactttaaatatattcaggcagcttgcaagactgggcaaatca

aagaagtagaaagaatctgtagagaaagcaactgctacgatcctgagcgagtcaagaattttcttaaggaagcaaaactaa

cagatcagctaccacttatcattgtgtgtgatcgatttgactttgtccatgatttggtgctctatttatatagaaataatcttcaaaagt

atatagagatatatgtacagaaggtgaatccaagtcgacttcctgtagttattggaggattacttgatgttgactgttctgaagatg

tcataaaaaacttgattcttgttgtaagaggtcaattctctactgatgagcttgttgctgaggttgaaaaaagaaacagattgaaa

ctgcttctgccttggctagaggccagaattcatgagggctgtgaggagcctgctactcacaatgccttagccaaaatctacata

gacagtaataacaacccggagagatttcttcgtgaaaatccctactatgacagtcgcgttgttggaaagtattgtgagaagag

agatccacatctggcctgtgttgcttatgaacgtggccaatgtgatctggaacttattaatgtttgcaatgagaattccctcttcaaa

agtctttctcgctacctggtacgtcgaaaggatccagaattgtggggcagcgtgctgctggaaagcaatccttacaggagacc

cctaattgaccaggttgtacaaacagctttgtctgagactcaggaccctgaagaagtgtcagtaactgtaaaggctttcatgact

gcagaccttcctaatgaactcattgaactgctggagaaaattgtccttgataactctgtattcagtgaacacaggaatctgcaaa

acctccttatcctcactgcaattaaggctgaccgtacacgtgttatggagtatattaaccgcctggataattatgatgccccagat

attgccaatatcgccatcagcaatgagctgtttgaagaagcatttgccattttccggaaatttgatgtcaatacttcagcagttcag

gtcttaattgagcatattggaaacttggatcgggcatatgagtttgctgaacgttgcaatgaacctgcggtctggagtcaacttgc

aaaagcccagttgcagaaaggaatggtgaaagaagccattgattcttatatcaaagcagatgatccttcctcctacatggaa

gttgttcaggctgccaatactagtggaaactgggaagaactggtgaagtacttgcagatggcccgtaagaaggctcgagagt

cctatgtggagacagaactgatattcgcactggctaaaacaaaccgccttgcagagttagaagaatttatcaatggaccaaat

aatgctcatatccaacaagttggtgaccgttgttatgatgaaaaaatgtatgatgctgctaagttgttgtacaataatgtttccaattt

tggacgtttggcatctaccctggttcacctgggtgaatatcaggcagctgttgatggggctaggaaagctaacagtactcgaac

atggaaagaggtctgcttcgcctgtgtagatgggaaagaattccgtcttgctcagatgtgtgtggacttcatattgttgtacatgcag

atgaattagaagaacttatcaactactatcaggatcgtggctattttgaagagctgatcaccatgttggaagcagcactgggac

ttgagcgagctcacatgggaatgtttactgaattagctattctatactctaaatttaagcctcagaaaatgagggagcacctgga

gctgttctggtctagagtgaatattcccaaggtgctaagagctgcagaacaagctcatctttgggcagaactggtgtttttgtatg

acaagtatgaagaatatgataatgccataattaccatgatgaatcatccaactgatgcctggaaagaagggcaattcaaaga

tatcattaccaaggttgccaatgtggaactatactacagagcaatacagttctacttagaattcaagcctctgttgttaaatgattt

gctgatggtgctgtctccacggttggatcacactcgtgcagtcaattatttcagcaaggttaaacagctaccactggtgaaacc

gtatttgcgttcagttcagaaccataacaacaaatctgtgaatgaatcattgaacaatctttttattacagaagaagattatcagg

ctctgcgaacatcaatagatgcttatgacaactttgacaatatctcgcttgctcagcgtttggaaaaacatgaactcattgagttc

aggagaattgctgcttatctcttcaaaggcaacaatcgctggaaacagagtgtagagctgtgcaagaaagacagcctttaca

aggatgcaatgcagtatgcttctgaatctaaagatactgaattggctgaagaactcctgcagtggttttttgcaggaagaaaaaa

gagagtgctttggagcttgtctgtttacctgttacgatcttttaaggccagatgtcgtcctagaaactgcatggaggcacaatatca

tggattttgccatgccctatttcatccaggtcatgaaggagtacttgacaaaggtggataaattagatgcttcagaatcactgag

aaaagaagaagaacaagctacagagacacaacccattgtttatggtcagccccagttgatgctgacagcaggacccagtg

ttgccgtccctccccaggcacctttttggttatggttataccgcaccaccgtatggacagccacagcctggctttgggtacagcat

gtgagatgaagcgctgatcctgtagtcacctattttcgtactgaaacatcgtctttacccacttctcagtttataatggggggaaaac

aggcaacgtgttcttgtaacctttatttcatgaaggacttcttttttgtttctaactataaacttggatcacctatgttaaaaccttatttca

cattccacatcattttagaatttattttcgaaggggaatagtttcaatgttttattcacttgggctttttttcttcccccctctttctttaaagaa

ctgctcaatattcaatctgttgtgaagaacctgatttgcactctgtagtgtttaaagaaacaaagaaactctaatattgaatctctta

aatttagtgtatgtaaacagcttacaaatacgtattgtctaaatgcatttaaatctgttttattcaaagaaaagctaaagcaaaaa

cactggcatatgaccatgcaagactgtcagtgccaacaaagacaacactaatcagcacatcgtacactggattgcagtgctt

cccagattattgaaaaatgttacagacaacttgcctgatttttaaatgagcgtaaaaggccctctaacctatgcaggtttccccat

tatgcatatagaaaatgctagtatgttttgctcacttcatatgtaacaggtgcccttatgttgtgctgtatcctgtgctttttctgtggga

ccattccattcaggagcaaagagcaccatgattccaatcttgtgtgtgtttactaacccttccctgaggtttgtgtatgttggatattg

tggtgttttagatcactgagtgtacagaagagagaaattcaaacaaaatattgctgttcttcagttttgtttgtggaatttgaaattac

tcaaatttaaaataaattactggactgtggaataacatagaattgaagtttttaattaaataccactcaaacgaaaagaacagt

agtttttgtagttttatattggatactgaggcattagggaggcatgaaaggaagaggaatgaggattgagacatgtgaagacat

tgtgcattatatcaatgtgcattcctgtagttcattaacaaggtacatgcaatagtctaaagaaccagagtcactactatagtggc

ttaacatttaatctgtctccaatattttaaccaagtgacaccgaggttttatcgaagcatttcacttaaatgaacaaatcatggctgt

tatattaacttgaaataaaatatatttaaacatgtagttaacatgctctttctcatcatgaattataaaggtgcccaaattatgggtat

cttgattttttcaaaaattggttgtgtgaatccagctcttgcttatggctgagaatgttcctaaagttcatttcactatcccttaatcctc

aatagctacacatacccccttgcctagttagtcttgcactgctttacaactctcttaggcaaattacctaacataagctccacaga

agagttttttaaaacttaaaatccctgtcccaccaggtgtggtggctcatgcctgtaatcccagcactttgggaggctgaggcag

gcggattgcttgaggccaggagttcgagacaagcctgaccaacatggtgactccatctctactaaaaataaaaaatcagcc

aggtgtgatggtggatgcctataagatcctagctgctactcaggaggcaggaggctgaggcatgagaatcacttgaacctgt

gaggcaaaggttgtagtgagccaagatcacgtcactgcactccatcctgggtgacagaatgagactcttgtctcaaaaaaca

aaaaaacctatttagtttcatccaggacattaagtgaaagtgtttaatgtgagtgcaggtcagtgaagaatacaatggtgcccct

gctttgatttgtgggaggtgccatcagtttttccccaccactgcttttgcaccctcagtgcaaatatcaacacagagaaaaaaaa

aataataatagtattatgaaaacattgactctgtggagaccctgtgggttctgcagagtatactttgaaaactataagattatgag

ttctataaaataccaagtacagatgaactagaaaacaagcatatatccacaattaccagaattttcatagtattcctttttagtaga

tatgccatgacagttacttagccaccccatgccatcctgttgggaatgccactacctttgaatttggagccatcagtctatctgag

gcaaacctcagaaattacctgagccagaatcatgcacttagcactgagttcaactcagtcttaacgttgcattttgttgcatcactt

ttgcagccagtaaacattctgtttagaaatagttgctattgtggcatcattcacagttgcaattttttctcctgtgctgtcatatgcctgtg

cgaagtgtattaaatgaatcaaatattagaaatttcaaggctgtctttagtgttctgcccacaatactccaccaatgaccttagac

cttgagatggtgctataacgctgcttatattgcagttaaccatagagaggtggagccacttaaaatgggcctattaccaaaattc

tccctggcatcattacagttcttggggatacactgattttagaaattactgtcagtatcctcctaaaagaaggcttaaatagtttcta

acaagatgactatcaggaagctatgtggcttggggagttgggaccctctgcctcatattctgggtaactggtataacatagtaat

tattaggattgtatcaagtgtatctaaagatcaaaagggaaaaaaggcacccttaattgtcaaagttagttctaggaaaaaata

ctagcagggatgacaaactacgttcactttacagttaccataaattcttacttgggcccacccatttccatctgttaaggtccataa

ggttactcaagtttcagtggcttcattgttacaagaaaggggggaagtgagaaactttaataggctgtgatttagaaccactgct

gattaacctttggccagtatgggtcagggaaggtgaacatgaaacactgcacagtaggttgtatcaatgctataaaac

**Statistical analysis**

The Cancer Genome Atlas (TCGA) prostate cancer data set (1) was used to identify a panel of genes that are prognostic for aggressive prostate cancer. TCGA data set included RNA sequencing data (on approximately 21,000 genes) and clinical data for 399 samples. Three methods were used to identify the panel of 11 genes:

1) Statistical modelling to identify genes that add statistically significant prognostic value to standard clinical information currently used by clinicians
2) ARACNe: gene regulation network analysis to identify genes that regulate prognostic genes identified in (1) above
3) Analysis of bioinformatic databases (Ingenuity Pathway Analysis: Database for Annotation, Visualization and Integrated Discovery were used to identify which of the genes from (1) and/or (2) above have biological functions relevant to cancer

The panel of 11 genes (namely, TTC21B, MCM3, MTUS1, FOXM1, CUL4A, MCM3, GPC1, PCNA, IRF7, BRIP1, ULK1, and SPAG5) were further evaluated for two clinical outcomes in a cohort of samples from Ireland (PCRC, N = 426 samples) by 100 iterations of 2-fold cross-validation of all 2,047 possible combinations of 1 to 11 genes.
The primary clinical outcome was adverse pathology (AP), defined as any of the following six features: pathological Gleason grade $\geq$ 4+3, pathological stage $\geq$ T3, node positive, tertiary Gleason 5 present, cribriform present, or biochemical recurrence. The AP outcome was modelled using binary logistic regression and the genes were evaluated using the likelihood ratio statistic (LRS, larger values are better) and the area under the receiving operator characteristics curve (AUC, which can have values from 0.5 to 1.0, larger values are better).
The secondary clinical outcome of the study was time to biochemical recurrence (BCR). The BCR outcome was modelled using Cox proportional hazards regression and the genes were evaluated using the likelihood ratio statistic (LRS, larger values are better) and the C index (which, analogous to the AUC above, can have values from 0.5 to 1.0, larger values are better).

**Immunohistochemistry**

TMA slides were deparaffinised in xylene and rehydrated in descending gradient alcohols. Heat-mediated antigen retrieval was performed using 10 mM sodium citrate buffer (pH 6.0) in a PT module (LabVision, UK) for 15 min at 95°C. The LabVision IHC kit (LabVision, UK) was used for staining. Endogenous peroxidase activity was blocked by incubation with 3% hydrogen peroxide for 10 min. Sections were blocked for 10 min in UV blocking agent and the relevant primary antibody was incubated for 1 hr. Sections were washed in phosphate buffered saline with 0.1% Tween 20 (PBS-T), following which primary antibody enhancer was applied for 20 min, and sections were washed in PBS-T. Sections were then incubated with HRP polymer for 15 min, washed in PBS-T and then developed for 10 min using diaminobenzidine (DAB) solution (LabVision, UK). All incubations and washing stages were carried out at room temperature. The sections were counterstained in haematoxylin, dehydrated in alcohol and xylene and mounted using DPX mounting medium. As a negative control, the primary antibody was substituted with PBS-T.
Primary antibodies used were anti-FOXM1 (MilliporeSigma/MerckKGaA Cat. No. SAB1412254), anti- PTTG1 (MilliporeSigma/MerckKGaA Cat. No. WH0009232M1), anti-ZNF367 (MilliporeSigma/MerckKGaA Cat. No. HPA015785), anti-AGER (MilliporeSigma/MerckKGaA Cat. No. AV41642), anti-CUL4A (MilliporeSigma/MerckKGaA Cat. No. SAB1411512), anti-TTC21B (MilliporeSigma/MerckKGaA Cat. No. HPA035495), anti-MCM3 (MilliporeSigma/MerckKGaA Cat. No. SAB1412669), anti-GPC1 (MilliporeSigma/MerckKGaA Cat. No. HPA030571), anti-PCNA (MilliporeSigma/MerckKGaA Cat. No. HPA030521), anti-MTUS1 (MilliporeSigma/MerckKGaA Cat. No. WH0057509M1), anti-IRF7 (MilliporeSigma/MerckKGaA Cat. No. PRS3941), anti-BRIP1 (MilliporeSigma/MerckKGaA Cat. No. HPA005474), anti-ULK1 (MilliporeSigma/MerckKGaA Cat. No. WH0008408M1), and anti-SPAG5 (MilliporeSigma/MerckKGaA Cat. No. HPA022479.

**TMA analysis**

Slides were scanned at 20x magnification using a ScanScope XT slide scanner (Aperio Technologies, CA). For manual scoring, staining of tumor cells was evaluated by a pathologist on the basis of intensity, on a scale of negative (0), weak (1), moderate (2) and strong (3); and percentage, on a scale of 0-6 (0 = 0-1%; 1 = 1-10%; 2 = 10-25%: 3 = 25-50%; 4 = 50-75%; 5 = 75-90%; 6 = 90-100%).

*Results*

Statistical modelling and ARACNe analysis of TCGA identified the following panel of 11 genes as statistically significantly prognostic for aggressive prostate cancer and/or a regulator of genes that are statistically significantly prognostic for aggressive prostate cancer: TTC21B, MCM3, MTUS1, FOXM1, CUL4A, GPC1, PCNA, IRF7, BRIP3, ULK1, and SPAG5. Analysis of bioinformatic databases confirmed that these genes are involved in biological processes relevant to cancer.

The panel of 11 genes were further evaluated for two clinical outcomes in a second cohort of samples (PCRC, N = 426 samples) by 100 iterations of 2-fold cross-validation of all 2,047 possible combinations of 1 to 11 genes.

Results for all 2,047 gene combinations

For the primary clinical outcome of adverse pathology:

- 1,831/2,047 (89.4%) of the gene combinations were statistically significantly (LRS p-value < 0.05) prognostic.
- 1,679/ 2,047 (82.0%) of the gene combinations add statistically significant (LRS p-value < 0.05) prognostic value to clinical information consisting of optimally weighted biopsy information Gleason score, clinical stage and PSA.
- 1,779/2,047 (86.9%) of the gene combinations add statistically significant (LRS p-value < 0.05) prognostic value to clinical information consisting of the Cancer of the Prostate Risk Assessment (CAPRA) 0 to 10 score (Cooperberg MR, Pasta DJ, Elkin EP, et al. The University of California, San Francisco Cancer of the Prostate Risk Assessment score: a straightforward and reliable preoperative predictor of disease recurrence after radical prostatectomy. J Urol 2005;173:1938-42).
- 1,798/2,047 (87.8%) of the gene combinations add statistically significant (LRS p-value < 0.05) prognostic value to clinical information consisting of the European Association of Urology risk categories of low, intermediate, or high (Mottet N, Bellmunt J, Briers E, et al.: members of the EAU - ESTRO - ESUR -SIOG Prostate Cancer Guidelines Panel. EAU - ESTRO - ESUR - SIOG Guidelines on Prostate Cancer. Retrieved from: https://uroweb.org/guideline/prostate-cancer/#4).

For the secondary clinical outcome biochemical recurrence, 1,660/2,047 (81.1%) of the gene combinations were statistically significantly (LRS p-value < 0.05) prognostic.

Results for all 512 gene combinations that include MTUS1 and TTC21B

The 512 gene combinations were particularly prognostic for aggressive prostate cancer. For the primary clinical outcome adverse pathology:

- All 512/512 (100.0%) of the gene combinations were statistically significantly (LRS p-value < 0.05) prognostic (see Figure 1(A)).
- All 512/512 (100.0%) of the gene combinations add statistically significant (LRS p-value < 0.05) prognostic value to clinical information consisting of optimally weighted biopsy information Gleason score, clinical stage and PSA (see Figure 1(B)).
- All 512/512 (100.0%) of the gene combinations add statistically significant (LRS p-value < 0.05) prognostic value to clinical information consisting of the Cancer of the Prostate Risk Assessment (CAPRA) 0 to 10 score (see Figure 1(C)).
- All 512/512 (100.0%) of the gene combinations add statistically significant (LRS p-value < 0.05) prognostic value to clinical information consisting of the European Association of Urology risk categories of low, intermediate, or high (see Figure 1(D)).

For the secondary clinical outcome biochemical recurrence, 496/512 (96.9%) of the gene combinations were statistically significantly (LRS p-value < 0.05) prognostic (see Figure 2).

Results for the 2-gene combination MTUS1 and TTC21B

For the primary clinical outcome adverse pathology, the 2-gene combination MTUS1 and TTC21B:

- Is statistically significantly (LRS p-value = 0.0013) prognostic (see Figure 1, "Molecular only" panel, red dot)
- Add statistically significant (LRS p-value = 0.0101) prognostic value to clinical information consisting of optimally weighted biopsy information Gleason score, clinical stage and PSA (see Figure 1, "Added value to clinical" panel, red dot)
- Adds statistically significant (LRS p-value = 0.0082) prognostic value to clinical information consisting of the Cancer of the Prostate Risk Assessment (CAPRA) 0 to 10 score (Ref A) (see Figure 1, "Added value to CAPRA" panel, red dot)

- Adds statistically significant (LRS p-value = 0.0028) prognostic value to clinical information consisting of the European Association of Urology risk categories of low, intermediate, or high (Ref B) (see Figure 1, "Added value to EAU" panel, red dot)

For the secondary clinical outcome biochemical recurrence, the 2-gene combination MTUS1 and TTC21B is statistically significantly (LRS p-value = 0.0042) prognostic (see Figure 2, red dot).

Results for the 4-gene combination FOXM1, MCM3, MTUS1 +, and TTC21B

For the primary clinical outcome adverse pathology, the 4-gene combination FOXM1, MCM3, MTUS1, and TTC21B:

- Is statistically significantly (LRS p-value < 0.0001) prognostic (see Figure 1(A), green dot)
- Add statistically significant (LRS p-value = 0.0001) prognostic value to clinical information consisting of optimally weighted biopsy information Gleason score, clinical stage and PSA (see Figure 1(B), green dot)
- Adds statistically significant (LRS p-value = 0.0003) prognostic value to clinical information consisting of the Cancer of the Prostate Risk Assessment (CAPRA) 0 to 10 score (see Figure 1(C), green dot)
- Adds statistically significant (LRS p-value < 0.0001) prognostic value to clinical information consisting of the European Association of Urology risk categories of low, intermediate, or high (see Figure 1(D), green dot)

For the secondary clinical outcome biochemical recurrence, the 4-gene combination FOXM1, MCM3, MTUS1, and TTC21B is statistically significantly (LRS p-value = 0.0009) prognostic (see Figure 2, green dot).

## *Discussion*

The genes identified in this study are individually prognostic for aggressive or recurrent prostate cancer, with the combination of the genes offering stronger prognostic performance. Particularly, the combination of TTC21B and MTUS1 offers superior prognostic performance as a 2-gene prognostic signature.

The prognostic performance of the gene combinations of the claimed invention provides significant improvement to the currently available clinical pathological variables alone. The improvement of the prognostic performance can enable more accurate risk stratification to the patients diagnosed with cancer, such as prostate cancer. Accurate risk stratification on the one hand can reduce the prescription of unnecessary aggressive treatment, and associated side-effect, to patients with a lower risk of having an aggressive or a recurrent cancer. On the other hand, identified patients who have higher risk of having an aggressive or a recurrent cancer are correctly considered as candidates for treatment which reduces the risk of the aggressiveness or the recurrence of cancer. Accurate stratification provided by the methods of the claimed invention, therefore, has potential to help reduce the healthcare costs associated with managing unwarranted side effects and reduce the costs associated with managing aggressive or recurrent cancer as a result of under-treatment. Most importantly, accurate stratification arising from the methods of the claimed invention can help to improve the quality of lives of patients whom, as a result of the accurate stratification, received the most optimal treatment. Additionally, a reported obstacle in accessing risk stratification information is the high cost associated with the test, which is partly due to the molecular method and the large panels of biomarkers analysed. The concise nature of the methods of the claimed invention offers advantages over the existing methods who employ larger panels of prognostic signatures by reducing the assay costs and reducing the technical barrier to introduce a point-of-care, near patient, and rapid prognostic test.

In the specification the terms "comprise, comprises, comprised and comprising" or any variation thereof and the terms "include, includes, included and including" or any variation thereof are considered to be totally interchangeable and they should all be afforded the widest possible interpretation and vice versa.

The invention is not limited to the embodiments hereinbefore described but may be varied in both construction and detail.

## Claims

1. A method of predicting risk of an aggressive or a recurrent cancer in an individual, the method comprising a step of assaying a biological sample from the individual for expression of at least two genes, or proteins encoded by said genes, selected from TTC21B, MCM3, MTUS1, FOXM1, CUL4A, GPC1, PCNA, IRF7, BRIP1, ULK1, and SPAG5, wherein when a combination of a normalised expression value of the at least two genes, or proteins encoded by said genes, is modified relative to a combination of a normalised expression value of the at least two genes, or proteins encoded by said, in a biological sample from a subject with non-aggressive or non-recurrent cancer, the individual is predicted to have an increased risk of having an aggressive or a recurrent cancer.

2. A method of predicting risk of recurrence or progression of cancer in an individual with cancer following treatment, the method comprising: assaying a biological sample obtained from the individual for expression of at least two genes, or proteins encoded by said genes, selected from TTC21B, MCM3, MTUS1, FOXM1, CUL4A, GPC1, PCNA, IRF7, BRIP1, ULK1, and SPAG5, wherein when a combination of a normalised expression value of the at least two genes, or proteins encoded by said genes, is modified relative to a combination of a normalised expression value of the at least two genes from a biological sample obtained from a subject following treatment with no recurrence or progression of cancer, the individual is predicted to have an increased risk of recurrence of cancer or risk of progression of cancer following the treatment.

3. A method of determining a 5-year survival rate or a 10-year survival rate of an individual diagnosed with cancer, the method comprising: assaying a biological sample from the individual for expression of at least two genes, or proteins encoded by those genes, selected from TTC21B, MCM3, MTUS1, FOXM1, CUL4A, GPC1, PCNA, IRF7, BRIP1, ULK1, and SPAG5; wherein when a combination of a normalised expression value of the at least two genes, or proteins encoded by said genes, is modified relative to a combination of a normalised expression value of the at least two genes from a biological sample obtained from a subject who survived cancer after 5 or 10 years following cancer diagnosis, the individual is predicted to have a decreased 5-year survival rate or 10-year survival rate.

4. A method of identifying a cancer patient that is suitable for treatment with a therapy for preventing recurrence or progression of the cancer, the method comprising: assaying a biological sample from the individual for expression of at least two genes, or proteins encoded by those genes, selected from TTC21B, MCM3, MTUS1, FOXM1, CUL4A, GPC1, PCNA, IRF7, BRIP1, ULK1, and SPAG5 , wherein when a combination of a normalised expression value of the at least two genes, or proteins encoded by said genes, is the same or increased following administration of the therapy treatment relative to the combination of a normalised expression value of the at least two genes in a biological sample from the same individual prior to administration of the therapy treatment, the individual is predicted to not be suitable for the therapy treatment.

5. A method according to Claim 4, wherein the treatment therapy is active surveillance, a neoadjuvant therapy, an adjuvant therapy, a surgery, or a combination thereof.

6. A method according to Claim 5, wherein the neoadjuvant therapy and adjuvant therapy is an agent selected from cyclophosphamide, methotrexate, 5-fluorouracil, gemcitabine, doxorubicin (Adriamycin®; $C_{27}H_{29}NO_{11}$), paclitaxel (Taxol®; $C_{47}H_{51}NO_{14}$), capecitabine (Xeloda®; $C_{15}H_{22}FN_3O_6$), docetaxel (Taxotere®; $C_{43}H_{53}NO_{14}$), cabazitaxel (Jeytana®; $C_{45}H_{57}NO_{14}$), mitoxantrone (Novantrone®; $C_{22}H_{28}N_4O_6$), and estramustine (Estradiol 3-(bis(2-chloroethyl)carbamate) ester; $C_{23}H_{31}Cl_2NO_3$); and luteinising hormone-releasing (LHRH) agonists selected from leuprorelin (Lupron®; $C_{59}H_{84}N_{16}O_{12}$); goserelin (Zoladex®; $C_{59}H_{84}N_{18}O_{14}$); triptorelin (Decapeptyl®; $C_{64}H_{82}N_{18}O_{13}$); leuprolide mesylate ($C_{60}H_{88}N_{16}O_{15}S$); degarelix (Firmagon®; $C_{82}H_{103}ClN_{18}O_{16}$); and relugolix (Orgovyx®; $C_{29}H_{27}F_2N_7O_5S$).

7. A method according to any one of Claims 1 to 6, wherein a first gene of the at least two genes is selected from TTC21B, PCNA, IRF7, ULK1, SPAG5, and FOXM1, and a second gene of the at least two genes is selected from BRIP1, CUL4A, GPC1, MCM3, and MTUS1; or wherein the at least two genes are optionally selected from TTC21B, PCNA, IRF7, ULK1, SPAG5, and FOXM1; or wherein the at least two genes are optionally selected from BRIP1, CUL4A, GPC1, MCM3, and MTUS1.

8. A method according to any one of Claims 1 to 6, wherein the at least two genes selected are MTUS1 and MCM3.

9. A method according to any one of Claims 1 to 6, wherein the at least two genes selected are TTC21B and MCM3.

10. A method according to any one of Claims 1 to 6, wherein the at least two genes selected are TTC21B and MTUS1.

11. A method according to any one of Claims 1 to 6, wherein at least three genes are selected from CUL4A, TTC21B, MCM3, GPC1, PCNA, MTUS1, IRF7, BRIP1, ULK1, SPAG5, and FOXM1, and wherein those three genes are TTC21B, MCM3, and MTUS1.

12. A method according to any one of Claims 1 to 6, wherein at least four genes are selected from CUL4A, TTC21B, MCM3, GPC1, PCNA, MTUS1, IRF7, BRIP1, ULK1, SPAG5, and FOXM1, and wherein those four genes are TTC21B, MCM3, MTUS1, and FOXM1.

13. A method according to any one of the preceding claims, wherein the cancer is selected from the group comprising multiple myeloma, prostate cancer, glioblastoma, lymphoma, fibrosarcoma; myxosarcoma; liposarcoma; chondrosarcoma; osteogenic sarcoma; chordoma; angiosarcoma; endotheliosarcoma; lymphangiosarcoma; lymphangioendotheliosarcoma; synovioma; mesothelioma; Ewing's tumour; leiomyosarcoma; rhabdomyosarcoma; colon carcinoma; pancreatic cancer; ovarian cancer; squamous cell carcinoma; basal cell carcinoma; adenocarcinoma; sweat gland carcinoma; sebaceous gland carcinoma; papillary carcinoma; papillary adenocarcinomas; cystadenocarcinoma; medullary carcinoma; bronchogenic carcinoma; renal cell carcinoma; hepatoma; bile duct carcinoma; choriocarcinoma; seminoma; embryonal carcinoma; Wilms' tumour; cervical cancer; uterine cancer; testicular tumour; lung carcinoma; small cell lung carcinoma; bladder carcinoma; epithelial carcinoma; glioma; astrocytoma; medulloblastoma; craniopharyngioma; ependymoma; pinealoma; hemangioblastoma; acoustic neuroma; oligodendroglioma; meningioma; melanoma; retinoblastoma; head and neck cancer types; bladder cancer types; colorectal cancers; and leukemias.

14. A method according to Claim 13, wherein the cancer is prostate cancer.

15. A method according to any one of Claims 1 to 12, wherein the cancer is a pre-cancer selected from the group comprising Barrett's oesophagus, Bowen's disease, Familial adenomatous polyposis, Lobular carcinoma in situ, Lynch syndrome, MEN2, Vaginal intra-epithelial neoplasia, and Vulval intraepithelial neoplasia.

Figure 1

Figure 2

All 512 possible combinations that include MTUS1 + TTC21B in black
2-gene MTUS1 + TTC21B combination in red
4-gene FOXM1 + MCM3 + MTUS1 + TTC21B in green
Values to the right of the dashed grey line are statistically significant at LRS p < 0.05

All 512 possible combinations that include MTUS1 + TTC21B in black
2-gene MTUS1 + TTC21B combination in red
4-gene FOXM1 + MCM3 + MTUS1 + TTC21B in green

**Figure 3**

Europäisches
Patentamt

European
Patent Office

Office européen
des brevets

## EUROPEAN SEARCH REPORT

**Application Number**

EP 22 16 5911

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2015/087088 A2 (ALMAC DIAGNOSTICS LTD [GB]) 18 June 2015 (2015-06-18) * claims 1-45 * * page 10, last paragraph - page 11, paragraph 3 * * page 33, last paragraph - page 35, paragraph 2 * | 1-15 | INV. C12Q1/6886 |
| Y | WO 2016/042164 A1 (PROVOST FELLOWS FOUNDATION SCHOLARS & THE OTHER MEMBERS OF BOARD OF TH) 24 March 2016 (2016-03-24) * claims 1-23 * | 1-15 | |
| Y | WO 03/078662 A1 (GENOMIC HEALTH [US]; BAKER JOFFRE B [US] ET AL.) 25 September 2003 (2003-09-25) * claims 1-43 * * page 50, paragraph 3 - page 51, paragraph 1 * | 1-15 | |
| Y | LV DAOJUN ET AL: "A novel immune-related gene-based prognostic signature to predict biochemical recurrence in patients with prostate cancer after radical prostatectomy", CANCER IMMUNOLOGY IMMUNOTHERAPY, SPRINGER, BERLIN/HEIDELBERG, vol. 70, no. 12, 2 May 2021 (2021-05-02), pages 3587-3602, XP037610215, ISSN: 0340-7004, DOI: 10.1007/S00262-021-02923-6 [retrieved on 2021-05-02] * the whole document * | 1-15 | TECHNICAL FIELDS SEARCHED (IPC) C12Q |

-/--

The present search report has been drawn up for all claims

| Place of search | Date of completion of the search | Examiner |
|---|---|---|
| The Hague | 19 September 2022 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 3**

Europäisches
Patentamt

European
Patent Office

Office européen
des brevets

**EUROPEAN SEARCH REPORT**

Application Number

EP 22 16 5911

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ABOU-OUF HATEM ET AL: "Validation of a 10-gene molecular signature for predicting biochemical recurrence and clinical metastasis in localized prostate cancer", JOURNAL OF CANCER RESEARCH AND CLINICAL ONCOLOGY, SPRINGER INTERNATIONAL, BERLIN, DE, vol. 144, no. 5, 6 March 2018 (2018-03-06), pages 883-891, XP036488913, ISSN: 0171-5216, DOI: 10.1007/S00432-018-2615-7 [retrieved on 2018-03-06] * the whole document * * page 890, column 1 * | 1-15 | |
| Y | WO 2012/135397 A2 (LISANTI MICHAEL P [US]; SOTGIA FEDERICA [US]) 4 October 2012 (2012-10-04) * claims 5-16 * | 1-15 | |
| Y | WO 2020/214718 A1 (MEMORIAL SLOAN KETTERING CANCER CENTER [US]) 22 October 2020 (2020-10-22) * claims 1-14 * * paragraphs [0007] - [0009], [0083] - [0086], [0139], [0151], [0199] - [0201] * | 1-15 | TECHNICAL FIELDS SEARCHED (IPC) |
| Y | WO 2015/127101 A1 (UNIV COLUMBIA [US]) 27 August 2015 (2015-08-27) * claims 1-9 * * paragraphs [0082] - [0085] * | 1-15 | |

-/--

The present search report has been drawn up for all claims

| Place of search | Date of completion of the search | Examiner |
|---|---|---|
| The Hague | 19 September 2022 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 22 16 5911

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | QIAN YUQUAN ET AL: "Prognostic Cancer Gene Expression Signatures: Current Status and Challenges", CELLS, vol. 10, no. 3, 15 March 2021 (2021-03-15), page 648, XP055962114, DOI: 10.3390/cells10030648 Retrieved from the Internet: URL:https://www.ncbi.nlm.nih.gov/pmc/artic les/PMC8000474/pdf/cells-10-00648.pdf> * the whole document * ----- | 1-5 | |
| A | YIJUN SUN ET AL: "Optimizing molecular signatures for predicting prostate cancer recurrence", THE PROSTATE, vol. 69, no. 10, 1 July 2009 (2009-07-01), pages 1119-1127, XP055048300, ISSN: 0270-4137, DOI: 10.1002/pros.20961 * the whole document * ----- | 1-5 | |

TECHNICAL FIELDS
SEARCHED (IPC)

The present search report has been drawn up for all claims

| Place of search | Date of completion of the search | Examiner |
|---|---|---|
| The Hague | 19 September 2022 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................
& : member of the same patent family, corresponding document

EPO FORM 1503 03.82 (P04C01)

EP 4 253 567 A1

## ANNEX TO THE EUROPEAN SEARCH REPORT
## ON EUROPEAN PATENT APPLICATION NO.

EP 22 16 5911

19-09-2022

| Patent document cited in search report | | | Publication date | Patent family member(s) | | | Publication date |
|---|---|---|---|---|---|---|---|
| WO 2015087088 | A2 | 18-06-2015 | | AU | 2014363173 | A1 | 07-07-2016 |
| | | | | BR | 112016013182 | A2 | 26-09-2017 |
| | | | | CA | 2932553 | A1 | 18-06-2015 |
| | | | | CN | 105940117 | A | 14-09-2016 |
| | | | | EP | 3080293 | A2 | 19-10-2016 |
| | | | | JP | 2017507320 | A | 16-03-2017 |
| | | | | KR | 20160098351 | A | 18-08-2016 |
| | | | | SG | 11201604508P | A | 28-07-2016 |
| | | | | US | 2016312294 | A1 | 27-10-2016 |
| | | | | WO | 2015087088 | A2 | 18-06-2015 |
| WO 2016042164 | A1 | 24-03-2016 | | AU | 2015316711 | A1 | 06-04-2017 |
| | | | | CA | 2961725 | A1 | 24-03-2016 |
| | | | | EP | 3194621 | A1 | 26-07-2017 |
| | | | | ES | 2753625 | T3 | 13-04-2020 |
| | | | | JP | 6730983 | B2 | 29-07-2020 |
| | | | | JP | 2017536085 | A | 07-12-2017 |
| | | | | US | 2018148788 | A1 | 31-05-2018 |
| | | | | US | 2020157643 | A1 | 21-05-2020 |
| | | | | WO | 2016042164 | A1 | 24-03-2016 |
| WO 03078662 | A1 | 25-09-2003 | | AT | 529535 | T | 15-11-2011 |
| | | | | AU | 2003253986 | A1 | 29-09-2003 |
| | | | | CA | 2478850 | A1 | 25-09-2003 |
| | | | | CA | 2992643 | A1 | 25-09-2003 |
| | | | | DK | 1918386 | T3 | 02-01-2012 |
| | | | | DK | 2258872 | T3 | 18-11-2013 |
| | | | | DK | 2261369 | T3 | 28-07-2014 |
| | | | | DK | 2799555 | T3 | 22-05-2017 |
| | | | | DK | 3115470 | T3 | 05-11-2018 |
| | | | | EP | 1488007 | A1 | 22-12-2004 |
| | | | | EP | 1918386 | A1 | 07-05-2008 |
| | | | | EP | 2241636 | A1 | 20-10-2010 |
| | | | | EP | 2258872 | A2 | 08-12-2010 |
| | | | | EP | 2258873 | A2 | 08-12-2010 |
| | | | | EP | 2261368 | A1 | 15-12-2010 |
| | | | | EP | 2261369 | A2 | 15-12-2010 |
| | | | | EP | 2799555 | A1 | 05-11-2014 |
| | | | | EP | 3115470 | A1 | 11-01-2017 |
| | | | | ES | 2374311 | T3 | 15-02-2012 |
| | | | | ES | 2433992 | T3 | 13-12-2013 |
| | | | | ES | 2486265 | T3 | 18-08-2014 |
| | | | | ES | 2616800 | T3 | 14-06-2017 |
| | | | | ES | 2685702 | T3 | 10-10-2018 |
| | | | | HK | 1120567 | A1 | 03-04-2009 |
| | | | | HK | 1148032 | A1 | 26-08-2011 |

EPO FORM P0459

For more details about this annex : see Official Journal of the European Patent Office, No. 12/82

**page 1 of 2**

EP 4 253 567 A1

## ANNEX TO THE EUROPEAN SEARCH REPORT
## ON EUROPEAN PATENT APPLICATION NO.

EP 22 16 5911

This annex lists the patent family members relating to the patent documents cited in the above-mentioned European search report.
The members are as contained in the European Patent Office EDP file on
The European Patent Office is in no way liable for these particulars which are merely given for the purpose of information.

19-09-2022

| Patent document cited in search report | | Publication date | Patent family member(s) | | Publication date |
|---|---|---|---|---|---|
| | | | HK | 1148034 A1 | 26-08-2011 |
| | | | HK | 1203568 A1 | 30-10-2015 |
| | | | JP | 4753741 B2 | 24-08-2011 |
| | | | JP | 5373019 B2 | 18-12-2013 |
| | | | JP | 5461729 B2 | 02-04-2014 |
| | | | JP | 5461730 B2 | 02-04-2014 |
| | | | JP | 5792765 B2 | 14-10-2015 |
| | | | JP | 2005519624 A | 07-07-2005 |
| | | | JP | 2006129880 A | 25-05-2006 |
| | | | JP | 2011250809 A | 15-12-2011 |
| | | | JP | 2013146277 A | 01-08-2013 |
| | | | JP | 2013146278 A | 01-08-2013 |
| | | | JP | 2013146279 A | 01-08-2013 |
| | | | US | 2003225528 A1 | 04-12-2003 |
| | | | US | 2007059737 A1 | 15-03-2007 |
| | | | US | 2007065845 A1 | 22-03-2007 |
| | | | US | 2007065846 A1 | 22-03-2007 |
| | | | US | 2007141587 A1 | 21-06-2007 |
| | | | US | 2007141588 A1 | 21-06-2007 |
| | | | US | 2007141589 A1 | 21-06-2007 |
| | | | US | 2008182255 A1 | 31-07-2008 |
| | | | US | 2010209920 A1 | 19-08-2010 |
| | | | WO | 03078662 A1 | 25-09-2003 |
| WO 2012135397 | A2 | 04-10-2012 | NONE | | |
| WO 2020214718 | A1 | 22-10-2020 | NONE | | |
| WO 2015127101 | A1 | 27-08-2015 | CA | 2939924 A1 | 27-08-2015 |
| | | | US | 2017051281 A1 | 23-02-2017 |
| | | | US | 2017058354 A1 | 02-03-2017 |
| | | | WO | 2015127101 A1 | 27-08-2015 |
| | | | WO | 2015127104 A1 | 27-08-2015 |

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4943522 A
- US 6485982 B
- US 6187598 B
- US 5770460 A
- US 5622871 A
- US 6565808 B
- US 278676
- US 579673
- US 717082
- US 4444880 A
- US 4305924 A
- US 4135884 A

### Non-patent literature cited in the description

- Methods in Immunodiagnosis. John Wiley & Sons, 1980
- **OELLERICH, M.** *J. Clin. Chem. Clin. Biochem.,* 1984, vol. 22, 895-904
- **TOWBIN et al.** *Proc. Nat. Acad. Sci.,* 1979, vol. 76, 4350
- Next Generation Genome Sequencing. Wiley-VCH
- High-Throughput Next Generation Sequencing. Humanna Press, 2011
- **SAMBROOK et al.** Molecular Cloning: A Laboratory Manual. Cold Spring Harbor Laboratory Press, 2012
- **ROIFF, A et al.** PCR: Clinical Diagnostics and Research. Springer, 1994
- **COOPERBERG MR ; PASTA DJ ; ELKIN EP et al.** The University of California, San Francisco Cancer of the Prostate Risk Assessment score: a straightforward and reliable preoperative predictor of disease recurrence after radical prostatectomy. *J Urol,* 2005, vol. 173, 1938-42
- **MOTTET N ; BELLMUNT J ; BRIERS E et al.** members of the EAU - ESTRO - ESUR -SIOG Prostate Cancer Guidelines Panel. *EAU - ESTRO - ESUR - SIOG Guidelines on Prostate Cancer, https://uroweb.org/guideline/prostate-cancer/#4*